(19) Europäisches Patentamt
European Patent Office
Office européen des brevets

(11) EP 2 298 792 A2

(12) EUROPEAN PATENT APPLICATION

(43) Date of publication:
**23.03.2011 Bulletin 2011/12**

(21) Application number: **10191014.9**

(22) Date of filing: **07.08.2006**

(51) Int Cl.:
***C07K 14/00*** *(2006.01)* ***A61K 38/10*** *(2006.01)*
***A61K 38/08*** *(2006.01)* ***A61K 38/16*** *(2006.01)*
***A61K 47/48*** *(2006.01)* ***C07K 7/06*** *(2006.01)*
***C07K 7/08*** *(2006.01)* ***C12N 9/12*** *(2006.01)*
***G01N 33/554*** *(2006.01)* ***G01N 33/566*** *(2006.01)*

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.08.2005 US 706064 P**
**22.02.2006 CA 2539218**

(62) Document number(s) of the earlier application(s) in accordance with Art. 76 EPC:
**06804620.0 / 1 922 328**

(71) Applicant: **Pharmagap Inc.**
**Ottawa, Ontario K1A 0R6 (CA)**

(72) Inventors:
- **Phipps, Jenny**
  **Chelsea, Québec J0X 1N0 (CA)**
- **Terreux, Raphael**
  **69008 Lyon (FR)**

(74) Representative: **Clegg, Richard Ian**
**Mewburn Ellis LLP**
**33 Gutter Lane**
**London**
**EC2V 8AS (GB)**

Remarks:
This application was filed on 12-11-2010 as a divisional application to the application mentioned under INID code 62.

(54) **Peptides targeted to protein kinase C isoforms and uses thereof**

(57) Peptides having a sequence of general formula (I), or the retro form thereof, and having an affinity for one or more mammalian protein kinase C-alpha isoforms are provided: X-[(HY-HB)$_n$-linker]$_m$ (HB-HY)$_2$-HB-(HY)$_m$-Z (I) wherein: HY represents a block of 1 to 4 hydrophobic amino acid residues selected from the group of: Ala, Gly, He, Leu, Phe and Val; HB represents a block of 1 to 4 amino acid residues capable of forming hydrogen bonds selected from the group of: Arg, Asn, Asp, Glu, Gln, Lys and Ser; 'linker' represents 1 to 4 Gly residues; n is 1, 2 or 3; m is 0 or 1; X represents the N-terminus of the peptide or a modified version thereof, and Z represents the C-terminus of the peptide or a modified version thereof. The peptides can be used as probes, screening agents, targeting agents, purification agents and diagnostic agents.

FIGURE 4

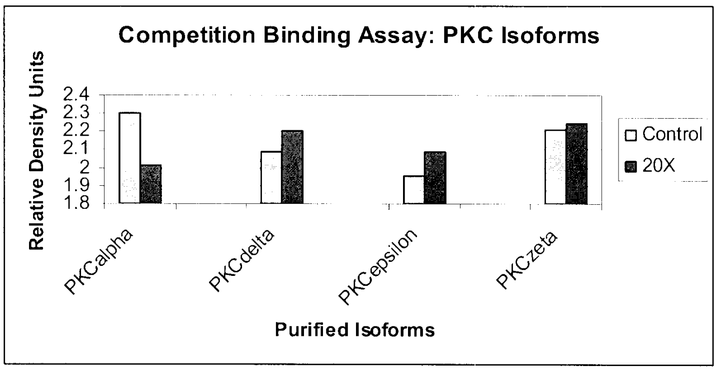


EP 2 298 792 A2

## Description

## FIELD OF THE INVENTION

The present invention relates to the field of protein kinases and, in particular, to peptides having affinity for protein kinase C isoforms.

## BACKGROUND OF THE INVENTION

Protein kinase C enzymes are phospholipid-dependent, cytoplasmic serine/threonine protein kinases that are key players in intracellular signal transduction. As such, PKCs are important mediators of a number of cellular events, including cell growth, differentiation and apoptosis. Due to their involvement in various cellular signalling events, PKCs are of interest to the pharmaceutical and biotech industries as potential drug targets.

There are currently eleven (11) known isoforms of PKC, which have been grouped into three sub-families according to their structure and cofactor regulation. The α, βI, βII and γ isoforms belong to the conventional or classical PKC sub-family; the δ, ε, θ, μ and η isoforms belong to the novel PKC sub-family, and the ζ, and i/λ isoforms belong to the atypical PKC sub-family. Each isoform is essential, at normal levels, for many cell processes (Dutil, E.M. & Newton, A.C. (2000) J. Biol. Chem., 275 (14), 10697-10701; Newton, A.C. (1995), J. Biol. Chem., 270 (48), 28495-28498).

Although a number of "broad-spectrum" compounds that demonstrate activity towards a range of PKCs have been developed (see Goekjian, P.G. & Jirousek, M.R., *ibid.;* Goekjian, P.G. & Jirousek, M.R., Expert Opin. Investig. Drugs, 10:2117-2140), identification of "isoform-specific" compounds that demonstrate activity only towards a specific PKC isoform or group of isoforms has proven to be more elusive. Isoforms of PKC are strongly conserved, especially in their catalytic and ATP-binding regions, making selectivity problematic (Xu et al., (2004) J. Biol. Chem., 279 (48), 50401-50409) and the full crystal structure of PKC has yet to be determined.

U.S. Patent No. 6,165,977 describes isozyme-specific activators/agonists of the PKC-ε isoform. The described activators/agonists are peptides having a sequence corresponding to the region of the PKC-ε protein between amino acids 85 and 92. Peptide inhibitors of PKC-ε have also been described by Johnson (Johnson, J.A., et al., (1996) J. Biol. Chem., 271:24962-24966). These peptides have a sequence that is derived from the VI region of the PKC-ε protein. U.S. Patent Application No. 2003/0223981 describes peptide inhibitors of the PKC-γ isoform havng a sequence derived from the V5 region of the PKC-γ protein, whereas International Patent Application No. PCT/EP93/00816 (WO 93/20101) describes peptide inhibitors that specifically target the PKC zeta isoform.

The α-isoform of PKC (PKC-α) has been implicated in a number of diseases including cancer, cardiovascular disease, diseases of the central nervous system and diabetes (see review by Goekjian, P.G. & Jirousek, M.R., (1999) Curr. Medicinal Chem., 6:877-903). Abnormal levels of PKC-α have been noted in a number of human tumours and aberrant over-expression of PKC-α occurs in many types of cancer, including non-small cell lung cancer (Clark et al., (2003) Cancer Research, 63:780-786), ovarian, breast (Lahn et αl., (2004) Acta-Haematol. 115:1-8), neuroblastoma, prostate (Powell et al., (1996) Cell Growth and Differentiation; 7:419-428), bladder (Koivunen et al., (2004) Cancer Research 64:5693-5701) and pancreatic cancer (Detjen et al., (2000) J. Cell Sci., 113:3025-3035). In cancer, PKC-α has been implicated in malignant transformation, proliferation, apoptosis, cell migration, cell activation and desensitizing tumour cells to chemotherapeutic agents leading to multi-drug resistance (see review by Hanauske, A-R., et al., (2004), Curr. Pharm. Design, 10:1923-1936, and Hofmann, J., (2004) Current Cancer Drug Targets, 4:125-146).

Compounds known to be capable of targeting the PKC-α isoform include various antibodies, ligands and pseudosubstrates. For example, phorbol esters activate the classical PKC and novel PKC sub-families of PKCs (Brooks G. et al. (1989) Carcinogenesis, 10, 283-288). These esters bind to the same site as the natural activator, diacylglycerol (DAG) (Wright M and McMaster C. (2002) Biol. Res., 35, 223-229). Lipids similar to DAG also bind to this site and exert an activation effect. The protein PICK-1 binds to the PKC-α isoform, but also binds to other proteins (including non-protein kinases). PICK-1 is believed to contribute to PKC intracellular translocation (Wang W-L et al. (2003) J. Biol. Chem. 278, 37705-37712). Another protein, RACK-1 that is present in the plasma membrane binds to activated PKC-α and PKC-β at their C2 domains (Rotenberg S and Sun X-G (1998) J. Biol. Chem., 273, 2390-2395).

A few isoform-selective PKC inhibitors are known that are capable of inhibiting PKC-α activity. For example, UCN-01 (an analogue of staurosporin), GF109203X and Go6976 are selective for classical PKC isoforms (α, βI, βII and γ). Aprinocarsen, an antisense oligonucleotide, is selective for PKC-α, but targets the mRNA encoding PKC-α rather than the protein itself (see Hanauske, A-R*., et al., ibid.).*

This background information is provided for the purpose of making known information believed by the applicant to be of possible relevance to the present invention. No admission is necessarily intended, nor should be construed, that any of the preceding information constitutes prior art against the present invention.

## SUMMARY OF THE INVENTION

An object of the present invention is to provide peptides targeted to protein kinase C isoforms and uses thereof. In accordance with one aspect of the present invention, there is provided a peptide of between about 5 and about 30 amino acid residues in length and having a sequence of general formula (I), or the retro form thereof:

$$X\text{-}[(HY\text{-}HB)_n\text{-linker}]_m\text{-}(HB\text{-}HY)_2\text{-}HB\text{-}(HY)_m\text{-}Z \qquad \textbf{(I)}$$

wherein:

HY represents 1 to 4 amino acid residues selected from the group of: Ala, Gly, Ile, Leu, Phe and Val;

HB represents 1 to 4 amino acid residues selected from the group of: Arg, Asn, Asp, Glu, Gln, Lys and Ser;

"linker" represents 1 to 4 Gly residues;

n is 1, 2 or 3;

m is 0 or 1;

X represents the N-terminus of the peptide or a modified version thereof; and

Z represents the C-terminus of the peptide or a modified version thereof.

In accordance with another aspect of the present invention, there is provided a composition comprising a peptide of between about 5 and about 30 amino acid residues in length and having a sequence of general formula (I), or the retro form thereof, and a physiologically acceptable diluent, carrier or excipient.

In accordance with another aspect of the present invention, there is provided a conjugate comprising a peptide of between about 5 and about 30 amino acid residues in length and having a sequence of general formula (I), or the retro form thereof, and a PKC inhibitor.

In accordance with another aspect of the present invention, there is provided a conjugate comprising a peptide of between about 5 and about 30 amino acid residues in length and having a sequence of general formula (I), or the retro form thereof, and a detectable label.

In accordance with another aspect of the present invention, there is provided a method of screening for a PKC isoform-specific targeting peptide comprising:

- providing a library of candidate peptides, each peptide having a sequence represented by general formula (I), or the retro form thereof:

$$X\text{-}[(HY\text{-}HB)_n\text{-linker}]_m\text{-}(HB\text{-}HY)_2\text{-}HB\text{-}(HY)_m\text{-}Z \qquad \textbf{(I)}$$

wherein:

HY represents 1 to 4 amino acid residues selected from the group of: Ala, Gly, Ile, Leu, Phe and Val;

HB represents 1 to 4 amino acid residues selected from the group of: Arg, Asn, Asp, Glu, Gln, Lys and Ser;

"linker" represents 1 to 4 Gly residues;

n is 1, 2 or 3;

m is 0 or 1;

X represents the N-terminus of the peptide or a modified version thereof; and

Z represents the C-terminus of the peptide or a modified version thereof.

- screening the library to determine the ability of the candidate peptides to bind to a PKC isoform or to reduce the binding of a specific antibody to a PKC isoform, and
- selecting a peptide capable of binding to the PKC isoform or of reducing the binding of a specific antibody to the PKC isoform.

In accordance with another aspect of the present invention, there is provided a PKC isoform-specific targeting peptide selected by the above method.

In accordance with another aspect of the present invention, there is provided a method of screening for the presence of one or more PKC isoforms in a cell comprising contacting said cell with a peptide of between about 5 and about 30 amino acid residues in length and having a sequence of general formula (I), or the retro form thereof, under conditions that permit binding of said peptide to the one or more PKC isoforms to form a peptide-PKC complex, and detecting said peptide-PKC complex.

In accordance with another aspect of the present invention, there is provided a method of targeting a compound to one or more PKC isoform in a cell comprising contacting said cell with a conjugate, said conjugate comprising the compound conjugated to a peptide of between about 5 and about 30 amino acid residues in length and having a sequence of general formula (I), or the retro form thereof.

In accordance with another aspect of the present invention, there is provided a use of a peptide of between about 5 and about 30 amino acid residues in length and having a sequence of general formula (I), or the retro form thereof, in the preparation of a conjugate, said conjugate comprising a PKC inhibitor or a detectable label conjugated to said peptide.

In accordance with another aspect of the present invention, there is provided a use of a peptide of between about 5 and about 30 amino acid residues in length and having a sequence of general formula (I), or the retro form thereof, in the manufacture of a medicament.

## BRIEF DESCRIPTION OF THE DRAWINGS

These and other features of the invention will become more apparent in the following detailed description in which reference is made to the appended drawings.

**Figure 1** depicts the subcellular localisation of endogenous PKC-$\alpha$ in untreated human neuroblastoma (IMR-32) cells.

**Figure 2** depicts the subcellular localisation of endogenous PKC-$\alpha$ in IMR-32 cells treated with peptide PRE 2.

**Figure 3** depicts the subcellular localisation of endogenous PKC-$\alpha$ in IMR-32 cells treated with peptide PRE 3.

**Figure 4** presents the results of a competition binding assay using a fluorescent antibody and a peptide (PRE 4) specific for PKC-$\alpha$ (20X concentration).

**Figure 5** depicts a representative (partial ribbon) image of a PKC-$\alpha$ molecule.

**Figure 6** presents the results of a competition binding assay using peptide PRE 1 with various PKC isoforms: (A) PKC-alpha; (B) PKC-beta I, (C) PKC-delta, (D) PKC-iota and (E) PKC-zeta.

**Figure 7** presents the results of a competition binding assay using peptide PRE 4 with various PKC isoforms: (A) PKC-alpha; (B) PKC-beta I, (C) PKC-beta I, (D) PKC-beta II, (E) PKC-delta, (F) PKC-epsilon, (G) PKC-iota and (H) PKC-zeta.

**Figure 8** presents the results of a competition binding assay using peptide PRE 6 with various PKC isoforms: (A) PKC-alpha; (B) PKC-beta I, (C) PKC-delta, (D) PKC-epsilon, (E) PKC-iota and (F) PKC-zeta.

**Figure 9** presents the results of a competition binding assay using peptide PRE 3 with various PKC isoforms: (A) PKC-alpha; (B) PKC-beta I, (C) PKC-beta II, (D) PKC-delta, (E) PKC-epsilon, (F) PKC-epsilon, (G) PKC-iota and (H) PKC-zeta.

**Figure 10** presents the results of a competition binding assay using peptide PRE 7 with various PKC isoforms: (A) PKC-alpha; (B) PKC-beta I, (C) PKC-delta, (D) PKC-epsilon, (E) PKC-iota and (F) PKC-zeta.

**Figure 11** presents the results of a competition binding assay using peptide PRE 8 with various PKC isoforms: (A) PKC-alpha; (B) PKC-beta II, (C) PKC-beta I and (D) PKC-epsilon.

**Figure 12** presents the results of a competition binding assay using peptide PRE 9 with various PKC isoforms: (A) PKC-alpha; (B) PKC-beta I, (C) PKC-beta II, (D) PKC-delta, (E) PKC-epsilon and (F) PKC-zeta.

**Figure 13** presents the results of a competition binding assay using peptide PRE 10 with various PKC isoforms: (A) PKC-alpha; (B) PKC-beta I, (C) PKC-beta II, (D) PKC-delta, (E) PKC-epsilon and (F) PKC-zeta.

**Figure 14** presents the results of a competition binding assay using peptide PRE 11 with various PKC isoforms: (A) PKC-alpha; (B) PKC-beta I, (C) PKC-delta, (D) PKC-epsilon and (E) PKC-zeta.

**Figure 15** presents the results of a competition binding assay using peptide PRE 12 with various PKC isoforms: (A) PKC-alpha; (B) PKC-beta I, (C) PKC-beta II, (D) PKC-delta, (E) PKC-epsilon, (F) PKC-iota and (G) PKC-zeta.

**Figure 16** presents the results of a competition binding assay using peptide PRE 13 with various PKC isoforms: (A) PKC-alpha; (B) PKC-beta I, (C) PKC-delta, (D) PKC-iota, (E) PKC-zeta and (F) PKC-epsilon.

**Figure 17** presents the results of a competition binding assay using peptide PRE 5 with various PKC isoforms: (A) PKC-alpha; (B) PKC-beta I, (C) PKC-beta II, (D) PKC-delta, (E) PKC-epsilon and (F) PKC-zeta.

**Figure 18** depicts PKC-$\alpha$ expression in IMR-32 neuroblastoma cells transfected with a PKC-$\alpha$ fragment (V5E) and either (A) no further treatment (control), (B) treatment with peptide PRE 1, (C) treatment with peptide PRE 4, (D) treatment with peptide PRE 6, (E) treatment with peptide PRE 3, or (F) treatment with peptide PRE 7.

**Figure 19** depicts PKC-$\alpha$ expression in IMR-32 neuroblastoma cells transfected with a PKC-$\alpha$ fragment (V5E) and either (A) no further treatment (control), (B) treatment with peptide PRE 8, (C) treatment with peptide PRE 9, (D) treatment with peptide PRE 10, (E) treatment with peptide PRE 11, or (F) treatment with peptide PRE 12.

**Figure 20** depicts PKC-$\alpha$ expression in (A) control IMR-32 neuroblastoma cells, (B) IMR-32 neuroblastoma cells transfected with a PKC-$\alpha$ fragment (V5E), (C) IMR-32 neuroblastoma cells transfected with a PKC-$\alpha$ fragment (V5E) and treated with peptide PRE 13, and (D) IMR-32 neuroblastoma cells transfected with a PKC-$\alpha$ fragment (V5E) and treated with peptide PRE 5.

## DETAILED DESCRIPTION OF THE INVENTION

The present invention provides for peptides that are targeted to a mammalian protein kinase C (PKC) isoform. The peptides of the invention have an affinity for one or more PKC isoform and thus represent effective PKC "recognition elements," which are useful, for example, as probes, screening agents, targeting agents, purification agents and diagnostic agents. The present invention thus also provides for methods of screening for the presence of PKC isoforms in a cell and methods of targeting a compound to a PKC isoform in a cell using a peptide of the invention. In one embodiment of the present invention, the peptides bind to the target PKC isoform(s).

The peptides of the present invention have a sequence represented by general formula (I), or the retro form thereof:

$$X\text{-}[(HY\text{-}HB)_n\text{-linker}]_m\text{-}(HB\text{-}HY)_2\text{-}HB\text{-}(HY)_m\text{-}Z \qquad \textbf{(I)}$$

wherein:

HY represents a block of 1 to 4 hydrophobic amino acid residues selected from the group of: Ala, Gly, Ile, Leu, Phe and Val;

HB represents a block of 1 to 4 amino acid residues capable of forming hydrogen bonds selected from the group

of: Arg, Asn, Asp, Glu, Gln, Lys and Ser;

"linker" represents 1 to 4 Gly residues;

n is 1, 2 or 3;

m is 0 or 1;

X represents the N-terminus of the peptide or a modified version thereof, and

Z represents the C-terminus of the peptide or a modified version thereof.

The peptides of the present invention can be specific for a PKC isoform, or they can recognise one or more PKC isoforms. In one embodiment of the present invention, the peptides recognise PKC-α and optionally one or more other PKC isoforms. In accordance with this embodiment, the affinity of the peptides for the PKC isoforms other than PKC-α may be equal to or less than the affinity of the peptides for PKC-α. In a specific embodiment of the present invention, the peptides recognise PKC-α and one or more of a sub-group of PKC isoforms consisting of PKC-βI, PKC-βII and PKC-ε. In a further embodiment, the peptides demonstrate a higher affinity for PKC-α than for other isoforms of PKC. One embodiment of the present invention provides for peptides that are also capable of altering the sub-cellular localisation of PKC-α and can, therefore, act as PKC-α antagonists.
The present invention also provides for a method of screening for a PKC isoform-specific targeting peptide comprising providing a library of peptides, each peptide having a sequence represented by general formula (I), or the retro form thereof, screening the library against a panel of PKCs, and selecting a peptide having the desired isoform-specificity.

***Definitions***

Unless defined otherwise, all technical and scientific terms used herein have the same meaning as commonly understood by one of ordinary skill in the art to which this invention belongs.
The "affinity" of a peptide of the present invention for a PKC isoform can be determined by assaying the ability of the peptide to interfere with the binding of an antibody specific for the PKC isoform to the target PKC. A peptide that is capable of interfering with the binding of an isoform-specific antibody to its target PKC is defined as having an affinity for that isoform.
The term "interfere with," as used herein, means to reduce or inhibit.
By "PKC isoform-specific" or "specific for a PKC isoform" as used herein with reference to a peptide of the present invention it is meant that the peptide has a greater affinity for a particular PKC isoform as compared to its affinity for other PKC isoforms when assessed under similar assay conditions, and/or that the peptide binds to the particular PKC isoform preferentially over other PKC isoforms. Thus, for example, the term "PKC-α specific" or "specific for PKC-α" as used herein with reference to a peptide of the present invention indicates that the peptide has a greater affinity for PKC-α than for other PKC isoforms under substantially identical assay conditions, and/or that the peptide binds to PKC-α preferentially over other PKC isoforms.
As used herein with reference to a peptide of the invention, the terms "retro," "inverso," and "retro-inverso" are defined as follows. A "retro" peptide is one in which the amino acid sequence of the peptide has been reversed as compared to the parent peptide. An "inverso" peptide is a peptide in which all L-amino acids of the parent peptide have been replaced with D-amino acids (i.e. amino acids of opposite chirality to the naturally-occurring L-forms). A "retro-inverso" peptide is one in which both the amino acid sequence of the parent peptide has been reversed and all L-amino acids in the parent peptide have been replaced with D-amino acids. For example, if the parent peptide has the sequence: Thr-Ala-Tyr, the retro form is Tyr-Ala-Thr, the inverso form is thr-ala-tyr, and the retro-inverso form is tyr-ala-thr (lower case letters indicating D-amino acids).
Naturally-occurring amino acids are identified throughout by the conventional three-letter or one-letter abbreviations indicated below, which are as generally accepted in the peptide art and are recommended by the IUPAC-IUB commission in biochemical nomenclature:

**Table 1. Amino acid codes**

| Name | 3-letter code | 1-letter code | Name | 3-letter code | 1-letter code |
|---|---|---|---|---|---|
| Alanine | Ala | A | Leucine | Leu | L |

(continued)

| Name | 3-letter code | 1-letter code | Name | 3-letter code | 1-letter code |
|---|---|---|---|---|---|
| Arginine | Arg | R | Lysine | Lys | K |
| Asparagine | Asn | N | Methionine | Met | M |
| Aspartic | Asp | D | Phenylalanine | Phe | F |
| Cysteine | Cys | C | Proline | Pro | P |
| Glutamic acid | Glu | E | Serine | Ser | S |
| Glutamine | Gln | Q | Threonine | Thr | T |
| Glycine | Gly | G | Tryptophan | Trp | W |
| Histidine | His | H | Tyrosine | Tyr | Y |
| Isoleucine | Ile | I | Valine | Val | V |

The peptide sequences set out herein are written according to the generally accepted convention whereby the N-terminal amino acid is on the left and the C-terminal amino acid is on the right. By convention also, L-amino acids are represented by upper case letters and D-amino acids by lower case letters.

***PEPTIDE RECOGNITION ELEMENTS***

The present invention provides for peptides that are capable of recognizing one or more protein kinase C (PKC) isoforms, *i.e.* PKC "peptide recognition elements" (PREs). The PREs of the invention are peptides between about 5 and about 30 amino acid residues in length and have a sequence represented by general formula (I) (as shown above), or the retro form thereof.

The retro form of general formula (I) corresponds to general formula (**I-R**):

$$\text{X-(HY)}_m\text{-HB-(HY-HB)}_2\text{-[linker-(HB-HY)}_n]_m\text{-Z} \quad \mathbf{(I\text{-}R)}$$

wherein HY, HB, "linker," n, m, X and Z are as defined above for formula (**I**).

In one embodiment, the PREs of the present invention have a sequence represented by general formula (II), or the retro form thereof (general formula (**II-R)**):

$$\text{X-[(HY-HB1)}_n\text{-linker]}_m\text{-(HB-HY)}_2\text{-HB2-(HY)}_m\text{-Z} \quad \mathbf{(II)}$$

$$\text{X-(HY)}_m\text{-HB2-(HY-HB)}_2\text{-[linker-( HB1-HY)}_n]_m\text{-Z} \quad \mathbf{(II\text{-}R)}$$

wherein:

HY, HB, "linker," n, m, X and Z are as defined above for formula (I), and

HB1 and HB2 represent sub-blocks of a HB block, wherein HB1 consists of 1 to 3 amino acid residues selected from the group specified above for HB and HB2 consists of 1 or 2 amino acid residues selected from the group specified above for HB.

In another embodiment of the present invention, the "linker" in formula (II) or (II-R) represents 1 to 3 Gly residues. In a further embodiment, the "linker" in formula (II) or (II-R) represents 1 or 2 Gly residues.

In another embodiment, the PREs of the present invention have a sequence represented by general formula (III), or the retro form thereof (general formula (III-R)):

$$\text{X-(HB-HY)}_2\text{-HB2-(HY)}_m\text{-Z} \quad \mathbf{(III)}$$

$$X\text{-}(HY)_m\text{-}HB2\text{-}(HY\text{-}HB)_2\text{-}Z \quad \textbf{(III-R)}$$

wherein:

HY, HB, HB2, m, X and Z are as defined above for formula (II).

In another embodiment, the PREs of the present invention have a sequence represented by general formula (IV), or the retro form thereof (general formula (IV-R)):

$$X\text{-}(HB\text{-}HY)_2\text{-}HB2\text{-}Z \quad \textbf{(IV)}$$

$$X\text{-}HB2\text{-}(HY\text{-}HB)_2\text{-}Z \quad \textbf{(IV-R)}$$

wherein:

HY, HB, HB2, X and Z are as defined above for formula (III).

In another embodiment, the PREs of the present invention have a sequence represented by general formula (V), or the retro form thereof (general formula (V-R)):

$$X\text{-}(HB\text{-}HY)_2\text{-}HB2\text{-}HY\text{-}Z \qquad \textbf{(V)}$$

$$X\text{-}HY\text{-}HB2\text{-}(HY\text{-}HB)_2\text{-}Z \quad \textbf{(V-R)}$$

wherein:

HY, HB, HB2, X and Z are as defined above for formula (III).

In another embodiment of the present invention, in formula (V) or (V-R), HB consists of 1 or 2 amino acid residues selected from the group specified above for HB. In a further embodiment, in formula (V) or (V-R), HB2 consists of 1 amino acid residue selected from the group specified above for HB.

In an alternative embodiment of the present invention, the PREs have a sequence represented by general formula (VI), or the retro form thereof (general formula (VI-R)):

$$X\text{-}(HY\text{-}HB1)_n\text{-linker-}(HB\text{-}HY)_2\text{-}HB2\text{-}(HY)_m\text{-}Z \qquad \textbf{(VI)}$$

$$X\text{-}(HY)_m\text{-}HB2\text{-}(HY\text{-}HB)_2\text{-linker-}(HB1\text{-}HY)_n\text{-}Z \quad \textbf{(VI-R)}$$

wherein:

HY, HB, HB1, HB2, "linker," n, m, X and Z are as defined above for formula (II).

In another embodiment, the PREs of the present invention have a sequence represented by general formula (VII), or the retro form thereof (general formula (VII-R)):

$$X\text{-}(HY\text{-}HB1)_3\text{-linker-}(HB\text{-}HY)_2\text{-}HB2\text{-}HY\text{-}Z \quad \textbf{(VII)}$$

$$X\text{-}HY\text{-}HB2\text{-}(HY\text{-}HB)_2\text{-linker-}(HB1\text{-}HY)_3\text{-}Z \quad \textbf{(VII-R)}$$

wherein:

HY, HB, HB1, HB2, "linker," X and Z are as defined above for formula (VI).

In another embodiment of the present invention, in formula (VII) or (VII-R), HB and HB1 consist of 1 or 2 amino acid residues selected from the group specified above for HB. In a further embodiment, in formula (VII) or (VII-R), HB2 consists of 1 amino acid residue selected from the group specified above for HB.

In another embodiment of the present invention, the PREs have a sequence represented by general formula (VIII), or the retro form thereof (general formula (VIII-R)):

$$X\text{-}HY\text{-}HB1\text{-linker-}(HB\text{-}HY)_2\text{-}HB2\text{-}HY\text{-}Z \quad \textbf{(VIII)}$$

$$X\text{-}HY\text{-}HB2\text{-}(HY\text{-}HB)_2\text{-linker-}HB1\text{-}HY\text{-}Z \quad \textbf{(VIII-R)}$$

wherein:

HY, HB, HB1, HB2, "linker," X and Z are as defined above for formula (VI).

In another embodiment of the present invention, in formula (VIII) or (VIII-R), HB consists of 1 or 2 amino acid residues selected from the group specified above for HB. In a further embodiment, in formula (VIII) or (VIII-R), HB2 consists of 1 amino acid residue selected from the group specified above for HB.

In another embodiment of the present invention, in formula (VI), (VI-R), (VII), (VII-R), (VIII) or (VIII-R), "linker" represents 1 to 3 Gly residues. In a further embodiment, in formula (VI), (VI-R), (VII), (VII-R), (VIII) or (VIII-R), "linker" represents 1 or 2 Gly residues.

In a further embodiment of the present invention, the PREs are less than about 25 amino acids residues in length. In another embodiment, the PREs are between about 5 and about 25 amino acid residues in length. In a further embodiment, the PREs are between about 6 and about 25 amino acid residues in length. In another embodiment, the PREs are between about 7 and about 25 amino acid residues in length. In another embodiment, the PREs are less than about 22 amino acids in length. In other embodiments, the PREs are between about 5 and about 22 amino acid residues in length; between about 6 and about 22 amino acid residues in length; between about 7 and about 22 amino acid residues in length; between about 7 and about 20 amino acid residues in length; between about 8 and about 20 amino acid residues in length and between about 10 and about 20 amino acid residues in length.

The present invention also contemplates PREs that are retro, inverso, or retro-inverso forms of any one of formulae (I), (II), (III), (IV), (V), (VI), (VII) or (VIII). Retro, inverso and retro-inverso peptides are known in the art (see, for example, Goodman et al. "Perspectives in Peptide Chemistry" pp. 283-294 (1981); U.S. Patent No. 4,522,752). A retro-inverso peptide, when compared to the parent peptide, has a reversed backbone while retaining substantially the original spatial conformation of the side chains, resulting in an isomer with a topology that closely resembles the parent peptide.

In one embodiment of the present invention, the PRE has a sequence that is the retro form of general formula (I). In another embodiment, the PRE has a sequence that is the inverso form of general formula (I). In a further embodiment, the PRE has a sequence that is the retro-inverso form of general formula (I). In another embodiment, the PRE has a sequence that is the retro, inverso or retro-inverso form of general formula (III).

X and Z in formulae (I), (I-R), (II), (II-R), (III), (III-R), (IV), (IV-R), (V), (V-R), (VI), (VI-R), (VII), (VII-R), (VIII) and (VIII-R) above can represent a free amino (N)-terminus and a free carboxy (C)-terminus, respectively, or a modified N-terminus and C-terminus. The PREs can thus have a modified N-terminus, a modified C-terminus, or both a modified N-terminus and a modified C-terminus. Examples of chemical substituent groups suitable for modifying the N-terminus

and/or C-terminus of peptides are known in the art and include, but are not limited to, alkyl, alkenyl, alkynyl, amino, aryl, aralkyl, heteroalkyl, hydroxy, alkoxy, aralkyloxy, aryloxy, carboxy, acyl, aroyl, halo, nitro, alkoxycarbonyl, aryloxycarbonyl, aralkoxycarbonyl, acylamino, aroylamino, dialkylamino, carbamoyl, alkylcarbamoyl, dialkylcarbamoyl, alkylthio, aralkylthio, arylthio, alkylene, and $NZ_1Z_2$ where $Z_1$ and $Z_2$ are independently hydrogen, alkyl, aryl, or aralkyl, and the like. Blocking groups such as Fmoc (fluorenylmethyl-O-CO-), carbobenzoxy (benzyl-O-CO-), monomethoxysuccinyl, naphthyl-NH-CO-, acetylamino-caproyl and adamantyl-NH-CO-, can also be used. Other modifications contemplated by the present invention include C-terminal amidation, esterification, hydroxymethyl modification and O-modification (for example, C-terminal hydroxymethyl benzyl ether), as well as N-terminal modifications such as substituted amides, for example alkylamides and hydrazides.

In one embodiment of the present invention, X represents a N-terminus modified with an acyl group. Non-limiting examples of suitable acyl groups are benzoyl, acetyl, t-butylacetyl, *p*-phenylbenzoyl, trifluoroacetyl, cyclohexylcarbonyl, phenylacetyl, 4-phenylbutanoyl, 3,3-diphenylpropanoyl, 4-biphenylacetyl, diphenylacetyl, 2-naphthylacetyl, 3-phenylbutanoyl, α-phenyl-*ortho*-toluoyl, indole-3-acetyl, 3-indolepropanoyl, 3-indolebutanoyl, 4-(4-methoxyphenyl)butanoyl, and the like. In another embodiment, X represents a N-terminus modified with an acetyl group. In another embodiment, Z represents a C-terminus modified with an amino group.

The term "amino acid residue," as used herein, encompasses both naturally-occurring amino acids and non-naturally occurring amino acids. Examples of non-naturally occurring amino acids include, but are not limited to, D-amino acids, N-α-methyl amino acids, C-α-methyl amino acids, β-methyl amino acids and D- or L-β-amino acids. Other non-naturally occurring amino acids include, for example, β-alanine (β-Ala), norleucine (Nle), norvaline (Nva), homoarginine (Har), 4-aminobutyric acid (y-Abu), 2-aminoisobutyric acid (Aib), 6-aminohexanoic acid (ε-Ahx), ornithine (orn), sarcosine, α-amino isobutyric acid, 3-aminopropionic acid, 2,3-diaminopropionic acid (2,3-diaP), D- or L-phenylglycine, D-(trifluoromethyl)-phenylalanine, and D-p-fluorophenylalanine.

The PRE can comprise one or more non-naturally occurring amino acids. One skilled in the art could readily select appropriate non-naturally occurring amino acids for inclusion in the PRE based on consideration of the characteristics of the natural amino acid to be replaced, such as charge, size, polarity, hydrophobicity, and the like. When the PRE comprises more than one non-naturally occurring amino acids, the non-naturally occurring amino acids can be the same or different. In one embodiment, the PRE comprises one or more D-amino acid. In another embodiment, the PRE is an inverso sequence, i. e. contains all D-amino acids.

The amino acid residues included in the PREs of the invention are linked together by peptide bonds. In the context of the present invention, a "peptide bond" can be a naturally-occurring peptide bond or a non-naturally occurring (modified) peptide bond. Examples of suitable modified peptide bonds are well known in the art and include, but are not limited to, $-CH_2NH-$, $-CH_2S-$, $-CH_2CH_2-$, -CH=CH- (*cis* or *trans*), $-COCH_2-$, $-CH(OH)CH_2-$, $-CH_2SO-$, -CS-NH- and -NH-CO- (*i.e.* a reversed peptide bond) (see, for example, Spatola, Vega Data Vol. 1, Issue 3, (1983); Spatola, in Chemistry and Biochemistry of Amino Acids Peptides and Proteins, Weinstein, ed., Marcel Dekker, New York, p. 267 (1983); Morley, J. S., Trends Pharm. Sci. pp. 463-468 (1980); Hudson et al., Int. J. Pept. Prot. Res. 14:177-185 (1979); Spatola et al., Life Sci. 38:1243-1249 (1986); Hann, J. Chem. Soc. Perkin Trans. I 307-314 (1982); Almquist et a/., J. Med. Chem. 23: 1392-1398 (1980); Jennings-White et al., Tetrahedron Lett. 23:2533 (1982); Szelke et al., EP 45665 (1982); Holladay et al., Tetrahedron Lett. 24:4401-4404 (1983); and Hruby, Life Sci. 31:189-199 (1982)). The PRE can comprise one, or more than one, modified peptide bonds. When the PRE comprises more than one modified peptide bonds, the modified peptide bonds can be the same or different.

The present invention further contemplates that the PREs can be conjugated to one or more chemical moieties to enhance the activity, stability, cell permeability or other property of the PRE. For example, the present invention contemplates PREs that are conjugated to other proteins or carriers, glycosylated PREs, phosphorylated PREs, PREs conjugated to lipophilic moieties (for example, octyl, caproyl, lauryl, stearoyl moieties), PREs conjugated to an antibody or other biological ligand, PREs conjugated to a cell permeability enhancer, PREs conjugated to a detectable label, PREs conjugated to a PKC inhibitor, and PREs conjugated to a moiety that facilitates preparation, isolation and/or purification of the PRE. Examples of cell permeability enhancers that can be conjugated to the PRE include, but are not limited to, the penetratin peptide derived from the *Drosophila* antennapedia protein (RQIKIWFQNRRMKWKK; also available in activated form as Penetratin™ 1 Peptide from Qbiogene, Inc., Irvine, CA); the cell-penetrating region of the HIV tat protein (amino acid 47-57: RRRQRRKKR) (see, Vives, E. & Lebleu, B. (2002) in Cell-Penetrating Peptides, ed. Langel, U. (CRC, Boca Raton, FL), Vol. 1, pp. 3-23); Transport™ (Cambrex BioScience Inc., Baltimore, MD) and BioTrek™ (Stratagene, La Jolla, CA).

Representative, non-limiting examples of PREs in accordance with the present invention are provided in Table 2. In a specific embodiment, the present invention provides for a PRE of less than about 30 amino acid residues in length that comprises an amino acid sequence selected from the group of: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34 and SEQ ID NO:35, or the retro, inverso, or retro-inverso form thereof, wherein each of the N-terminus and

C-terminus of the PRE are independently either free or modified. In another embodiment, the present invention provides for a PRE of less than about 30 amino acids in length that comprises an amino acid sequence selected from the group of: PRE 1, PRE 2, PRE 3, PRE 4, PRE 5, PRE 6, PRE 7, PRE 8, PRE 9, PRE 10, PRE 11, PRE 12, PRE 13, PRE 14, PRE 15, PRE 16, PRE 17, PRE 18, PRE 19, PRE 20, PRE 21, PRE 22, PRE 23, PRE 24, PRE 25, PRE 26, PRE 27, PRE 28, PRE 29, PRE 30, PRE 31, PRE 32, PRE 33, PRE 34 and PRE 35 (as shown in Table 2). In a further embodiment, the present invention provides for a PRE of less than about 30 amino acids in length that comprises an amino acid sequence selected from the group of: PRE 1, PRE 2, PRE 3, PRE 4, PRE 5, PRE 6, PRE 7, PRE 8, PRE 9, PRE 10, PRE 11, PRE 12, PRE 13, PRE 14, PRE 15, PRE 16, PRE 17, PRE 18, PRE 19, PRE 20, PRE 21, PRE 22, PRE 23, PRE 24 and PRE 25 (as shown in Table 2).

**Table 2: Exemplary PRE Sequences**

| PRE # | Sequence | SEQ ID NO |
|---|---|---|
| 1 | RRKKGGKDFVVKR | 1 |
| 14 | KDAQNLIGISI | 2 |
| 15 | KDANQLIGISI | 3 |
| 16 | AKGIQEVKGGDAQNLIGISI | 4 |
| 8 | ILEDKGGDAQNLIGISI | 5 |
| 17 | RDAQNLIGISI | 6 |
| 18 | AKGIQEVKGGKDAQNLIGISI | 7 |
| 19 | KDAQNLIGISL | 8 |
| 20 | KDAQNLI | 9 |
| 21 | RDAQNLI | 10 |
| 2 | KDAQNLIGISL-$NH_2$ | 11 |
| 3 | Ac-AKGIQEVKGGDAQNLIGISI-$NH_2$ | 12 |
| 4 | Ac-KDAQNLIGISI-$NH_2$ | 13 |
| 5 | Ac-AKGIQEVKGGKDAQNLIGISI-$NH_2$ | 14 |
| 22 | Dansylglycine-KDAQNLIGISI-$NH_2$ | 15 |
| 6 | Ac-KDANQLIGISI-$NH_2$ | 16 |
| 7 | Ac-ISIGILQNADK-$NH_2$ | 17 |
| 9 | Ac-isigilqnadk- $NH_2$ | 18 |
| 10 | Ac- ISIGILNQADK-$NH_2$ | 19 |
| 11 | Ac-RDAQNLIGISI-$NH_2$ | 20 |
| 12 | Ac-KDAQNLI-$NH_2$ | 21 |
| 13 | Ac-RDAQNLI-$NH_2$ | 22 |
| 23 | ISIGILQNADK | 23 |
| 24 | isigilqnadk | 24 |
| 25 | ISIGILNQADK | 25 |
| 26 | RRRRGQQNNLS | 26 |
| 27 | KKKKGGNLVKRIL | 27 |
| 28 | ARIQQEILKKRGGGKDAQNLIGISL | 28 |
| 29 | ARGIQEFRGGKEAQNLVISIL | 29 |
| 30 | REAQNLIGISI | 30 |
| 31 | EAQNLIGISI | 31 |

(continued)

| PRE # | Sequence | SEQ ID NO |
|---|---|---|
| 32 | EAQNVIVISIL | 32 |
| 33 | EAQVSI | 33 |
| 34 | KAQNISI | 34 |
| 35 | RDAQVVRIV | 35 |

The present invention also contemplates PREs having a sequence that is a chimeric form of general formula (I), i.e. comprises two or more sequences of general formula (I) joined together. In one embodiment, therefore, the present invention provides for a PRE of less than about 30 amino acid residues in length that comprises one or more of the amino acid sequences: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34 and SEQ ID NO:35, or the retro, inverso, or retro-inverso form thereof, wherein each of the N-terminus and C-terminus of the PRE are independently either free or modified.

***PREPARATION OF THE PEPTIDE RECOGNITION ELEMENTS***

The PREs of the present invention can be readily prepared by standard peptide synthesis techniques known in the art, for example, by standard solution, suspension or solid phase techniques, such as exclusive solid phase synthesis, partial solid phase synthesis methods, fragment condensation and classical solution synthesis.

In one embodiment of the present invention, the PREs are synthesized using solid phase techniques. The principles of solid phase chemical synthesis of peptides are well known in the art and may be found in general texts in the area such as Pennington, M.W. and Dunn, B.M., Methods in Molecular Biology, Vol. 35 (Humana Press, 1994); Dugas, H. and Penney, C., Bioorganic Chemistry (1981) Springer-Verlag, New York, pgs. 54-92; Merrifield, J. M., Chem. Soc., 85:2149 (1962), and Stewart and Young, Solid Phase Peptide Synthesis, pp. 24-66, Freeman (San Francisco, 1969).

In general, for solid phase chemical synthesis, an insoluble polymer support (or resin) is used to prepare the starting material by attaching a protected version of the required α-amino acid to the resin. The resin acts to anchor the peptide chain as each additional α-amino acid is attached and is composed of particles (generally between about 20-50 μm diameter) that are chemically inert to the reagents and solvents used in solid phase peptide synthesis. These particles swell extensively in solvents, which makes the linker arms more accessible. Examples of resins used in solid phase peptide synthesis include chloromethylated resins, hydroxymethyl resins, benzhydrylamine resins, and the like. Various resins suitable for solid phase peptide synthesis applications are available commercially, for example, phenylacetamidomethyl (PAM) resin, hydroxymethyl polystyrene-vinylbenzene copolymer, polyamide, p-benzyloxybenzyl alcohol resin (Wang resin) and modified versions thereof, 4-hydroxymethylphenoxymethyl-copoly(styrene-1 % divinylbenzene), 4-(2',4'-dimethoxyphenyl-Fmoc-aminomethyl)phenoxyacetamidoethyl and [5-(4-Fmoc-aminomethyl-3,5-dimethoxyphenoxy) valeric acid]-polyethylene glycol-polystyrene resins (which are commercially available from Applied Biosystems, Foster City, CA) and can be used in the preparation of the PREs of the invention.

The α-amino acid is coupled to the resin using a standard coupling reagent such as N,N'-dicyclohexylcarbodiimide (DCC), N,N'-diisopropylcarbodiimide (DIC) or O-benzotriazol-1-yl-N,N,N',N'-tetramethyluronium-hexafluorophosphate (HBTU), with or without an additional reagent such as 4-dimethylaminopyridine (DMAP), 1-hydroxybenzotriazole (HOBT), benzotriazol-1-yloxy-tris(dimethylamino) phosphonium-hexafluorophosphate (BOP) or bis(2-oxo-3-oxazolidinyl)phosphine chloride (BOPC1). The coupling generally takes place in an organic solvent such as dichloromethane, DMF or NMP.

After the initial coupling, the α-amino protecting group is removed using a standard reagent, such as a solution of trifluoroacetic acid (TFA), hydrochloric acid in an organic solvent or 20% piperidine in DMF solvent.

Suitable α-amino protecting groups are also known in the art and include, for example, acyl type protecting groups (such as formyl, trifluoroacetyl, acetyl), aromatic urethane type protecting groups (such as benzyloxycarboyl (Cbz) and substituted Cbz), aliphatic urethane protecting groups (such as t-butyloxycarbonyl (Boc), isopropyloxycarbonyl and cyclohexyloxycarbonyl), alkyl type protecting groups (such as benzyl and triphenylmethyl) and 9-fluorenylmethoxy carbonyl (Fmoc). A labile group protects the alpha-amino group of the amino acid. This group should be easily removed after each coupling reaction so that the next α-amino protected amino acid may be added.

Side chain protecting groups, when used, remain intact during coupling and typically are not removed during the deprotection of the amino-terminus protecting group or during coupling. Side chain protecting groups are generally selected such that they are removable upon the completion of the synthesis of the final peptide and under reaction

conditions that will not alter the target peptide. Examples of side chain protecting groups include, but are not limited to, benzyl, 2,6-dichlorobenzyl, methyl, ethyl, and cyclohexyl for Asp; acetyl, benzoyl, trityl, tetrahydropyranyl, benzyl, 2,6-dichlorobenzyl, and Cbz for Ser; nitro, Tosyl (Tos), Cbz, adamantyloxycarbonyl mesitoylsulfonyl (Mts), or Boc for Arg and Cbz, 2-chlorobenzyloxycarbonyl (2-Cl-Cbz), and 2-bromobenzyloxycarbonyl (2-BrCbz), ivDde, Tos, or Boc for Lys. Other examples are known in the art.

After removal of the α-amino protecting group, the remaining protected amino acids are coupled in the desired order to the peptide chain in a stepwise manner. An excess of each protected amino acid is generally used with an appropriate carboxyl group activator, such as dicyclohexylcarbodiimide (DCC) in methylene chloride and/or dimethyl formamide (DMF), *N*-[(dimethylamino)-1*H*-1,2,3-triazolo[4,5-*b*]pyridin-1-ylmethylene]-*N*-methylmethanaminium hexafluorophosphate *N*-oxide (HATU), *N*-[1*H*-benzotriazol-1-yl)-(dimethylamino)methylene]-*N*-methylmethanaminium hexafluorophosphate *N*-oxide (HBTU) and (benzotriazol-1-yl-*N* oxy)tris(dimethylamino)phosphonium hexafluorophosphate (BOP).

Once the desired amino acid sequence has been synthesized, the stable blocking groups are removed and the peptide is decoupled from the resin support by treatment with a suitable reagent, such as Reagent K, which includes TFA (82.5%), Thioanisole (5%), Phenol (5%), $H_2O$ (5%), and 1,2-ethanedithiol (EDT, 2.5%). The decoupling reagent may simultaneously cleave any side chain protecting groups. Alternatively, the side chain protecting groups can be cleaved off using a separate reagent, for example, 20% piperidine in DMF for Fmoc groups or 2% hydrazine in DMF for ivDde groups.

During and/or after synthesis, the PRE can be submitted to one or more purification procedures. These purification procedures can be conducted when the peptide is still protected or can be conducted once the deprotected peptide is obtained. Methods of purifying peptides are well known in the art (see, for example, Current Protocols in Protein Science, Coligan, J.E., et al. (eds.), John Wiley & Sons, (2005 & updates)) and can include one or more chromatographic steps, for example, ion exchange chromatography, hydrophobic adsorption/interaction chromatography, silica gel adsorption chromatography and various forms of high performance liquid chromatography (HPLC), such as reverse-phase HPLC.

In one embodiment of the present invention, the PREs are synthesized on a commercially available peptide synthesizer (such as the Pioneer Peptide Synthesizer available from Applied Biosystems, Foster City, CA, or the Liberty System from CEM Corporation, Matthews, North Carolina) following the manufacturer's instructions and employing suitable protecting groups to protect the amino acid side chains, as necessary.

The above techniques can also be used to synthesize PREs which include one or more non-naturally occurring amino acids. Covalent modifications of the PRE can be introduced, for example, by reacting targeted amino acid residues with an organic derivatising agent that is capable of reacting with selected amino acid side chains or with the terminal residue(s) as is known in the art. Selection of appropriate derivatising agent(s) can be readily accomplished by a worker skilled in the art.

Methods of synthesizing peptides having one or more modified peptide bonds are known in the art (see, for example, "Solid Phase Peptide Synthesis" Methods in Enzymology (ed. Fields, G.B. (1997) Academic Press, San Diego).

The PREs of the present invention can also be prepared in their salt form. The peptides may be sufficiently acidic or sufficiently basic to react with a number of inorganic bases, inorganic acids or organic acids, to form a salt. Acids commonly employed to form acid addition salts are inorganic acids such as hydrochloric acid, hydrobromic acid, hydroiodic acid, sulphuric acid, phosphoric acid, and the like, and organic acids such as p-toluenesulphonic acid, methanesulphonic acid, oxalic acid, p-bromophenyl-sulphonic acid, carbonic acid, succinic acid, citric acid, benzoic acid, acetic acid, and the like.

Base addition salts include those derived from inorganic bases, such as ammonium or alkali or alkaline earth metal hydroxides, carbonates, bicarbonates, and the like. Examples of bases useful in preparing the salts include, but are not limited to, sodium hydroxide, potassium hydroxide, ammonium hydroxide, potassium carbonate, and the like.

Alternatively, when the PRE comprises naturally occurring amino acids or slightly modified versions thereof, the PRE can be prepared by known genetic engineering techniques. Such methods can be found generally described in Ausubel et al. (Current Protocols in Molecular Biology, Wiley & Sons, NY (1997 and updates)) and Sambrook et al. (Molecular Cloning: A Laboratory Manual, Cold-Spring Harbor Press, NY (2001)). In general, a DNA sequence encoding the PRE is prepared and inserted into a suitable expression vector. The expression vector is subsequently introduced into a suitable host cell or tissue by one of a variety of methods known in the art, for example, by stable or transient transfection, lipofection, electroporation, or infection with a recombinant viral vector. The host cell or tissue is cultured under conditions that allow for the expression of the PRE and the PRE is subsequently isolated from the cells/tissue.

Examples of suitable expression vectors include, but are not limited to, plasmids, phagemids, cosmids, bacteriophages, baculoviruses and retroviruses, and DNA viruses. The selected expression vector can further include one or more regulatory elements to facilitate expression of the PRE, for example, promoters, enhancers, terminators, and polyadenylation signals. One skilled in the art will appreciate that such regulatory elements may be derived from a variety of sources, including bacterial, fungal, viral, mammalian or insect genes.

In the context of the present invention, the expression vector may additionally contain heterologous nucleic acid sequences that facilitate the purification of the expressed PRE, improve the expression of the PRE and/or increase the stability of the expressed PRE. Examples of such heterologous nucleic acid sequences include, but are not limited to, affinity tags such as metal-affinity tags, histidine tags, avidin / strepavidin encoding sequences, glutathione-S-transferase (GST) encoding sequences, biotin encoding sequences, stability enhancing sequences and the like.

One skilled in the art will understand that selection of the appropriate host cell for expression of the recombinant PRE will be dependent upon the vector chosen. Examples of suitable host cells include, but are not limited to, bacterial, yeast, insect, plant and mammalian cells.

If the PRE cannot be encoded or expressed but is very similar to a peptide that can be encoded or expressed, genetic engineering techniques such as those described above can be employed to prepare the encodable peptide, followed by one or more steps in which the encoded peptide is modified by chemical or enzymatic techniques to prepare the final PRE.

The present invention also provides for PREs that are conjugated to a second compound, such as another peptide, a protein, a lipophilic moiety, an antibody, a biological ligand, a PKC inhibitor, and the like. Once the PRE has been prepared, it can be readily conjugated to another compound using standard chemical ligation techniques known in the art (see, for example, Morrison and Boyd, Organic Chemistry, 6th Ed. (Prentice Hall, 1992); J. March, Advanced Organic Chemistry, 4th Ed. (Wiley 1992); G. T. Harmanson, Bioconjugate Techniques, (Academic Press, Inc. 1995), and S. S. Wong, Chemistry of Protein Conjugation and Cross-Linking, (CRC Press, Inc. 1991)). Alternatively, when the second compound is a protein or peptide, the PRE and second compound can be co-expressed as described above.

The structural link and the ligation method should be chosen so that the ability of the PRE to recognise its one or more target PKC isoforms and the biological activity or properties of the compound being conjugated are minimally compromised. The second compound can be conjugated to the PRE through an existing chemical group on the PRE by modification of such a group to introduce a new chemical group capable of conjugating the second compound. A variety of chemical groups can be subject to conjugation reactions. For example, hydroxyl groups (-OH) can be used to conjugate a second compound to the PRE through reaction with alkyl halides (R-Cl, R-Br), acyl anhydrides, acyl halides, aldehydes (-CHO), hydrazides (R-CO-NH-$NH_2$), and the like. Primary amino groups (-$NH_2$) can be used to conjugate a second compound to the PRE through reaction with alkyl halides (R-Cl, R-Br, R-I), aryl azides, acyl anhydrides, acyl halides, acyl esters, carboxylates activated with carbodiimides, aldehydes (-CHO), and the like. Carboxylic groups (-COOH) can also be used to conjugate a second compound to the PRE after the group has been activated. Suitable activation agents include, for example, organic or inorganic acid halides (for example pivaloyl chloride, ethyl chloroformate, thionyl chloride, $PC1_5$), carbodiimides (R-CO-OH+R'-N=C=N-R", for example EDC, DCC), benzotriazolyl uronium or phosphonium salts (TBTU, BOP, PyBOP, HBTU).

Some of the above reagents can also be used as bifunctional cross-linking reagents that can be employed to conjugate the second compound to the PRE. A variety of such cross-linking reagents is known in the art and many are commercially available (see, for example, S. S. Wong, *ibid.,* and catalogues from Pierce Chemical Co. and Sigma-Aldrich). Examples include, but are not limited to, diamines, such as 1,6-diaminohexane; dialdehydes, such as glutaraldehyde; bis-N-hydroxysuccinimide esters, such as ethylene glycol-bis(succinic acid N-hydroxysuccinimide ester), disuccinimidyl glutarate, disuccinimidyl suberate, and ethylene glycol-bis(succinimidylsuccinate); diisocyantes, such as hexamethylenediisocyanate; bis oxiranes, such as 1,4 butanediyl diglycidyl ether; dicarboxylic acids, such as succinyidisalicylate; 3-maleimidopropionic acid N-hydroxysuccinimide ester, and the like.

### *TESTING THE PEPTIDE RECOGNITION ELEMENTS*

#### *Affinity and Binding Assays*

In accordance with the present invention, the PREs have an affinity for one or more PKC isoforms. As noted above, the term "affinity" means that the PRE is capable of interfering with the binding of a PKC- isoform specific antibody to its target isoform. The affinity of candidate PREs for a target PKC isoform or isoforms can be tested using one or more of a number of standard assay techniques known in the art.

Typically, the ability of a candidate PRE to interfere with the binding of a PKC-isoform specific antibody to its target PKC is tested in a competitive binding assay, in which the candidate PRE and a PKC-isoform specific antibody are combined with the target PKC and the extent to which the PRE decreases binding of the antibody to the PKC is determined by comparison with a control assay conducted in the absence of the PRE. The extent to which the PRE has decreased binding of the antibody to the PKC in the assay can be determined for example, by quantifying the amount of protein:antibody complex that has formed in the assay and comparing this to the amount of protein:antibody complex that has formed in the control assay. The target PKC can be provided in the assay as a purified or partially purified protein, or it may be provided as a crude or partially purified cell extract or as a cell lysate.

The anti-PKC antibody can be labelled with a detectable label in order to facilitate detection and/or quantitation

of the protein:antibody complexes. Alternatively, the anti-PKC antibody (primary antibody) can be detected using a labelled secondary antibody that specifically recognises the primary antibody. If necessary, the protein:antibody complexes can be separated from free PKC (and other reagents, as required) prior to detection and/or quantification. Examples of suitable separation techniques are known in the art and include, for example, filtration, polyacrylamide gel electrophoresis, differential centrifugation, size exclusion chromatography, and the like.

Detectable labels are moieties having a property or characteristic that can be detected directly or indirectly. One skilled in the art will appreciate that when a detectable label is employed, it is selected such that it does not affect the affinity of the antibody for PKC. Examples of suitable labels include, but are not limited to, radioisotopes, fluorophores, chemiluminophores, colloidal particles, fluorescent microparticles, chromophores, fluorescent semiconductor nanocrystals, enzymes, enzyme substrates, enzyme cofactors, enzyme inhibitors, dyes, metal ions, metal sols, ligands (such as biotin, strepavidin or haptens), and the like. One skilled in the art will understand that these labels may require additional components, such as triggering reagents, light, binding partners, and the like to enable detection of the label.

Indirectly detectable labels are typically binding elements that are used in conjunction with a "conjugate" that in turn is attached or coupled to a directly detectable label. The binding element and the conjugate represent two members of a "binding pair," of which one component, the binding element, binds specifically to the target molecule (PRE, PKC or primary antibody) and the other of which, the conjugate, specifically binds to the binding element allowing its detection. Binding between the two members of the pair is typically chemical or physical in nature. Examples of such binding pairs include, but are not limited to, antigen/hapten and antibody; antibody and anti-antibody; receptor and ligand; enzyme/enzyme fragment and substrate/substrate analogue/ligand; biotin/lectin and avidin/streptavidin; lectin and carbohydrate; digoxin and anti-digoxin; His-tags and $Ni^{2+}$ ions; benzamidine and trypsin or other serine proteases; protein A and immunoglobulin; pairs of leucine zipper motifs (see, for example, U.S. Patent No. 5,643,731), bacitracin and undecaphosphoprenyl pyrophosphate as well as various homodimers and heterodimers known in the art.

In one embodiment of the present invention, the ability of candidate PREs to interfere with the binding of a PKC-isoform specific antibody to its target PKC is tested using the following general method. Cell lysates are obtained from an appropriate cell line using standard protocols. The proteins of the extract are separated by gel electrophoresis and immobilized on a suitable membrane by Western blotting. The membrane is then blocked using an appropriate blocking buffer to which varying concentrations of the candidate PRE have been added. A primary PKC-isoform specific antibody is then added under conditions that permit binding of the primary antibody to its target PKC and is subsequently detected by standard procedures using a suitable secondary antibody conjugate.

In another embodiment of the present invention, the candidate PREs are screened by adding various concentrations of the PRE directly to the cell extract prior to separating the proteins of the extract by gel electrophoresis and Western blotting as described above. Other methods are known in the art and include, but are not limited to, those described in the Examples provided herein.

As described above, in one embodiment of the present invention, the PRE has an affinity for PKC-$\alpha$ and optionally one or more other PKC isoforms. A PRE of the present invention is considered to be PKC-$\alpha$ specific if it has a greater affinity for PKC-$\alpha$ than for other PKC isoforms, when the affinity for each isoform is tested under the same conditions (i.e. under the same general assay procedure using the same concentration of PRE).

In accordance with one embodiment of the present invention, the PREs bind to their target PKC isoform(s). The ability of a candidate PRE to bind to a PKC can be determined by standard binding assays known in the art. In general these assays involve combining the candidate PRE and the target PKC under conditions that permit formation of a peptide:protein complex and then detecting the presence of any complexes as an indication of PRE binding to the PKC. As is the case for the affinity assays described above, the PKC can be provided in the binding assay as a purified or partially purified protein, or it may be provided as a crude or partially purified cell extract or as a cell lysate.

Either the candidate PRE or the PKC can be labelled with a detectable label in order to facilitate detection of the peptide:protein complexes. If necessary, the complexes can be separated from free PRE and free PKC (and other reagents, as required) prior to detection. Examples of suitable separation techniques are known in the art and include those indicated above. Suitable detectable labels are also described above. One skilled in the art will appreciate that the detectable label is chosen such that it does not affect the binding of the PRE to the target PKC.

Various techniques for the detection of protein:peptide complexes are known in the art and can be employed in the screening assays of the present invention (see, for example, Current Protocols in Protein Science, Coligan, J.E., et al. (eds.), John Wiley & Sons, (2005 & updates)). Examples include, but are not limited to, polyacrylamide gel electrophoresis, differential centrifugation, size exclusion chromatography, fluorescence polarisation spectrometry, scintillation proximity assay (SPA, which utilises scintillant incorporated into microspheres), Western analysis, Far-Western analysis, equilibrium sedimentation centrifugation (SEC), SEC with on-line light scattering, sedimentation velocity ultracentrifugation, surface plasmon resonance (SPR; for example, using BIACORE® technology; Biacore International AB, Uppsala, Sweden), and chemical cross-linking.

In one embodiment of the present invention, the binding between the candidate PRE and the target PKC is determined by attaching the candidate PRE to magnetic beads, for example via a biotin-streptavidin binding pair, and

then contacting the PRE with a solution or cell extract containing the PKC. After the beads have been incubated for an appropriate time with the solution/cell extract, the beads are separated from the other components of the assay, for example, by centrifugation or filtration. The separated beads are treated with an appropriate reagent to release any PRE/PKC complexes from the beads and the released complexes are then detected by Western blotting using an anti-PKC antibody.

In another embodiment of the present invention, the binding between the candidate PRE and the target PKC is determined by competition binding. PKCs are immunoprecipitated from cell extracts containing PKC, for example, using ProteinA/G-plus agarose beads (from Santa Cruz Biotechnlogy Inc.). The PKCs are separated by electrophoresis and transferred onto appropriate membranes via electrotransfer. Increasing concentrations of PRE are applied to separate membranes together with a fixed concentration of specific anti-PKC primary antibody. The PKC bands are detected with an alkaline phosphatase conjugated secondary antibody and the density of the band measured by densitometry scanning. The relative band density of the PKC isoform bands decreases by binding with PRE due to competition with the primary antibody. The results are expressed as percentage of the band density of controls untreated (no PRE), i.e. relative intensity. The decrease in relative intensity correlates to the amount of binding of the PRE to the PKC isoform.

The PKC isoform(s) used in the above affinity and binding screening assays can be purified or partially purified proteins (either native or recombinant), or they can be in the form of crude or partially purified cell extracts or cell lysates. Suitable purified PKC proteins derived from a variety of sources (including human) and various recombinant PKC proteins are available commercially (for example, from Sigma-Aldrich, MO; Merck Biosciences GmbH, Germany; Cell Sciences, Inc., MA; Oxford Biomedical Research, Inc., MI, and Tebu-bio SA, France). Alternatively, the PKC can be isolated from an appropriate source using standard methodology (see, for example, Dianoux, A.C., et al., (1989) Biochemistry 28: 424-431; Greene, N.M., et αl., (1995) J. Biol. Chem. 270:6710-6717 Ohguro, H., et al., (1996) J. Biol. Chem. 271: 5215-5224 and Huang, K.-P., et al., (1986) J. Biol. Chem. 261:12134-12140).

PKCs are present in almost all cells, therefore, extracts from or lysates of a variety of different cell types can be used as a source of PKCs in the above assays. For example, as is known in the art, PKC-α is overexpressed in a number of different cancers, and cancer cell extracts and or lysates are thus also examples of suitable sources for PKC-α. Other examples of suitable cells include, but are not limited to, neuroblastoma cells, glioma cells, oestrogen-receptor negative breast cancer cells and non-small cell lung cancer cells. Cancer cells are also appropriate sources for other PKC isoforms. For example, lung cancer cells, breast cancer cells, colon cancer cells, prostate cancer cells and bladder cancer cells can be used as a source for PKC-βI, PKC-βII, PKC-δ, PKC-ε, PKC-τ and PKC-ζ. Neuroblastoma, mesangial, promyelocytic leukemia and pancreatic neoplasm cells can also be used as a source of PKC-βI, as well as malignant lymphoma tumour, proximal pancreatic duct and dendritic cells for PKC-βII; endothelial cells and colon cancer cells for PKC-δ; neuroblastoma, upper airway, pancreatic duct and primary gastric tumour cells for PKC-ε; ovarian cancer, non small cell lung cancer and breast cancer cells for PKC-τ; and fibroblasts, immature CD34 monocytes and adipocytes for PKC-ζ.

The specific anti-PKC antibody employed in the above assays can be a polyclonal or a monoclonal antibody. Various anti-PKC antibodies are commercially available (for example, from Sigma-Aldrich, MO; Oxford Biomedical Research, Inc., MI, and Santa Cruz Biotechnology, Inc., CA).

A variety of other reagents may be included in the screening assays. For example, reagents that facilitate optimal protein-antibody, antibody-antibody and/or protein-peptide interactions, reduce non-specific or background interactions and/or otherwise improve the efficiency of the assay can be included. Non-limiting examples of such reagents include, but are not limited to, buffers; salts; neutral blocking proteins, such as albumin; detergents; protease inhibitors; phosphatase inhibitors; nuclease inhibitors; anti-microbial agents, and the like.

The screening assays can be carried out in solution or can be carried out in or on a solid support, or can employ some combination of solution and solid phases. For example, one or more of the components (such as the candidate PRE, target PKC, primary antibody, or one of the members of a binding pair) can be immobilised on a solid support. Examples of suitable solid supports are known in the art (see, for example, Current Protocols in Protein Science, Coligan, J.E., et al. (eds.), John Wiley& Sons, (2005 & updates); Affinity Chromatography: Principles & Methods, Pharmacia LKB Biotechnology (1988), and Doonan, Protein Purification Protocols, The Humana Press (1996)). Examples include, but are not limited to, various resins and gels (such as silica-based resins/gels, cellulosic resins/gels, cross-linked polyacrylamide, dextran, agarose or polysaccharide resins/gels), membranes (such as nitrocellulose or nylon membranes), beads (such as glass beads, agarose beads, cross-linked agarose beads, polystyrene beads, various coated or uncoated magnetic beads, polyacrylamide beads, latex beads and dimethylacrylamide beads), chitin, sand, pumice, glass, metal, silicon, rubber, polystyrene, polypropylene, polyvinylchloride, polyvinyl fluoride, polycarbonate, latex, diazotized paper, the internal surface of multi-well plates, and the like, wherein the solid support is insoluble under the conditions of the assay.

As indicated above, the solid support can be particulate (pellets, beads, and the like), or can be in the form of a continuous surface (membranes, meshes, plates, slides, disks, capillaries, hollow fibres, needles, pins, chips, solid fibres, gels, and the like). These supports can be modified as necessary with reactive groups that allow attachment of

proteins or peptides, such as amino groups, carboxyl groups, sulphydryl groups, hydroxyl groups, activated versions of the preceding groups, and/or carbohydrate moieties. Examples of coupling chemistries that can be employed to immobilise the candidate PRE, target PKC or primary antibody on the solid support include cyanogen bromide activation, N-hydroxysuccinimide activation, epoxide activation, sulfhydryl activation, hydrazide activation, and carboxyl and amino derivatives for carbodiimide coupling chemistries. Other techniques are known in the art.

Alternatively, the PRE, target PKC or primary antibody can be modified with a group that allows for attachment of the peptide or protein to an appropriately modified solid support. For example, a His-tag that allows the peptide/protein to be immobilised on a solid support modified to contain $Ni^{2+}$ ions; biotin that allows the peptide/protein to be immobilised on a solid support modified to contain avidin/streptavidin, or an antigen that allows the peptide/protein to be immobilised on a solid support modified with the corresponding specific antibody. Other examples are known in the art and include the binding pairs described above.

Immobilisation of one or more component of the binding assay can facilitate "high-throughput" screening of candidate PREs. High-throughput screening provides the advantage of processing a plurality samples simultaneously and significantly decreases the time required to screen a large number of samples. For high-throughput screening, reaction components are usually housed in a multi-container carrier or platform, such as a multi-well plate, which allows a plurality of assays each containing a different candidate PRE to be monitored simultaneously. Many high-throughput screening or assay systems are now available commercially, as are automation capabilities for many procedures such as sample and reagent pipetting, liquid dispensing, timed incubations, formatting samples into a high-throughput format and microplate readings in an appropriate detector, resulting in much faster throughput times.

*Effect on PKC Activity*

In one embodiment of the present invention, the PRE inhibits the activity of its target PKC. The ability of the PRE to inhibit PKC activity can be assessed using standard protocols known in the art (see for example, Current Protocols in Pharmacology (Enna & Williams, Ed., J. Wiley & Sons, New York, NY)).

In general, the ability of a PRE to inhibit the activity of a selected PKC is assessed by adding the PRE to a reaction mixture comprising the target PKC in an appropriate buffer, together with a substrate, ATP, and any necessary co-factors (such as phosphatidylserine, phorbol esters, $Mn^2$+ and/or $Ca^{2+}$). After a suitable incubation time, the extent of phosphorylation of the substrate is monitored and compared to a control reaction, for example, a reaction conducted in the absence of the PRE, or in the presence of a known PKC inhibitor. The substrate used in the assay is a protein or a peptide that is capable of being phosphorylated by the particular PKC being investigated. In most assays, peptide substrates are used.

Standard, commercially available methods include the use of fluorescently labelled substrates (see, for example, PepTag® Non-Radioactive Assays, Promega, Madison, WI), fluorescently labelled substrates together with a quencher molecule (for example, the IQ® Assays from Pierce Biotechnology Inc., Rockford, IL) and luminescent detection of unreacted ATP (for example, the Kinase-Glo™ Luminescent Kinase Assays from Promega, Madison, WI). Methods based on fluorescence polarisation techniques that include the addition, at the end of the incubation period, of a fluorescently labelled tracer molecule and an antibody capable of binding the phosphorylated substrate and the tracer molecule (see PanVere® PolarScreen™ kits from Invitrogen, Carlsbad, CA).

***Effect on Sub-cellular Localisation of PKC-α***

In one embodiment of the present invention, the PRE affects the sub-cellular localisation of *PKC-α in vitro* and/or *in vivo.* The ability of the PRE to exert an effect on the localisation of PKC-α within a PKC-α expressing cell can be determined by contacting a cell culture with the *PRE in vitro* or by administering the PRE to a test animal *in vivo,* and employing standard immunohistochemical techniques known in the art. For example, the PRE can be introduced into an appropriate PKC-α expressing cell line *in vitro* and the localisation of PKC-α detected using a PKC-α-specific antibody, which is subsequently detected with a secondary antibody. Changes in the subcellular localization of PKC-α can be assessed by comparison of the treated cells with an appropriate control, for example, by comparison with untreated cells. The cells can optionally be treated with a compound known to stimulate PKC-α translocation, such as TPA, PMA or bryostatin. In a specific embodiment of the invention, the PRE interferes with the translocation of PKC-α to the cell membrane.

***METHOD OF SCREENING FOR ISOFORM-SPECIFIC PEPTIDE RECOGNITION ELEMENTS***

The present invention provides for a method of screening for a PKC isoform-specific PRE that specifically binds to one isoform of PKC. The method generally comprises the steps of providing a library of candidate isoform-specific PREs, each PRE having a sequence represented by general formula (I), or the retro form thereof, screening the library

against one or more PKC isoforms, and selecting a peptide having the desired isoform-specificity.

A "library" in this context comprises a plurality of candidate PREs, for example, between two and about 1000 candidate PREs. The size of the library can be selected based on the capacity of the screening technique being employed. For example, when high-throughput screening techniques are available, the library can comprise a large number of candidate PREs, such as between about 20 and about 1000 candidate PREs, or between about 50 and 1000 candidate PREs. When low throughput screening techniques are employed, the library can comprise a smaller number of candidate PREs, for example, between about two and about 50, or between about two and about 20 candidate PREs.

Libraries of candidate PREs can be readily prepared by standard peptide synthesis techniques, such as solid-phase peptide synthesis or solution peptide synthesis as described above. The candidate PREs can be screened for their affinity for a particular PKC isoform using assay methods such as those described above, for example, by a competitive or other binding assay. The candidate PREs can be screened against a single PKC isoform, or they can be screened against a plurality of different isoforms. The method can be readily adapted to high throughput, thus allowing large numbers of candidate PREs to be screened and/or allowing candidate PREs to be screened against a plurality of PKCs simultaneously.

***USES OF THE PEPTIDE RECOGNITION ELEMENTS***

The PREs of the present invention have numerous applications in the areas of diagnostics and therapeutics, as well as in research relating to PKC and development of PKC antagonists and agonists.

For example, the PREs can be labelled and used to probe for the presence of, and/or to determine the subcellular localisation of, one or more PKC isoforms in cells or tissues, including living cells, fixed cells, biological fluids, tissue homogenates, biopsy samples and the like.

PREs that bind to one or more PKC isoforms can be used as commercial reagents in place of, or as competitors of, PKC-specific antibodies in various medical research and diagnostic applications. For example, labelled PREs can be used for *in situ* staining, fluorescence-activated cell sorting (FACS) analysis, Western blotting, ELISAs, and the like. The PREs can also be used as affinity tags and/or purification reagents for PKC isoforms, for example, by immobilization of the PRE to a suitable solid support. The present invention this provides for a method of screening for the presence of a PKC isoform in a cell comprising contacting the cell with a PRE under conditions that permit binding of the PRE to its target PKC to form a PRE:PKC complex, and then detecting the complex.

The PREs can be used *in vitro* as tools for understanding the biological role of PKC isoforms, including the evaluation of factors thought to influence, and be influenced by, the activation of a PKC. Similarly the PREs can be used to evaluate the expression pattern and/or localization of PKC isoforms in cells and tissues, such as cancer cells. The PREs can be used as competitive binders in the screening and development of other PKC binding compounds and/or antagonists, such as new PKC inhibiting therapeutics.

The PREs can also be used to target conjugated compounds to one or more PKC isoform. For example, the PRE can be conjugated to a PKC inhibitor, either directly or via an appropriate linker moiety, and used to target this inhibitor specifically to one or more than one PKC isoform *in vitro* or *in vivo.* PREs that are capable of specifically targeting for example PKC-$\alpha$ are useful in therapeutic applications as most PKC inhibitors are non-specific and inhibit more than one PKC isoform in the cell. Targeting the inhibitor specifically to PKC-$\alpha$ can thus help to minimise undesirable side-effects *in vivo* that occur due to the non-specific activity of the inhibitor. Accordingly, in one embodiment of the present invention, the PRE is conjugated to a PKC inhibitor and used to target the PKC inhibitor specifically to PKC-$\alpha$. Another embodiment of the present invention provides for a method of targeting a compound to PKC-$\alpha$ in a cell comprising contacting the cell with a conjugate of the compound and a PRE.

One embodiment of the present invention provides for the use of the PREs as PKC-$\alpha$ inhibitors. In a specific embodiment of the invention, the PRE interferes with the translocation of PKC-$\alpha$ to the cell membrane. As is known in the art, translocation of PKC-$\alpha$ to the cell membrane is essential for the function of this enzyme. Accordingly, a PRE that prevents the translocation of PKC-$\alpha$ to the cell membrane will also attenuate PKC-$\alpha$ activity in the cell.

The present invention also provides compositions for pharmaceutical or diagnostic applications comprising one or more PREs in association with a physiologically acceptable carrier, diluent or excipient. The PRE can be included in the composition either alone as an active ingredient of the composition, or as a conjugate, for example, conjugated to a PKC inhibitor or a detectable label, and acts as a PKC targeting compound. Methods of preparing pharmaceutical compositions are well-known in the art (see, for example, "Remington: The Science and Practice of Pharmacy" (formerly "Remingtons Pharmaceutical Sciences"); Gennaro, A., Lippincott, Williams & Wilkins, Philidelphia, PA (2000)).

The compositions can be administered to a mammal to inhibit one or more PKC *isoforms in vivo.* Thus, the present invention encompasses methods for therapeutic treatment of PKC-related disorders in a mammal that comprise administering a composition comprising a PRE alone or conjugated to a PKC inhibitor in an amount sufficient to inhibit PKC activity *in vivo.* The present invention further provides for administration of the PREs to a mammal for diagnostic purposes, for example, to detect one or more PKC isoforms.

### *KITS*

The present invention further provides for kits comprising one or more PRE. The kits can be, for example, research kits, diagnostic kits or pharmaceutical kits.

### *Research and Diagnostic Kits*

For research and diagnostic applications, the PRE provided in the kit can incorporate a detectable label, such as a fluorophore, radioactive moiety, enzyme, biotin/avidin label, chromophore, chemiluminescent label, or the like, or the kit may include reagents for labelling the PRE. The PRE can be provided in a single container, aliquoted into separate containers, or pre-dispensed into an appropriate assay format, for example, into microtitre plates and/or immobilised on a solid support.

The kits can optionally include reagents required to conduct screening or diagnostic assays, such as buffers, salts, antibodies, enzymes, enzyme co-factors, substrates, culture media, detection reagents, and the like. Other components, such as buffers and solutions for the isolation and/or treatment of a test sample, may also be included in the kit. The kit may additionally include one or more controls, such as purified or partially purified PKC-$\alpha$.

One or more of the components of the kit may be lyophilised and the kit may further comprise reagents suitable for the reconstitution of the lyophilised components. The various components of the kit are provided in suitable containers. For example, for screening and diagnostic purposes one or more of the containers may be a microtitre plate. Where appropriate, the kit may also optionally contain reaction vessels, mixing vessels and other components that facilitate the preparation of reagents or the test sample. The kit may also include one or more instrument for assisting with obtaining a test sample, such as a syringe, pipette, forceps, measured spoon, or the like.

The kit can optionally include instructions for use, which may be provided in paper form or in computer-readable form, such as a disc, CD, DVD or the like.

### *Pharmaceutical Kits*

For therapeutic or *in vivo* diagnostic applications, the kit can comprise a PRE in the form of a composition. The composition can comprise the PRE alone, or a PRE conjugated to a PKC inhibitor or to a detectable label. Individual components of the kit can be packaged in separate containers, associated with which, when applicable, can be a notice in the form prescribed by a governmental agency regulating the manufacture, use or sale of pharmaceuticals or biological products, which notice reflects approval by the agency of manufacture, use or sale for human or animal administration. The kit may optionally contain instructions or directions outlining the method of use or dosing regimen for the PRE and/or the PKC inhibitor.

When the components of the kit are provided in one or more liquid solutions, the liquid solution can be an aqueous solution, for example a sterile aqueous solution. In this case the container means may itself be an inhalant, syringe, pipette, eye dropper, or other such like apparatus, from which the composition may be administered to a patient or applied to and mixed with the other components of the kit.

The components of the kit may also be provided in dried or lyophilised form and the kit can additionally contain a suitable solvent for reconstitution of the lyophilised components. Irrespective of the number or type of containers, the kits of the invention also may comprise an instrument for assisting with the administration of the composition to a patient. Such an instrument may be an inhalant, syringe, pipette, forceps, measured spoon, eye dropper or similar medically approved delivery vehicle.

The invention will now be described with reference to specific examples. It will be understood that the following examples are intended to describe embodiments of the invention and are not intended to limit the invention in any way.

## EXAMPLES

The following peptides were made by standard solid phase synthetic procedures.

**Table 3: Sequences of Exemplary PREs**

| Peptide | Sequence | SEQ ID NO |
|---|---|---|
| PRE 1 | RRKKGGKDFVVKR | 1 |
| PRE 2 | KDAQNLIGISL-$NH_2$ | 11 |
| PRE 3 | Ac-AKGIQEVKGGDAQNLIGISI-$NH_2$ | 12 |

(continued)

| Peptide | Sequence | SEQ ID NO |
|---|---|---|
| PRE 4 | Ac-KDAQNLIGISI-$NH_2$ | 13 |
| PRE 5 | Ac-AKGIQEVKGGKDAQNLIGISI-$NH_2$ | 14 |
| PRE 6 | Ac-KDANQLIGISI-$NH_2$ | 16 |
| PRE 7 | Ac-ISIGILQNADK-$NH_2$ | 17 |
| PRE 8 | ILEDKGGDAQNLIGISI | 5 |
| PRE 9 | Ac-isigilqnadk- $NH_2$ | 18 |
| PRE 10 | Ac- ISIGILNQADK-$NH_2$ | 19 |
| PRE 11 | Ac-RDAQNLIGISI-$NH_2$ | 20 |
| PRE 12 | Ac-KDAQNLI-$NH_2$ | 21 |
| PRE 13 | Ac-RDAQNLI-$NH_2$ | 22 |

As demonstrated in the examples below, all the PREs tested have an affinity for at least one PKC isoform, and some are specific for one isoform or a group of isoforms. As it was expected that the measured level of specificity of the binding of the PREs to the various PKC isoforms may vary somewhat depending on the protocol selected for testing, several procedures were used to assess the binding specificity of the PRE as described below. Possible causes of variation between and within protocols include the fact that the PKC isoform specific primary antibodies do not bind their target to the same degree, which does not allow for quantitative comparison among isoforms, but does allow for a precise comparison of dose response of PRE-binding to a particular isoform. In addition, when using commercially purified enzymes, the preparations may include partially unfolded protein, which can alter the binding capacity assessment for the PRE binding, and when using cell extracts, which contain a complex mixture of molecules, unknown molecules may compete for PRE binding. Finally, in cells, an excess of PRE may saturate the binding site of its targeted isoform depending of the intracellular content of this isoform and its sublocalization. Despite these limitations of the different procedures, one skilled in the art will appreciate that the results provide a good indication of the overall binding and specificity of each of the tested PREs.

**EXAMPLE 1: *In vitro* Competition Experiments with PKC-α: Protocol A**

The ability of the peptides to interfere with the binding of a PKC-α-specific monoclonal antibody to PKC-α was determined using the following protocol. Cell lysates from either IMR-32 (human neuroblastoma) cells or C6Cx43 cells (rat glioma transfected cells overexpressing connexin 43) were obtained using standard protocols and the proteins of the lysate were separated by SDS PAGE electrophoresis and electrotransferred onto a nitrocellulose membrane. The membrane was incubated for 30 minutes in blocking buffer (TBST) containing the test peptide at either 5X or 20X the concentrations of the primary antibody. A primary polyclonal antibody specific for PKC-α (Santa Cruz Biotechnology, Inc., CA) was then added (15 μg/ml) and the membrane incubated for a further 45 minutes. Finally, the primary antibody was detected with a secondary antibody conjugated to alkaline phosphatase using standard procedures. The intensity of the band corresponding to PKC-α was assessed by scanning and densitometry using the Gel-Pro software (Media Cybernetics) to obtain relative band intensities (average of 3 replicas). Control assays were conducted as described above except that blocking buffer without peptide was used.

The results are summarised in Tables 4 and 5 below. The results are expressed as relative band intensity and as a percentage of the intensity of the corresponding band in the control assay ("Relative intensity (%)"). "% inhibition" relates to the percentage of the PKC-α band that is inaccessible to the antibody.

The results clearly indicate that both PRE 1 and PRE 2 mask the antigenic site of PKC-α on the membrane and that PRE 2 appears to be more efficient in this regard than PRE 1. Under these assay conditions, PRE 3 did not show an effect on antibody binding.

**Table 4. Inhibition of Antibody Binding to PKC-α by PRE 1 and PRE 2 in IMR-32 Neuroblastoma Cells: Protocol A**

| Peptide | Band Intensity | Relative Intensity (%) | Inhibition (%) |
|---|---|---|---|
| **None (control)** | 2.087 | -- | -- |

(continued)

| Peptide | | Band Intensity | Relative Intensity (%) | Inhibition (%) |
|---|---|---|---|---|
| **PRE 1** | **(75 μg)**<br>**(300 μg)** | 1.040<br>0.771 | 49.8<br>36.9 | 50.2<br>63.1 |
| **PRE 2** | **(75 μg)**<br>**(300 μg)** | 0.917<br>0.192 | 43.9<br>9.1 | 56.1<br>90.9 |

**Table 5. Inhibition of Antibody Binding to PKC-α by PRE 3: Protocol A**

| Peptide | IMR-32 Cells | | | C6Cx43 Cells | | |
|---|---|---|---|---|---|---|
| | Band Intensity | Relative Intensity (%) | Inhibition(%) (%) | Band Intensity | Relative Intensity (%) | Inhibition (%) |
| **None (control)** | 0.895 | -- | -- | 0.927 | -- | -- |
| **PRE 3** | | | | | | |
| **(75 μg)** | 0.889 | 99.3 | 0.7 | 0.909 | 98.0 | 0.0 |
| **(300 μg)** | 0.922 | 103.0 | 0.0 | 0.888 | 95.8 | 0.0 |
| **(600 μg)** | 0.888 | 99.2 | 0.0 | 0.889 | 95.9 | 0.0 |
| **None (control)** | 0.591 | -- | -- | 0.572 | -- | -- |
| **PRE 3** | | | | | | |
| **(3.75 mg)** | 0.600 | 101.5 | 0.0 | 0.603 | 105.4 | 0.0 |
| **(5.25 mg)** | 0.603 | 102.0 | 0.0 | 0.599 | 104.7 | 0.0 |
| **(7.5 mg)** | 0.609 | 103.0 | 0.0 | 0.589 | 103.0 | 0.0 |

**EXAMPLE *2: In vitro* Competition Experiments with PKC-β: Protocol A**

Peptides PRE 1, PRE 2 and PRE 3 (see Table 3) were tested for their ability to interfere with the binding of a PKC-β-specific polyclonal antibody (Santa Cruz Biotechnology, Inc.) to PKC-β using the general protocol described in Example 1. The results are shown in Table 6 and show that there is some cross reactivity between both PRE 1 and PRE 2 and PKC-β. It is worth noting in this regard that PKC-α and PKC-β belong to the same sub-group of PKCs (cPKCs). The effect with PKC-β, however, is fairly limited indicating that these two peptides have a reasonable degree of specificity for PKC-α. Under these assay conditions, PRE 3 did not show an effect on antibody binding to PKC-β.

**Table 6. Inhibition of Antibody Binding to PKC-β by PRE 1, PRE 2 and PRE 3 in IMR-32 Neuroblastoma Cells: Protocol A**

| Peptide | | Band Intensity | Relative Intensity (%) | Inhibition (%) |
|---|---|---|---|---|
| **None (control)** | | 1.160 | -- | -- |
| **PRE 1** | **(75 μg)**<br>**(300 μg)** | 0.825<br>0.528 | 71.1<br>45.5 | 28.9<br>54.5 |
| **PRE 2** | **(75 μg)**<br>**(300 μg)** | 0.800<br>0.403 | 69.0<br>34.7 | 31.0<br>65.3 |
| **None (control)** | | 1.855 | -- | -- |
| **PRE 3** | **(75 μg)**<br>**(300 μg)**<br>**(600 μg)** | 1.900<br>1.900<br>1.847 | 102.4<br>102.4<br>99.6 | 0.0<br>0.0<br>0.0 |

**EXAMPLE 3: *In vitro* Competition Experiments with PKC-α: Protocol B**

The ability of the peptides PRE 2 and PRE 3 (see Table 3) to interfere with the binding of a PKC-α-specific polyclonal antibody (Santa Cruz Biotechnology, Inc.) to PKC-α was determined using a modified version of the protocol outlined above in which the test peptide was added directly to the cell extract prior to electrophoresis at a concentration of either 5X or 15X the concentration of the protein applied to each well of the gel for the Western blots (20 μg).

The results are shown in Table 7. The results show that the interaction between each peptide and PKC-α was sufficiently strong to prevent dissociation during electrophoresis and that both PRE 2 and PRE 3 effectively interfered with PKC-α antibody binding to PKC-α. PRE 3 was more efficient than PRE 2 under these assay conditions.

**Table 7. Inhibition of Antibody Binding to PKC-α by PRE 2 and PRE 3 in IMR-32 Neuroblastoma Cells: Protocol B**

| Peptide | | Band Intensity | Relative Intensity (%) | Inhibition (%) |
|---|---|---|---|---|
| None (control) | | 0.759 | -- | -- |
| PRE 2 | (100 μg) | 0.0103 | 0.135 | 99.9 |
| | (200 μg) | 0.0 | 0.0 | 100.0 |
| | (300 μg) | 0.0 | 0.0 | 100.0 |
| PRE 3 | (100 μg) | 0.0 | 0.0 | 100.0 |
| | (200 μg) | 0.0 | 0.0 | 100.0 |
| | (300 μg) | 0.0 | 0.0 | 100.0 |

**EXAMPLE *4: In vitro* Competition Experiments with PKC-β: Protocol B**

Peptides PRE 2 and PRE 3 (see Table 3) were tested for their ability to interfere with the binding of a PKC-β-specific polyclonal antibody (Santa Cruz Biotechnology, Inc.) to PKC-β using the general protocol described in Example 3. The results are shown in Table 8 and show that there is some cross reactivity between PRE 2 and PKC-β. At low concentrations, however, the effect is fairly limited indicating that PRE 2 has a reasonable degree of specificity for PKC-α when used at lower concentrations under these assay conditions. PRE 3 showed a similar effect on antibody binding to PKC-β under these conditions to that shown on antibody binding to PKC-α.

**Table 8. Inhibition of Antibody Binding to PKC-β by PRE 2 and PRE 3 in IMR-32 Neuroblastoma Cells: Protocol B**

| Peptide | | Band Intensity | Relative Intensity (%) | Inhibition (%) |
|---|---|---|---|---|
| None (control) | | 0.071 | -- | -- |
| PRE 2 | (100 μg) | 0.0322 | 45.3 | 54.7 |
| | (200 μg) | 0.0 | 0.0 | 100.0 |
| | (300 μg) | 0.0 | 0.0 | 100.0 |
| PRE 3 | (100 μg) | 0.0 | 0.0 | 100.0 |
| | (200 μg) | 0.0 | 0.0 | 100.0 |
| | (300 μg) | 0.0 | 0.0 | 100.0 |

**EXAMPLE 5: *In vitro* Competition Experiments with PKC-α, PKC-βI and PKC-βII: Protocol B**

Peptides PRE 3 and PRE 4 (see Table 3) were tested for their ability to interfere with the binding of isoform-specific polyclonal antibodies (Santa Cruz Biotechnology, Inc.) to PKC-α, PKC-βI or PKC-βII using the general protocol described in Example 3. The results are shown in Table 9. The results indicate that while PRE 4 shows some cross-reactivity with PKC-βI and PKC-βII, at low concentrations this peptide is reasonably specific for PKC-α. In agreement with the results shown in Table 8 above, PRE 3 showed a similar effect on antibody binding to PKC-βI and PKC-βII under these conditions to that shown on antibody binding to PKC-α.

**Table 9. Inhibition of Antibody Binding to PKC-α, PKC-βI and PKC-βII by PRE 3 and PRE 4 in IMR-32 Neuroblastoma Cells: Protocol B**

| **PKC-α Control Band intensity: 302.5** | | | |
|---|---|---|---|
| **+ PRE 3** | **100 μg** | **200 μg** | **300 μg** |
| Band Intensity<br>Relative Intensity (%)<br>Inhibition (%) | 0.0<br>0.0<br>100.0 | 0.0<br>0.0<br>100.0 | 0.0<br>0.0<br>100.0 |
| **+ PRE 4** | **100 μg** | **200 μg** | **300 μg** |
| Band Intensity<br>Relative Intensity (%)<br>Inhibition (%) | 32.2<br>10.6<br>89.4 | 1.5<br>0.5<br>99.5 | 0.0<br>0.0<br>100.0 |
| **PKC-βI** Control Band intensity: 170.5 | | | |
| **+ PRE 3** | **100 μg** | **200 μg** | **300 μg** |
| Band Intensity<br>Relative Intensity (%)<br>Inhibition (%) | 0.0<br>0.0<br>100.0 | 0.0<br>0.0<br>100.0 | 0.0<br>0.0<br>100.0 |
| **+ PRE 4** | **100 μg** | **200 μg** | **300 μg** |
| Band Intensity<br>Relative Intensity (%)<br>Inhibition (%) | 112.5<br>66.0<br>34.0 | 0.0<br>0.0<br>100.0 | 0.0<br>0.0<br>100.0 |
| **PKC-βII** Control Band intensity: 98.6 | | | |
| **+ PRE 3** | **100 μg** | **200 μg** | **300 μg** |
| Band Intensity<br>Relative Intensity (%)<br>Inhibition (%) | 0.0<br>0.0<br>100.0 | 0.0<br>0.0<br>100.0 | 0.0<br>0.0<br>100.0 |
| **+ PRE 4** | **100 μg** | **200 μg** | **300 μg** |
| Band Intensity<br>Relative Intensity (%)<br>Inhibition (%) | 69.58<br>69.5<br>29.0 | 0.0<br>0.0<br>100.0 | 0.0<br>0.0<br>100.0 |

**EXAMPLE *6: In vitro* Competition Experiments with PKC-ε: Protocol B**

Peptides PRE 2, PRE 3 and PRE 4 (see Table 3) were tested for their ability to interfere with the binding of isoform-specific polyclonal antibodies (Santa Cruz Biotechnology, Inc.) to PKC-ε using the general protocol described in Example 3. Two bands, representing alternate splicing variants of PKC-ε, were identified on the Western blot using this anti-PKC-ε antibody. The results with respect to both bands are summarised in Table 10. The results indicate that while PRE 2 and PRE 4 show some cross-reactivity with PKC-ε at low concentrations, these peptides are reasonably specific for PKC-α. PRE 3 showed a similar effect on antibody binding to PKC-ε under these conditions to that shown on antibody binding to PKC-α.

**Table 10. Inhibition of Antibody Binding to PKC-ε by PRE 3 and PRE 4 in IMR-32 Neuroblastoma Cells: Protocol B**

| **Peptide** | **PKC-ε Band 1** | | | **PKC-ε Band 2** | | |
|---|---|---|---|---|---|---|
| | **Band Intensity** | **Relative Intensity (%)** | **Inhibition (%)** | **Band Intensity** | **Relative Intensity (%)** | **Inhibition (%)** |
| **None (control)** | 420.92 | -- | -- | 260.97 | -- | -- |

(continued)

| Peptide | PKC-ε Band 1 | | | PKC-ε Band 2 | | |
|---|---|---|---|---|---|---|
| | Band Intensity | Relative Intensity (%) | Inhibition (%) | Band Intensity | Relative Intensity (%) | Inhibition (%) |
| **PRE 2** | | | | | | |
| **(20 μg)** | 322.51 | 76.62 | 23.4 | 160.35 | 61.4 | 38.6 |
| **(50 μg)** | 0.0 | 0.0 | 100.0 | 0.0 | 0.0 | 100.0 |
| **(100 μg)** | 0.0 | 0.0 | 100.0 | 0.0 | 0.0 | 100.0 |
| **None (control)** | 420.16 | -- | -- | 280.95 | -- | -- |
| **PRE 3** | | | | | | |
| **(20 μg)** | 5.12 | 0.12 | 99.0 | 0.0 | 0.0 | 100.0 |
| **(50 μg)** | 0.0 | 0.0 | 100.0 | 0.0 | 0.0 | 100.0 |
| **(100 μg)** | 0.0 | 0.0 | 100.0 | 0.0 | 0.0 | 100.0 |
| **None (control)** | 323.11 | -- | -- | 286.22 | -- | -- |
| **PRE 4** | | | | | | |
| **(20 μg)** | 184.38 | 57.1 | 42.9 | 152.66 | 53.33 | 46.6 |
| **(50 μg)** | 0.0 | 0.0 | 100.0 | 0.0 | 0.0 | 100.0 |
| **(100 μg)** | 0.0 | 0.0 | 100.0 | 0.0 | 0.0 | 100.0 |

**EXAMPLE *7: In vitro* Toxicity Tests**

*In vitro* cytotoxicity testing of the peptides PRE 3, PRE 4 and PRE 5 (see Table 3) was conducted following the general protocol outlined below (modified from "Fluorimetric DNA assay for cell growth estimation" Rao J, Otto W., Analytical Biochem. 207:186-192, 1992).

The assay was performed in 96 well plates, with 3,000 IMR-32 neuroblastoma cells seeded per well and 8 replicas were performed per treatment. The cells were pretreated with either plain medium and a pinocytic endocytosis reagent (Molecular Probes) or medium containing the PRE under evaluation and the pinocytic endocytosis reagent. The cells were allowed to grow under conventional conditions for 3 days. The DNA content of each well was assessed at 24, 48 and 72 hours using Hoechst reagent according to standard procedures. The fluorescence intensity per well was measured using the plate reader "CytoFluor 2350" from Millipore. Excitation was 360 nm and emission was 460nm. The number of cells is directly correlated to the DNA content.

The results are shown in Tables 11-13 as the average relative fluorescence intensity measured for the 8 replica wells, as well as the percentage of survival as compared with matching untreated controls. No cytotoxicity was observed for any of the tested peptides.

**Table 11. IMR-32 Cell Survival after Treatment with PRE 4**

| | 24h | | 48h | | 72h | |
|---|---|---|---|---|---|---|
| | AFI* | % survival | ANI* | % survival | AFI* | % survival |
| **Untreated Control Cells** | 124 | 100 | 266 | 100 | 915 | 100 |
| **Cells + Pinocytic Endocytosis Reagent** | 169 | 100 | 244 | 100 | 706 | 100 |
| **PRE 4** | | | | | | |
| **2.5 mg/ml** | 179 | 106 | 240 | 99 | 777 | 110 |
| **10 mg/ml** | 173 | 103 | 247 | 101 | 809 | 115 |
| * Average fluorescence intensity | | | | | | |

**Table 12. IMR-32 Cell Survival after Treatment with PRE 3**

| | 24h | | 48h | | 72h | |
|---|---|---|---|---|---|---|
| | **AFI*** | **% survival** | **AFI*** | **% survival** | **AFI*** | **% survival** |
| **Untreated Control Cells** | 152 | 100 | 362 | 100 | 820 | 100 |
| **Cells + Pinocytic Endocytosis Reagent** | 149 | 100 | 260 | 100 | 709 | 100 |
| **PRE 3** | | | | | | |
| **2.5 mg/ml** | 193 | 127 | 389 | 150 | 871 | 123 |
| **10 mg/ml** | 182 | 120 | 334 | 129 | 830 | 117 |
| *Average fluorescence intensity | | | | | | |

**Table 13. Cell Survival IMR32 Cells (3000 cells/100μl,well) after Treatment with PRE 5**

| | 24h | | 48h | | 72h | |
|---|---|---|---|---|---|---|
| | **AFI*** | **% survival** | **AFI*** | **% survival** | **AFI*** | **% survival** |
| **Untreated Control Cells** | 633 | 100 | 929 | 100 | 1286 | 100 |
| **PRE 5** | | | | | | |
| **62.5 μg/ml** | 717 | 113 | 1084 | 117 | 1290 | 100 |
| **125 μg/ml** | 593 | 94 | 991 | 107 | 1200 | 93 |
| **250 μg/ml** | 557 | 88 | 899 | 97 | 1150 | 89 |
| **500 μg/ml** | 492 | 78 | 891 | 96 | 1082 | 84 |

**EXAMPLE 8: Effect of PRE 3 and PRE 4 on the Subcellular Localisation of PKC-α**

Peptides PRE 3 or PRE 4 (10 mg/ml) were introduced into human neuroblastoma cells (IMR-32) by pinocytic endocytosis. The cells were fixed and stained with rabbit PKC-α primary antibody and anti-rabbit Alexa-488 or Alexa-800 secondary antibody.

Figure 1 shows the results for control, untreated cells. In most of the cells PKC-α can be seen to be located in the cytoplasm, around the nucleus and at the plasma membrane (where it becomes activated).

Figure 2 shows the results for cells treated with PRE 4. PKC-α can be seen to have accumulated in the cytoplasm of the treated cells as illustrated by the increased fluorescence intensity when compared to control cells (Figure 1). PRE 4 treatment has thus prevented translocation of PKC-α to the membrane, which will also prevent activation of the enzyme.

Figure 3 shows the results for cells treated with PRE 3. PKC-α can be seen to be located mostly on the membrane (white arrows) or around the nucleus (black arrows), suggesting that PRE 3 does not alter the subcellular localisation of PKC-α.

**EXAMPLE 9: Use of a PRE to Prepare a Targeted PKC-α Inhibitor - PhGal, a Pre-Clinical Drug Candidate for Use in Treating Drug-Resistant Cancer**

A computer model for PKC-α (shown in representative form only in Figure 5) was used to develop a rigorous screening process for peptide "fragments", with the most potent fragments incorporated into the final design of the targeted PKC-α inhibitor PhGα1. PhGαl has a three component structure (shown below). The three components are: (a) a "selector" element (or PRE) specific to PKC-α; (b) a more general "inhibitory" element targeting the catalytic site of PKC-α; and (c) a flexible "linker" element joining these two moieties. The PRE used to prepare PhGα1 was PRE 4 (see Table 3).

**PhGα1:**

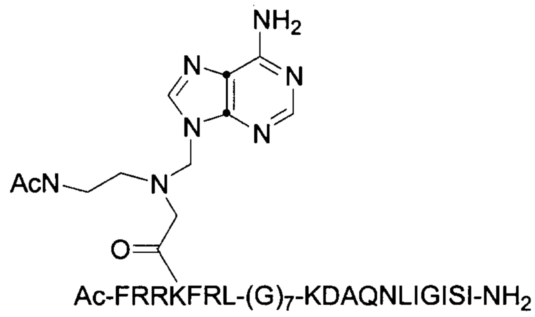

PhGαl selectively targets and inhibits the activity of the PKC-α isoform. Both *in vitro* and *in vivo* data support the efficacy of PhGα1 in limiting the effect of multi-drug resistance (MDR) in colorectal drug-resistant tumours when used in combination treatment with chemotherapy. Specifically, studies have shown that establishment of LS180 colon cancer tumours in mice (M1 stage) treated with PhGαl in combination with an anthracycline chemotherapeutic, doxorubicin, was delayed approximately 100% (p<0.03) versus control cohorts and mice treated with only doxorubicin (28 days compared to 14 days). Results were also observed for tumour transition from the M1 to M2 stage. PhGα 1 also has a good safety profile in toxicity studies conducted in animals.

As shown in Figure 4, the PRE moiety of PhGα1 exhibits a high degree of binding specificity to PKC-α versus other PKC isoforms *in vitro.* Figure 4 presents data from conventional antibody competitive binding assays using PKC-α versus a representative battery of PKC isoforms (commercially available). PKC isoforms representing the three subgroups of the PKC family were assessed (*classical* PKCs = PKCalpha; *novel* PKCs = PKCdelta and epsilon; *atypical* PKCs = PKCzeta). Controls received the appropriate PKC isoform fluorescent antibody. Treated samples received the PRE sequence from PhGα1 (20X concentration) and the same PKC antibody. Specific affinity to PKC-α in the treated sample by the PRE will therefore decrease the amount of binding by the fluorescent antibody, represented by a decrease in fluorescent intensity in the treated sample. As can be seen in Figure 4, a decrease in antibody binding (darker bar) occurs only with the PKC-α sample and not with PKCdelta, epsilon or zeta, indicating specific binding affinity for the alpha isoform.

**EXAMPLE 10: *In vitro* Competitive Binding Assays with Purified PKC Isoforms**

Peptides PRE 1, 2, 3, 4, 5, 6, 7, 8, 9, 10, 11, 12 and 13 (see Table 3) were tested for their ability to interfere with binding of PKC isoform-specific antibodies to their target PKC isoforms using a competitive binding assay and purified PKC isoforms.

***Procedure:*** Competitive binding was assessed using a 96 well plate ELISA-based assay as described below.

Greiner 96 well ELISA plates were coated with the appropriate PKC isoform diluted in PBSN (PBS with Calcium/Magnesium + 0.05% Sodium azide w/w). 50 μL/well at 250 ng/mL was used. Control wells contained PBSN only. Plates were incubated overnight at 4°C.

The plates were washed 3 times with 200 μL of $dH_2O$ per well, then 100 μL of blocking solution was added per well and the plate incubated for 1h at 37°C. The washing steps with $dH_2O$ were repeated and 50 μL of the appropriate PRE solution (PRE stock solution [20mM in DMSO] diluted in PBSN) was added and the plate incubated for 1h at room temperature.

After incubation, 5 μL of 1.1:400 anti-PKC (isoform specific antibody) was added per well (to provide a final concentration of 1:4000). For PKC-epsilon, 1.1:800 dilution was used and for PKC-zeta a 1.1:100 dilution was used due to high and low binding affinity, respectively. The plates were then incubated 1h at room temperature, followed by washing with $dH_2O$ as described above.

50 μL of 1:1000 dilution of Alkaline Phosphatase (AP) conjugated antibody (anti-rabbit for all except zeta, which was anti-goat) was then added and the plate incubated for 1h at room temperature. Washing with $dH_2O$ was then repeated. Finally, 100 μL of pNPP solution in pNPP buffer (2mM $MgC1_2$, 100mM Sodium bicarbonate in water, pH 9.8) was added to all wells and incubated for 8 minutes for α, βI, βII ∈, and ζ, or for 15-20mins for δ (until sufficient coloration is observed). The reaction was stopped by addition of Stop solution (2N NaOH) and the absorbance at 405nm was read on a Galaxy Plate reader.

Controls were PKC without PRE; blank controls, and controls containing the final concentrations of DMSO used for solubilizing the PREs. The non-specific binding capacity related to DMSO was also measured.

The antibodies used were as follows: anti-PKC α (Cat. No. SC-208); anti-PKC βI (Cat. No. SC-209); anti-PKC βII (Cat. No. SC-210); anti-PKC δ (Cat. No. SC-213); anti-PKC ∈ (Cat. No. SC-214); anti-PKC ζ (Cat. No. SC-216-G)

(all from Santa Cruz Biotechnology, Inc.). The AP-conjugated antibodies were obtained from Jackson Immuno Research Laboratories (West Grove, PA).
***Calculations:*** The OD values measured at 405 nm represent the free PKC coated per well.

$$OD_{405}\text{ PKC alone} - \text{Blank } OD_{405}\text{ of each sample} = OD_{405}\text{ coated isoform}$$

$$(OD_{405}\text{ of sample X} / OD_{405}\text{ coated isoform}) \times 100 = \%\, n \text{ that represents the percentage of X binding compared to control.}$$

100- % n measures the relative binding capacity of X towards the tested isoform.
The apparent binding capacity of the DMSO samples was then subtracted from X binding capacity.
***Results:*** The results are shown in Tables 14-19. All measurements were made in triplicate and the values in the table represent the averaged calculated binding capacity values after subtraction of the DMSO apparent binding capacity (averaged from 12 values, respectively 10.80, 11.20 and 16.40 corresponding to the concentrations of DMSO used to dilute the tested isoform at 200, 100 and 50 μM respectively). The results allow for quantitative comparison of the binding capacity of each PRE towards an individual isoform within each table, but not among isoforms due to differences in sensitivity of the specific antibodies toward the secondary antibody. This applies particularly to PKC-delta. As noted above, the colour development duration was increased 2-3 times and, as a result, the $OD_{405}$ measurements may be overestimated for this isoform.

**Table 14: Competitive Binding Assay with PKC-α**

| | Concentration / μm | | |
|---|---|---|---|
| **PRE** | **200** | **100** | **50** |
| **PRE 1** | 2.10 | 0 | 0.69 |
| **PRE 4** | 22.8 | 21.9 | 6.46 |
| **PRE 6** | 0 | 0 | 0 |
| **PRE 3** | 22.61 | 26.5 | 17.47 |
| **PRE 7** | 6.59 | 6.72 | 0 |
| **PRE 8** | 0 | 0 | 0 |
| **PRE 9** | 0 | 5.35 | 0 |
| **PRE 10** | 0 | 0 | 0 |
| **PRE 11** | 13.3 | 5.96 | 0 |
| **PRE 12** | 0 | 5.16 | 3.29 |
| **PRE 13** | 0 | 0 | 0 |
| **PRE 5** | 0 | 0 | 0 |

**Table 15: Competitive Binding Assay with PKC-βI**

| | Concentration / μm | | |
|---|---|---|---|
| **PRE** | **200** | **100** | **50** |
| **PRE 1** | 13.41 | 13.93 | 19.83 |
| **PRE 4** | 7.46 | 15.71 | 6.02 |
| **PRE 6** | 6.14 | 7.93 | 0 |
| **PRE 3** | 23.04 | 22.49 | 26.01 |
| **PRE 7** | 0 | 0 | 2.10 |

(continued)

| PRE | Concentration / μm | | |
|---|---|---|---|
| PRE | 200 | 100 | 50 |
| PRE 8 | 0 | 0 | 0 |
| PRE 9 | 5.72 | 8.82 | 11.53 |
| PRE 10 | 0 | 4.35 | 3.77 |
| PRE 11 | 12.5 | 14.23 | 9.86 |
| PRE 12 | 0 | 0 | 0.79 |
| PRE 13 | 0 | 0 | 0 |
| PRE 5 | 0 | 0 | 0 |

**Table 16: Competitive Binding Assay with PKC-βII**

| PRE | Concentration / μm | | |
|---|---|---|---|
| PRE | 200 | 100 | 50 |
| PRE 1 | 0 | 0 | 0 |
| PRE 4 | 1.18 | 13.53 | 2.69 |
| PRE 6 | 0 | 0 | 0.08 |
| PRE 3 | 3.72 | 0 | 0 |
| PRE 7 | 2.59 | 11.07 | 5.42 |
| PRE 8 | 0 | 0 | 1.11 |
| PRE 9 | 0 | 0.3 | 8.14 |
| PRE 10 | 0 | 0 | 0 |
| PRE 11 | 6.40 | 11.04 | 7.14 |
| PRE 12 | 0 | 0 | 4.55 |
| PRE 13 | 0 | 1.63 | 0 |
| PRE 5 | 0 | 0 | 0 |

**Table 17: Competitive Binding Assay with PKC-δ**

| PRE | Concentration / μM | | |
|---|---|---|---|
| PRE | 200 | 100 | 50 |
| PRE 1 | 49.43 | 45.20 | 35.71 |
| PRE 4 | 17.31 | 30.7 | 29.25 |
| PRE 6 | 13.96 | 13.15 | 17.46 |
| PRE 3 | 57.01 | 56.01 | 48.27 |
| PRE 7 | 0 | 0 | 3.06 |
| PRE 8 | 6.68 | 3.38 | 6.96 |
| PRE 9 | 9.95 | 19.27 | 16.23 |
| PRE 10 | 9.47 | 0 | 0 |
| PRE 11 | 18.74 | 21.48 | 34.06 |

(continued)

| PRE | Concentration / μM | | |
|---|---|---|---|
| | 200 | 100 | 50 |
| PRE 12 | 23.49 | 1.02 | 2.26 |
| PRE 13 | 17.85 | 0.79 | 0 |
| PRE 5 | 4.31 | 0 | 0.20 |

**Table 18: Competitive Binding Assay with PKC-ε**

| PRE | Concentration / μm | | |
|---|---|---|---|
| | 200 | 100 | 50 |
| PRE 1 | 0.83 | 5.47 | 19.84 |
| PRE 4 | 2.58 | 0 | 0 |
| PRE 6 | 0 | 0.46 | 2.61 |
| PRE 3 | 30.98 | 31.51 | 33.99 |
| PRE 7 | 3.78 | 13.99 | 8.04 |
| PRE 8 | 0 | 0 | 0 |
| PRE 9 | 0 | 8.53 | 16.35 |
| PRE 10 | 1.75 | 0 | 0.91 |
| PRE 11 | 9.84 | 13.02 | 2.25 |
| PRE 12 | 0 | 0.28 | 10.44 |
| PRE 13 | 0.85 | 0 | 0 |
| PRE 5 | 1.80 | 0 | 0 |

**Table 19: Competitive Binding Assay with PKC-ζ**

| PRE | Concentration / μM | | |
|---|---|---|---|
| | 200 | 100 | 50 |
| PRE 1 | 0 | 0 | 4.11 |
| PRE 4 | 5.85 | 0 | 0 |
| PRE 6 | 0 | 0 | 0 |
| PRE 3 | 29.15 | 23.94 | 14.00 |
| PRE 7 | 1.54 | 0 | 1.83 |
| PRE 8 | 0 | 0 | 0 |
| PRE 9 | 0 | 0 | 0 |
| PRE 10 | 0 | 0 | 0 |
| PRE 11 | 0 | 0 | 0 |
| PRE 12 | 0.33 | 8.77 | 4.20 |
| PRE 13 | 4.29 | 0 | 0 |
| PRE 5 | 0 | 0 | 0 |

As can be seen from Tables 14-19 above, PKC-α is targeted most strongly by PRE 3 and PRE 4; PKC-βI is

targeted most strongly by PRE 1 and PRE 3; PKC-βII is targeted most strongly by PRE 9 (at 50μM); PKC-δ is targeted most strongly by PRE 1, PRE 3, PRE 11 and PRE 4; PKC-ε is targeted most strongly by PRE 3, and PKC-ζ is targeted by PRE 3 only.

PRE 4 demonstrates specificity for PKC-α with the exception of some possible affinity for PKC-δ, which may be overestimated for reasons outlined above.

PRE 3 appears to be a "universal" PKC targeting peptide, with the exception of the PKC-βII isoform. This is of interest since the discrimination between the two isoforms PKC-βI and PKC-βII is traditionally difficult because they result from alternative splicing.

## EXAMPLE 11: *In vitro* Competitive Binding Assays using Cell Extracts

Binding efficiency and specificity of the peptides PRE 2, 3, 4, 5, 6, 7, 8, 9, 10, 11, 12 and 13 (see Table 3) was also tested using cytoplasmic extracts from different cell lines expressing appropriate PKC isoforms as described for Example 1. Briefly, the protein cytoplasmic extracts were separated by electrophoresis and transferred onto nitrocellulose membranes by standard Western blotting procedures. The bands on the Western blots were detected with matching primary antibodies and alkaline phosphatase conjugated secondary antibody. The bands were then scanned and the relative density measurements obtained.

This procedure to assess the binding characteristics of the PREs is based on competition binding of the PRE with the primary anti-isoform PKC specific antibodies. The lower the measured band density, the greater the binding of the PRE to the PKC isoform. The PREs were added at 10 and 20 times (10X, 20X) the primary antibody concentration, according to classical competition between antigenic peptide and primary antibody. The following cell lines (obtained from the ATCC) were used: H661 - NCI human lung carcinoma NSCLC; MDAMB231 - human highly invasive breast cancer cell line from pleural effusion; LS180 - human colon adenocarcinoma; LnCAP - human prostate adenocarcinoma; CCD16Lu - human lung fibroblasts; Du145 - human prostate carcinoma brain metastasis, and T24 - human bladder carcinoma.

The results are shown in Figures 6-17. As can be seen from the Figures, the controls (without PRE) were sometimes lower in density than the 10X challenged protein bands. In these cases, the 20X band density figures relative to control and 10X values were reliable. The results are also summarised in Table 20 below.

Figure 6 shows the effect of PRE 1 on (A) PKC-α in H661, MDAMB231 and LS180 cells; (B) PKC-βI in H661, MDAMB231 and LS180 cells, (C) PKC-δ in H661, MDAMB231 and LS180 cells, (D) PKC-τ in MDAMB231 and LnCAP cells and (E) PKC-ζ in MDAMB231, LnCAP and Du-145 cells.

Figure 7 shows the effect of PRE 4 on (A) PKC-α, in H661, MDAMB231 and LS180 cells; (B) PKC-βI (first band on Western blot) in H661, MDAMB231 and LS180 cells, (C) PKC-βI (second band on Western blot) in H661, MDAMB231 and LS180 cells, (D) PKC-βII (catalytic fragment) in H661, MDAMB231 and LS180 cells, (E) PKC-δ in H661, MDAMB231 and LS180 cells, (F) PKC-ε in CCD16, LnCAP and Du-145 cells, (G) PKC-ι in H661, CCD16 and LnCAP cells and (H) PKC-ζ in H661, CCD16 and LnCAP cells.

Figure 8 shows the effect of PRE 6 on (A) PKC-α in H661, MDAMB231 and LS180 cells; (B) PKC-βI in H661, MDAMB231 and LS180 cells, (C) PKC-δ in H661, MDAMB231 and LS180 cells, (D) PKC-ε in CCD16, LnCAP and Du-145 cells, (E) PKC-τ in H661, CCD16 and LnCAP cells and (F) PKC-ζ in H661, CCD16 and LnCAP cells.

Figure 9 shows the effect of PRE 3 on (A) PKC-α in H661, MDAMB231 and LS180 cells; (B) PKC-βI in H661, MDAMB231 and LS180 cells, (C) PKC-βII in H661, MDAMB231 and LS180 cells, (D) PKC-δ in H661, MDAMB231 and LS180 cells, (E) PKC-ε (Band 1 in Western blot) in MDAMB231, LnCAP and Du-145 cells, (F) PKC-ε (Band 2 in Western blot) in MDAMB231, LnCAP and Du-145 cells, (G) PKC- ι in MDAMB231, LnCAP and Du-145 cells and (E) PKC-ζ in MDAMB231, LnCAP and Du-145 cells.

Figure 10 shows the effect of PRE 7 on (A) PKC-α in H661, MDAMB231 and LS 180 cells; (B) PKC-βI in H661, MDAMB231 and LS180 cells, (C) PKC-δ in H661, MDAMB231 and LS180 cells, (D) PKC-ε in MDAMB231, LnCAP and Du-145 cells, (E) PKC-ι in CCD16, LnCAP and Du-145 cells and (E) PKC-ζ in MDAMB231, LnCAP and Du-145 cells.

Figure 11 shows the effect of PRE 8 on (A) PKC-α in H661, MDAMB231 and LS180 cells; (B) PKC-βII in H661, MDAMB231 and LS180 cells, (C) PKC-βI in H661, MDAMB231 and LS180 cells and (D) PKC-ε in CCD16, MDAMB231 and Du-145 cells.

Figure 12 shows the effect of PRE 9 on (A) PKC-α in H661, MDAMB231 and LS180 cells; (B) PKC-βI in H661, MDAMB231 and LS180 cells, (C) PKC-βII in H661, MDAMB231 and LS 180 cells, (D) PKC-δ in H661, MDAMB231 and LS 180 cells, (E) PKC-ε in MDAMB231, LnCAP and Du-145 cells, and (F) PKC-ζ in MDAMB231, LnCAP and Du-145 cells.

Figure 13 shows the effect of PRE 10 on (A) PKC-α in H661, MDAMB231 and LS180 cells; (B) PKC-βI in H661, MDAMB231 and LS180 cells, (C) PKC-βII (catalytic fragment) in H661, MDAMB231 and LS180 cells, (D) PKC-δ in H661, MDAMB231 and LS180 cells, (E) PKC-ε in MDAMB231, LnCAP and Du-145 cells, and (F) PKC-ζ in MDAMB231, LnCAP and Du-145 cells.

Figure 14 shows the effect of PRE 11 on (A) PKC-α in H661, T24 and LS180 cells; (B) PKC-βI in H661, T24

and LS 180 cells, (C) PKC-δ in H661, T24 and LS 180 cells, (D) PKC-ε in MDAMB231, LnCAP and Du-145 cells, and (E) PKC-ζ in MDAMB231, LnCAP and Du-145 cells.

Figure 15 shows the effect of PRE 12 on (A) PKC-α in H661, MDAMB231 and LS180 cells; (B) PKC-βI in H661, MDAMB231 and LS180 cells, (C) PKC-βII (catalytic fragment) in H661, MDAMB231 and LS180 cells, (D) PKC-δ in H661, MDAMB231 and LS 180 cells, (E) PKC-ε in MDAMB231, LnCAP and LS 180 cells, (F) PKC-ɂ in MDAMB231, LnCAP and LS 180 cells and (G) PKC-ζ in MDAMB231, LnCAP and Du-145 cells.

Figure 16 shows the effect of PRE 13 on (A) PKC-α in H661, MDAMB231 and LS180 cells; (B) PKC-βI in H661, MDAMB231 and LS180 cells, (C) PKC-δ in H661, MDAMB231 and LS180 cells, (D) PKC-τ in MDAMB231, LnCAP and LS180 cells, (E) PKC-ζ in MDAMB231, LnCAP and Du-145 cells, and (F) PKC-ε in MDAMB231, LnCAP and LS180 cells.

Figure 17 shows the effect of PRE 5 on (A) PKC-α in H661, MDAMB231 and LS180 cells; (B) PKC-βI in H661, T24 and LS180 cells, (C) PKC-βII in H661, T24 and LS180 cells, (D) PKC-δ in H661, T24 and LS180 cells, (E) PKC-ε in MDAMB231, LnCAP and Du-145 cells, and (F) PKC-ζ in MDAMB231, LnCAP and Du-145 cells.

The results obtained in this Example with cell lysates correlate in many respects with the results obtained using the purified isoforms in Example 10. Notably, PRE 4 is shown in this Example to have a strong affinity for PKC-α, as was the case with the purified isoform results in Example 10. Some discrepancies, however, also occur. Specfically, using cell extracts, PRE 4 showed a good affinity for PKC-βI and none for PKC-δ, whereas using purified isoforms, PRE 4 showed an affinity for PKC-δ. This inconsistency may originate from the fact that the enzymes were denatured and linearized on the Western blots, while the purified enzymes retained their 3-dimensional configurations. In addition, the relative concentrations of the enzymes versus the PRE concentrations can be better controlled when purified enzymes are used and a difference in this enzyme:PRE ratio between the two techniques may introduce differences in the sensitivity of the experiments. Finally, there may be some binding of the PREs to unknown proteins which are present but undetected on the Western blots and which are expressed differently in selective cell lines.

The results of the two sets of experiments reported in Examples 10 and 11 are summarised in Table 20.

**Table 20: Affinity of PRE 2-13 for Various PKC Isoforms***

| | | PKC Isoform | | | | | | |
|---|---|---|---|---|---|---|---|---|
| | | α | βI | βII | δ | ε | ɂ | ζ |
| **PRE 1** | Purified isoform | - | H | - | H | - | ND | - |
| | Cell extract | H | H | ND | - | ND | - | L |
| **PRE 4** | Purified isoform | H | L | L | H | - | ND | - |
| | Cell extract | H | H | L | - | H/L | M/L | - |
| **PRE 6** | Purified isoform | - | L | - | L | - | ND | - |
| | Cell extract | H/L | H | ND | M/H | M | - | - |
| **PRE 3** | Purified isoform | H | H | - | H | H | ND | H |
| | Cell extract | H | L | L | H | L | L | - |
| **PRE 7** | Purified isoform | L | - | - | - | L | ND | - |
| | Cell extract | L | - | ND | L | - | - | - |
| **PRE 8** | Purified isoform | - | - | - | - | - | ND | - |
| | Cell extract | M/L | ND | H | L | L | ND | ND |
| **PRE 9** | Purified isoform | - | L | H | L | L | ND | - |
| | Cell extract | M/L | M | - | H | M/L | ND | M/L |
| **PRE 10** | Purified isoform | - | - | L | - | - | ND | - |
| | Cell extract | H/L | M | - | H | MIL | ND | M/L |
| **PRE 11** | Purified isoform | L | - | - | H | L | ND | - |
| | Cell extract | H/L | H | ND | - | - | ND | - |
| **PRE 12** | Purified isoform | - | - | - | L | - | ND | - |
| | Cell extract | - | - | L | - | - | - | M/H |
| **PRE 13** | Purified isoform | - | - | - | L | - | ND | - |
| | Cell extract | M/H | - | ND | - | - | - | - |
| **PRE 5** | Purified isoform | - | - | - | L | - | ND | - |

(continued)

| | PKC Isoform | | | | | | |
|---|---|---|---|---|---|---|---|
| | α | βI | βII | δ | ε | ɂ | ζ |
| Cell extract | M | H | H | - | L | ND | L/H |

*** Legend:**
- = no detectable affinity
L = Low to very low affinity at 10X or 20X or both PRE concentrations in at least one cell line in the cell extract assay; Low affinity in purified isoform assay.
M = Moderate affinity at 10X or 20X or both PRE concentrations in at least one cell line in the cell extract assay.
H = High affinity at 10X or 20X or both PRE concentrations in at least one cell line in the cell extract assay; High affinity in purified isoform assay.
ND = not determined.

**EXAMPLE 12: *In vitro* Inhibition of PKC-α Activity by PRE 4**

The ability of peptide PRE 4 (see Table 3) to inhibit the activity of PKC-α was tested using increasing concentrations of PRE 4 and the Kinase-Glo™ Luminescent Kinase Assay (Promega, Cat # V6712/3/4) following the manufacturer's instructions. Briefly, the assay is based on the Luciferase luminescent reaction with ATP. The substrate was myelin basic protein (MBP, a classical substrate of the PKC family). ATP was added to the PKC-α reaction solution at a given concentration and the remaining ATP was measured by luminescence. Luminescence of blank solutions, ATP provided at the beginning of the reaction and PKC-α activity in the presence or absence of PRE 4 were measured. The presence of PRE 4 did not alter the luminescence of the ATP.

**Calculations:**

Blanks are subtracted from all values.

ATP remaining in all reactions was subtracted from the ATP values at the start = relative enzyme activity (Ea). This is an indirect measurement of the enzyme activity: the more ATP remaining the less active the enzyme.

The relative activities were all measured and the Ea in the presence of PRE 4 are reported relative to the Ea of PKC-α alone taken as 100. The difference between 100 and the % Ea of PKC-α exposed to PRE 4 represents the relative level of inhibition.

The results of three sets of experiments are shown in Table 21. Although the first set of experiments shows a lower degree of inhibition, the other two sets show similar levels of inhibition of PKC-α activity in the presence of PRE 4. The results suggest that PRE 4 binds PKC-α at a site that inhibits its activity. The inhibition is likely to be of an allosteric type.

**Table 21: Inhibition of PKC-α Activity by PRE 4**

| Concentration of PRE 4 / μM | % Inhibition | | |
|---|---|---|---|
| | Experiment 1 | Experiment 2 | Experiment 3 |
| 150 | 6.15 | 28.9 | 40.1 |
| 300 | 9.76 | 40.66 | 35.53 |
| 600 | 16.37 | 53.48 | 41.56 |

**EXAMPLE 13: Cloning and Expression of PKC-α V5 Domain**

A portion of the V5 variable domain of PKC-α was cloned and expressed in neuroblastoma IMR-32 cells as outlined below.

V5E sequence amino-acid sequence (corresponding to amino acid residues 652-672 of the PKC-α sequence): DFEGFSYVNPQFVHPILQSAV [SEQ ID NO:36]

V5E sequence nucleotide sequence (corresponding to nucleotides 1998-2063 of the PKC-α coding sequence):

GAT TTT GAA GGG TTC TCG TAT GTC AAC CCC CAG TTT GTG CAC CCC ATC TTA CAG AGT GCA GTA TGA [SEQ ID NO:37]

Sense Primer:
GGGGACAAGTTTGTACAAAAAAGCAGGCTTGGATTTTGAAGGGTTCTCG [SEQ ID N0:38]
Antisense Primer:
GGGGACCACTTTGTACAAGAAAGCTGGGTTCATACTGCACTCTGTAA [SEQ ID NO:39]

The V5E nucleotide sequence [SEQ ID NO:37] was obtained from MCF-7 RNA by RT-PCR using M-MuLV Reverse Transcriptase (New England Biolabs) following the manufacturer's protocol for first strand synthesis. The reaction product was diluted to 50μL with water and 2μL were used for PCR amplification of the V5E sequence.

**PCR Reactions:**

| Samples | RT product (ul) | $ddH_2O$ (ul) | $Mgc1_2$ (ul) | dNTPs (ul) | Sense Primer (ul) | Antisense Primer (ul) | 10X PCR buffer(ul) | Taq DNA polymerase (ul) |
|---|---|---|---|---|---|---|---|---|
| MCF-7 RT (product) | 2ul | 35 | 0 | 1 | 2 | 2 | 5 | 1 |
| MCF-7 RT (product) | 2ul | 34 | 1 | 1 | 2 | 2 | 5 | 1 |
| MCF-7 RT (product) | 2ul | 33 | 2 | 1 | 2 | 2 | 5 | 1 |

**PCR cycles:**

| | | | |
|---|---|---|---|
| 95°C | Hold (for hot start) | | |
| 95°C | 5 minutes | | |
| 35 cycles at: | | 95°C | 30 seconds |
| | | 56.3°C | 30 seconds |
| | | 72°C | 30 seconds |
| 72°C | 3 minutes | | |
| 4°C | Hold | | |

The PCR product (126bp) was purified from a 1.5% Agarose gel and then cloned into the pDONRT221 plasmid. The presence of the insert was confirmed by AplaI digestion. The insert was sub-cloned in the expression vector pCDNA3.1 and the final construct (pCDNA3.InV-alphaV5E) confirmed by restriction enzyme digestion and sequencing.

The expression construct pCDNA3.1nV-alphaV5E was transfected into neuroblastoma IMR-32 cells following standard protocols and the expression of the V5E peptide confirmed by Western blotting.

**EXAMPLE 14: Binding of PREs to PKC-α in IMR-32 Cells and Effect on Cell Morphology**

Neuroblastoma cells from the IMR-32 cell line were stably transfected with a vector containing the V5E sequence of PKC-α as outlined above in Example 13. The V5E sequence is part of the antigenic amino acid sequence that is used to raise the commercially available anti-PKC-α isoform-specific antibody.

The peptides PRE 1, 2, 3, 4, 5, 6, 7, 8, 9, 10, 11, 12 and 13 (see Table 3) were incorporated into the transfected IMR-32 cells via pinocytic endocytosis (Molecular Probes) according to the supplier's recommendations. Following

fixation and permeabilization, PKC-α was detected using an Alexa fluor secondary antibody (as described above in Example 8).

Results: The expressed V5E fragment adds to the decoration of PKC-α in the transfected IMR-32 cells since it is recognized by the same antibody (compare Figure 20A and 20B). As shown in Figures 18, 19 and 20, the PREs bind to either the V5E fragment or to PKC-α as evidenced by the decrease in the intensity of the label in the PRE-containing cells compared to the controls. The results are summarised in Table 21.

Cell proliferation of control untransfected cells and the transfected IMR-32 cells with or without endocytosis was normal. The transfected IMR-32 cells also have a normal morphology. Incorporation of PRE 4 resulted in a decreased label as expected (see Figure 18C). The label shows as mottled in the cytoplasm and the nucleus. The cells also showed morphological changes with enlarged cells.

In contrast, incorporation of PRE 1 resulted in many dividing cells and abnormal morphology (see Figure 18B). Speckles are visible in the nucleus. Similar trends were found in cells into which PRE 7 and PRE 8 had been incorporated (see Figures 18F and 19B, respectively). Incorporation of PRE 3 resulted in a more intense perinuclear decoration (see Figure 18E).

It is of interest to note that some of the PREs have an activity of their own without showing any substantive decrease in fluorescence. For example, cells incorporating PRE 9 (with a strong perinuclear label; Figure 19C) and cells incorporating PRE 5 or PRE 11, which showed a poor morphology and apoptotis, respectively (see Figures 20D and 19E, respectively). Incorporation of some PREs also resulted in aborted karyokinesis (PRE 5, PRE 6 and PRE 10).

Incorporation of PRE 12 and PRE 13 had little effect on the cell morphology, or on the amount of label (see Figures 19F and 20C, respectively), suggesting that they did not bind substantively to PKC-α and V5E in the IMR-32 cellular environment.

**Table 21: IMR-32 Fluorescence Density Quantification (mean)**

| Peptide | Relative Fluorescence Intensity | Number of Cells |
|---|---|---|
| PRE 1 | 79 | 18 |
| PRE 4 | 79 | 28 |
| PRE 6 | 73 | 30 |
| PRE 3 | 83 | 30 |
| PRE 7 | 75 | 30 |
| PRE 8 | 81 | 30 |
| PRE 9 | 121 | 35 |
| PRE 10 | 122 | 38 |
| PRE 11 | 131 | 36 |
| PRE 12 | 124 | 33 |
| PRE 13 | 116 | 34 |
| PRE 5 | 124 | 34 |
| Transfected IMR-32 | 102 | 32 |
| Transfected IMR-32 + Pinocytic Endocytosis Reagent | 113 | 42 |
| IMR-32 (untransfected) | 88 | 52 |
| IMR-32 (untransfected) + Pinocytic Endocytosis Reagent | 84 | 31 |

The disclosure of all patents, publications, including published patent applications, and database entries referenced in this specification are specifically incorporated by reference in their entirety to the same extent as if each such individual patent, publication, and database entry were specifically and individually indicated to be incorporated by reference.

Although the invention has been described with reference to certain specific embodiments, various modifications thereof will be apparent to those skilled in the art without departing from the spirit and scope of the invention as outlined in the claims appended hereto.

The following numbered paragraphs (paras.) contain statements of broad combinations of the inventive technical features herein disclosed:-

1. A peptide of between about 5 and about 30 amino acid residues in length and having a sequence of general formula (I), or the retro form thereof:

$$X\text{-}[(HY\text{-}HB)_n\text{-linker}]_m\text{-}(HB\text{-}HY)_2\text{-}HB\text{-}(HY)_m\text{-}Z \qquad (I)$$

wherein:

HY represents 1 to 4 amino acid residues selected from the group of: Ala, Gly,Ile, Leu, Phe and Val;
HB represents 1 to 4 amino acid residues selected from the group of: Arg, Asn, Asp, Glu, Gln, Lys and Ser;
"linker" represents 1 to 4 Gly residues;
n is 1,2 or 3;
m is 0 or 1 ;
X represents the N-terminus of the peptide or a modified version thereof; and
Z represents the C-terminus of the peptide or a modified version thereof.

2. The peptide according to paragraph 1, wherein said peptide has a sequence of general formula (II), or the retro form thereof:

$$X\text{-}[(HY\text{-}HB1)_n\text{-linker}]_m\text{-}(HB\text{-}HY)_2\text{-}HB2\text{-}(HY)_m\text{-}Z \qquad (II)$$

wherein:

HB1 represents 1 to 3 amino acid residues selected from the group of: Arg, Asn, Asp, Glu, Gin, Lys and Ser; and
HB2 consists of 1 or 2 amino acid residues selected from the group of: Arg, Asn, Asp, Glu, Gln, Lys and Ser.

3. The peptide according to paragraph 1, wherein said peptide has a sequence of general formula (III), or the retro form thereof:

$$X\text{-}(HB\text{-}HY)_2\text{-}HB2\text{-}(HY)_m\text{-}Z \qquad (III)$$

wherein:

HB2 represents 1 or 2 amino acid residues selected from the group of: Arg, Asn, Asp, Glu, Gin, Lys and Ser.

4. The peptide according to paragraph 1 or 2, wherein said "linker" represents 1 to 3 Gly residues.

5. The peptide according to paragraph 1 or 2, wherein said "linker" represents 1 to 2 Gly residues.

6. The peptide according to any one of paragraphs 1, 2 or 3, wherein said peptide comprises one or more non-naturally-occurring amino acids.

7. The peptide according to any one of paragraphs 1, 2 or 3, wherein said peptide comprises one or more modified peptide bonds.

8. The peptide according to any one of paragraphs 1, 2 or 3, wherein said peptide comprises one or more D-amino acids.

9. The peptide according to paragraph 1, wherein said peptide comprises an amino acid sequence selected from the group of: SEQ ID NO.1, SEQ ID NO:2, SEQ ID NO.3, SEQ ID NO:4, SEQ ID NO:5, SEQ ID NO:6, SEQ ID NO: 7, SEQ ID NO:8, SEQ ID NO:9, SEQ ID NO: 10, SEQ ID NO:26, SEQ ID NO:27, SEQ ID NO:28, SEQ ID NO:29,

SEQ ID NO:30, SEQ ID N0:31, SEQ ID NO:32, SEQ ID NO:33, SEQ ID NO:34 and SEQ ID NO:35, or the retro, inverso, or retro-inverso form thereof.

10. The peptide according to paragraph 1, wherein said peptide comprises an amino acid sequence selected from the group of: SEQ ID NO:1, SEQ ID NO:2, SEQ ID NO:3, SEQ ID NO:4, SEQ ID NO:5, SEQ ID NO:6, SEQ ID NO: 7, SEQ ID NO:8, SEQ ID NO:9, SEQ ID N0:10, SEQ ID N0:11, SEQ ID NO:12, SEQ ID NO:13, SEQ ID NO:14, SEQ ID NO:15, SEQ ID NO:16, SEQ ID NO:17, SEQ ID N0:18, SEQ ID NO:19, SEQ ID NO:20, SEQ ID N0:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID N0:31, SEQ ID NO:32, SEQ ID NO:33, SEQ ID NO:34 and SEQ ID NO:35.

11. The peptide according to paragraph 1 , wherein said peptide comprises an amino acid sequence selected from the group of: SEQ ID N0:1, SEQ ID NO:5, SEQ ID N0:11, SEQ ID NO:12, SEQ ID NO:13, SEQ ID NO:14, SEQ ID NO:16, SEQ ID NO: 17, SEQ ID NO: 18, SEQ ID NO: 19, SEQ ID NO:20, SEQ ID NO:21 and SEQ ID NO:22.

12. The peptide according to paragraph 1, wherein said peptide comprises a sequence as set forth in SEQ ID NO: 2 or SEQ ID N0:13.

13. A composition comprising a peptide according to any one of paragraphs 1, 2, 3, 4, 5, 5, 6, 7, 8, 9, 10, 11 or 12 and a physiologically acceptable diluent, carrier or excipient.

14. A conjugate comprising the peptide according to any one of paragraphs 1, 2, 3, 4, 5, 5, 6, 7, 8, 9, 10, 11 or 12 and a PKC inhibitor.

15. A conjugate comprising the peptide according to any one of paragraphs 1, 2, 3, 4, 5, 5, 6, 7, 8, 9, 10, 11 or 12 and a detectable label.

16. A method of screening for a PKC isoform-specific targeting peptide comprising:

- providing a library of candidate peptides, each peptide having a sequence represented by general formula (I), or the retro form thereof:

$$X\text{-}[(HY\text{-}HB)_n\text{-linker}]_m\text{-}(HB\text{-}HY)_2\text{-}HB\text{-}(HY)_m\text{-}Z \qquad (I)$$

  wherein:

  HY represents 1 to 4 amino acid residues selected from the group of: Ala, Gly,Ile, Leu, Phe and Val;
  HB represents 1 to 4 amino acid residues selected from the group of: Arg, Asn, Asp, Glu, Gln, Lys and Ser;
  "linker" represents 1 to 4 Gly residues;
  n is1,2 or 3;
  mis0or1 ;
  X represents the N-terminus of the peptide or a modified version thereof; and
  Z represents the C-terminus of the peptide or a modified version thereof.

- screening the library to determine the ability of the candidate peptides to bind to a PKC isoform or to reduce the binding of a specific antibody to a PKC isoform, and
- selecting a peptide capable of binding to the PKC isoform or of reducing the binding of a specific antibody to the PKC isoform.

17. A PKC isoform-specific targeting peptide selected by the method according to paragraph 16.

18. A method of screening for the presence of one or more PKC isoforms in a cell comprising contacting said cell with the peptide according to any one of paragraphs 1, 2, 3, 4, 5, 5, 6, 7, 8, 9, 10, 11 or 12 under conditions that permit binding of said peptide to the one or more PKC isoforms to form a peptide-PKC complex, and detecting said peptide-PKC complex.

19. The method according to paragraph 18, wherein said one or more PKC isoforms are selected from the group

of: PKC-alpha, PKC-beta I, PKC-beta II, PKC-delta, PKC-epsilon, PKC-iota and PKC-zeta.

20. The method according to paragraph 18, wherein said one or more PKC isoforms are selected from the group of: PKC-alpha, PKC-beta I, PKC-beta II, PKC-delta and PKC-epsilon.

21. The method according to paragraph 18, wherein said PKC isoform is PKC-alpha.

22. The method according to any one of paragraphs 18, 19, 20 or 21, wherein said method is an *in vitro* method.

23. The method according to any one of paragraphs 18, 19, 20 or 21, wherein said method is an *in vivo* method.

24. A method of targeting a compound to one or more PKC isoform in a cell comprising contacting said cell with a conjugate, said conjugate comprising the compound conjugated to a peptide of any one of paragraphs 1, 2, 3, 4, 5, 5, 6, 7, 8, 9, 10, 11 or 12.

25. The method according to paragraph 24, wherein said one or more PKC isoforms are selected from the group of: PKC-alpha, PKC-beta I, PKC-beta II, PKC-delta, PKC-epsilon, PKC-iota and PKC-zeta.

26. The method according to paragraph 24, wherein said one or more PKC isoforms are selected from the group of: PKC-alpha, PKC-beta I, PKC-beta II, PKC-delta and PKC-epsilon.

27. The method according to paragraph 24, wherein said PKC isoform is PKC-alpha.

28. The method according to any one of paragraphs 24, 25, 26 or 27, wherein said method is an *in vitro* method.

29. The method according to any one of paragraphs 24, 25, 26 or 27, wherein said method is an *in vivo* method.

30. Use of a peptide according to any one of paragraphs 1, 2, 3, 4, 5, 5, 6, 7, 8, 9, 10, 11 or 12 in the preparation of a conjugate, said conjugate comprising a PKC inhibitor or a detectable label conjugated to said peptide.

31. Use of a peptide according to any one of paragraphs 1, 2, 3, 4, 5, 5, 6, 7, 8, 9, 10, 11 or 12 in the manufacture of a medicament.

## Claims

1. A peptide of between about 6 and about 25 amino acid residues in length and having a sequence of general formula (V):

$$X\text{-}(HB\text{-}HY)_2\text{-}HB2\text{-}HY\text{-}Z \qquad \mathbf{(V)}$$

wherein:

HY represents 1 to 4 amino acid residues selected from the group of: Ala, Gly, Ile, Leu, Phe and Val;
HB represents 1 to 4 amino acid residues selected from the group of: Arg, Asn, Asp, Glu, Gln, Lys and Ser;
HB2 represents 1 or 2 amino acid residues selected from the group of: Arg, Asn, Asp, Glu, Gln, Lys and Ser;
X represents the N-terminus of the peptide or a modified version thereof; and
Z represents the C-terminus of the peptide or a modified version thereof.

**2.** The peptide according to claim 1, wherein HB represents 1 to 2 amino acid residues selected from the group of: Arg, Asn, Asp, Glu, Gln, Lys and Ser, or wherein HB is selected from the group of: Arg, Asn, Asp, Glu, Gln and Lys.

**3.** The peptide according to claim 1 or 2, wherein HB2 represents 1 amino acid residue selected from the group of: Arg, Asn, Asp, Glu, Gln, Lys and Ser, or wherein HB2 is selected from the group of: Arg, Lys and Ser.

**4.** The peptide according to any one of claims 1, 2 or 3, wherein HY is selected from the group of: Ala, Gly, Ile, Leu and Val.

**5.** The peptide according to any one of claims 1, 2, 3, or 4, wherein said peptide is between about 6 and about 22 amino acid residues in length.

**6.** The peptide according to any one of claims 1, 2, 3, 4, or 5, wherein said peptide comprises one or more non-naturally-occurring amino acids, or wherein said peptide comprises one or more D-amino acids, or wherein said peptide comprises all D-amino acids..

**7.** The peptide according to any one of claims 1, 2, 3, 4, 5, or 6, wherein said peptide comprises one or more modified peptide bonds.

**8.** The peptide according to any one of claims 1, 2, 3, 4, 5, 6, or 7, wherein X represents a modified version of the N-terminus of the peptide, wherein the N-terminus is optionally modified with an acetyl group.

**9.** The peptide according to any one of claims 1, 2, 3, 4, 5, 6, 7, or 8, wherein Y represents a modified version of the C-terminus of the peptide, wherein the C-terminus is optionally modified with an amino group.

**10.** The peptide according to any one of claims 1, 2, 3, 4, 5, 6, 7, 8, or 9, wherein said peptide comprises: SEQ ID NO: 2, SEQ ID NO:3, SEQ ID NO:6, SEQ ID NO:8, SEQ ID NO:11, SEQ ID NO:13, SEQ ID NO: 15, SEQ ID NO:16, SEQ ID NO:20, SEQ ID NO:27, SEQ ID NO:30, SEQ ID NO:31, SEQ ID NO:32, SEQ ID NO:33, SEQ ID NO:34 or SEQ ID N0:35.

**11.** The peptide according to any one of claims 1, 2, 3, 4, 5, 6, 7, 8, or 9, wherein said peptide comprises SEQ ID N0: 2, SEQ ID NO:13 or SEQ ID NO:15.

**12.** A conjugate comprising the peptide according to any one of claims 1, 2, 3, 4, 5, 6, 7, 8, 9, 10, or 11, conjugated to one or more other chemical moieties.

**13.** The conjugate according to claim 12, wherein said peptide is conjugated to a permeability enhancer, and/or a PKC inhibitor.

**14.** The conjugate according to claim 12 or 13, wherein said peptide is conjugated to one or more additional peptides of any one of claims 1, 2, 3, 4, 5, 6, 7, 8, 9, 10, or 11.

**15.** A composition comprising a peptide according to any one of claims 1, 2, 3, 4, 5, 6, 7, 8, 9, 10, or 11, or the conjugate according to any one or claims 12, 13, or 14, and a physiologically acceptable diluent, carrier or excipient.

**16.** A conjugate according to claim 15 for use to inhibit one or more PKC isoforms.

**17.** The conjugate according to claim 16, wherein said one or more PKC isoforms comprises PKC-alpha.

**18.** A peptide according to any one of claims 1, 2, 3, 4, 5, 6, 7, 8, 9, 10, or 11, or the conjugate according to any one of claims 12, 13, or 14, for use as a medicament.

FIGURE 1

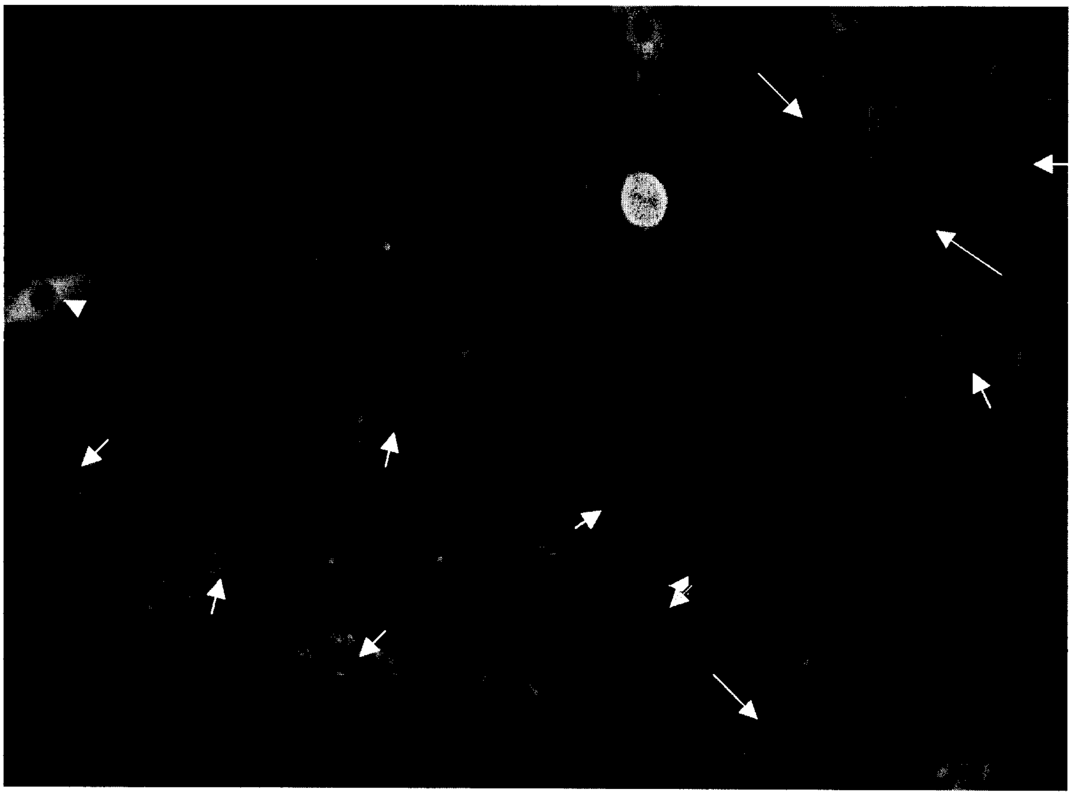

FIGURE 2

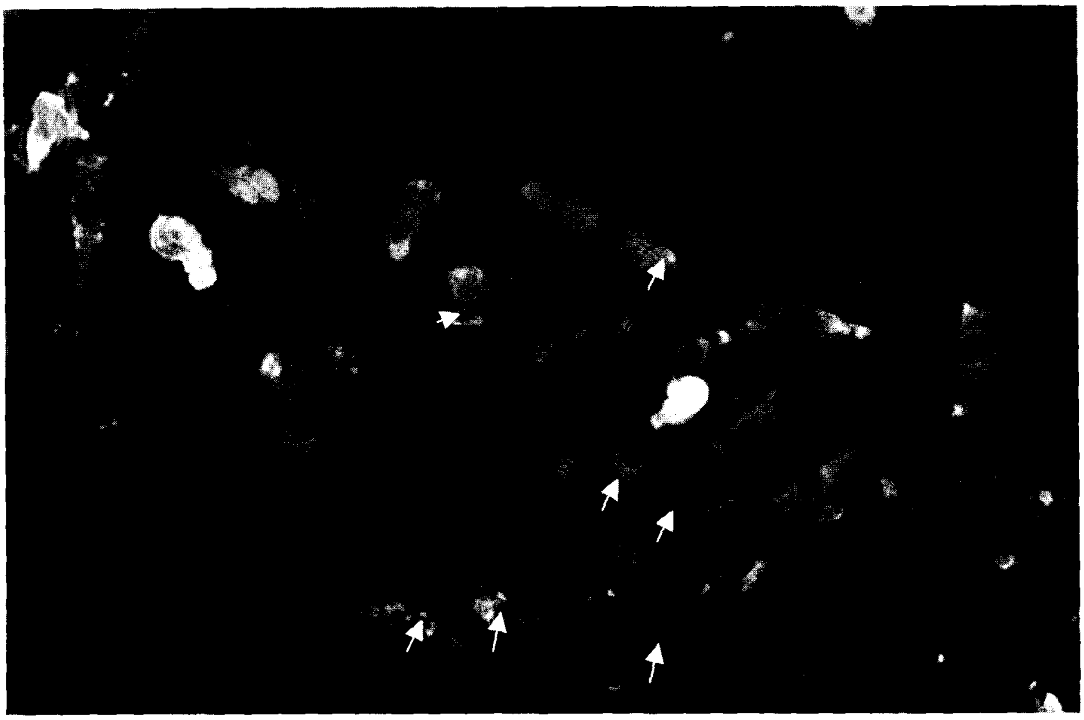

FIGURE 3

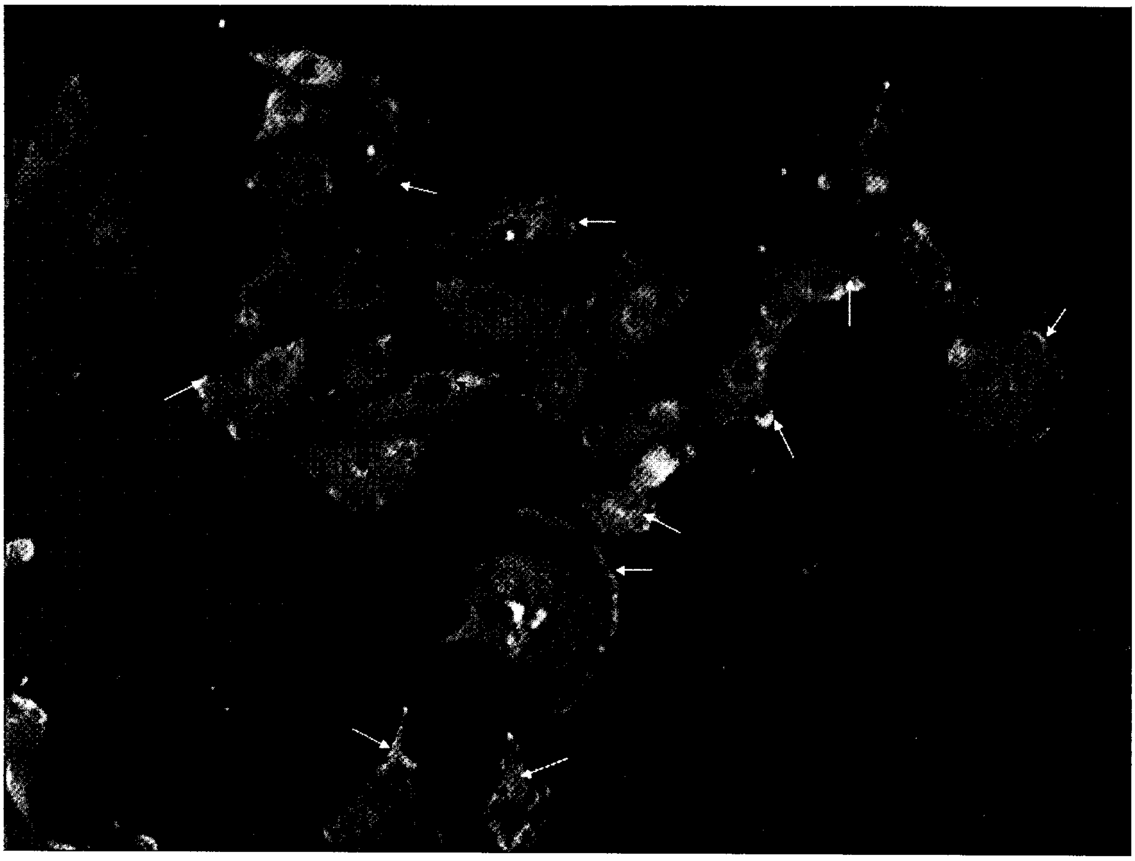

# FIGURE 4

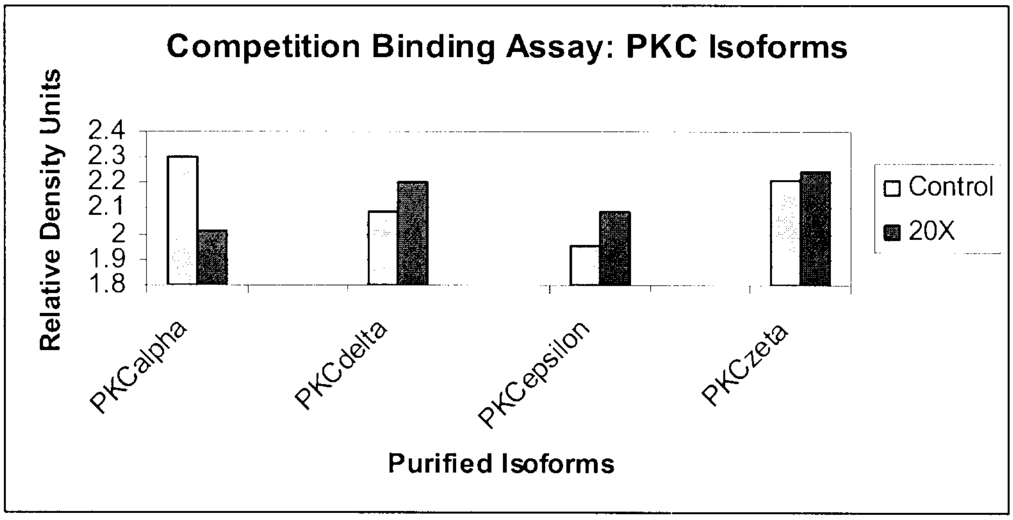
Competition Binding Assay: PKC Isoforms
Relative Density Units
2.4
2.3
2.2
2.1
2
1.9
1.8
PKCalpha
PKCdelta
PKCepsilon
PKCzeta
Purified Isoforms
Control
20X

FIGURE 5

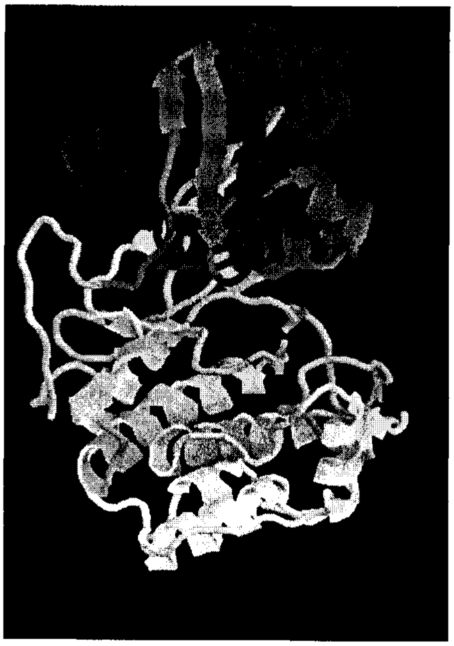

# FIGURE 6

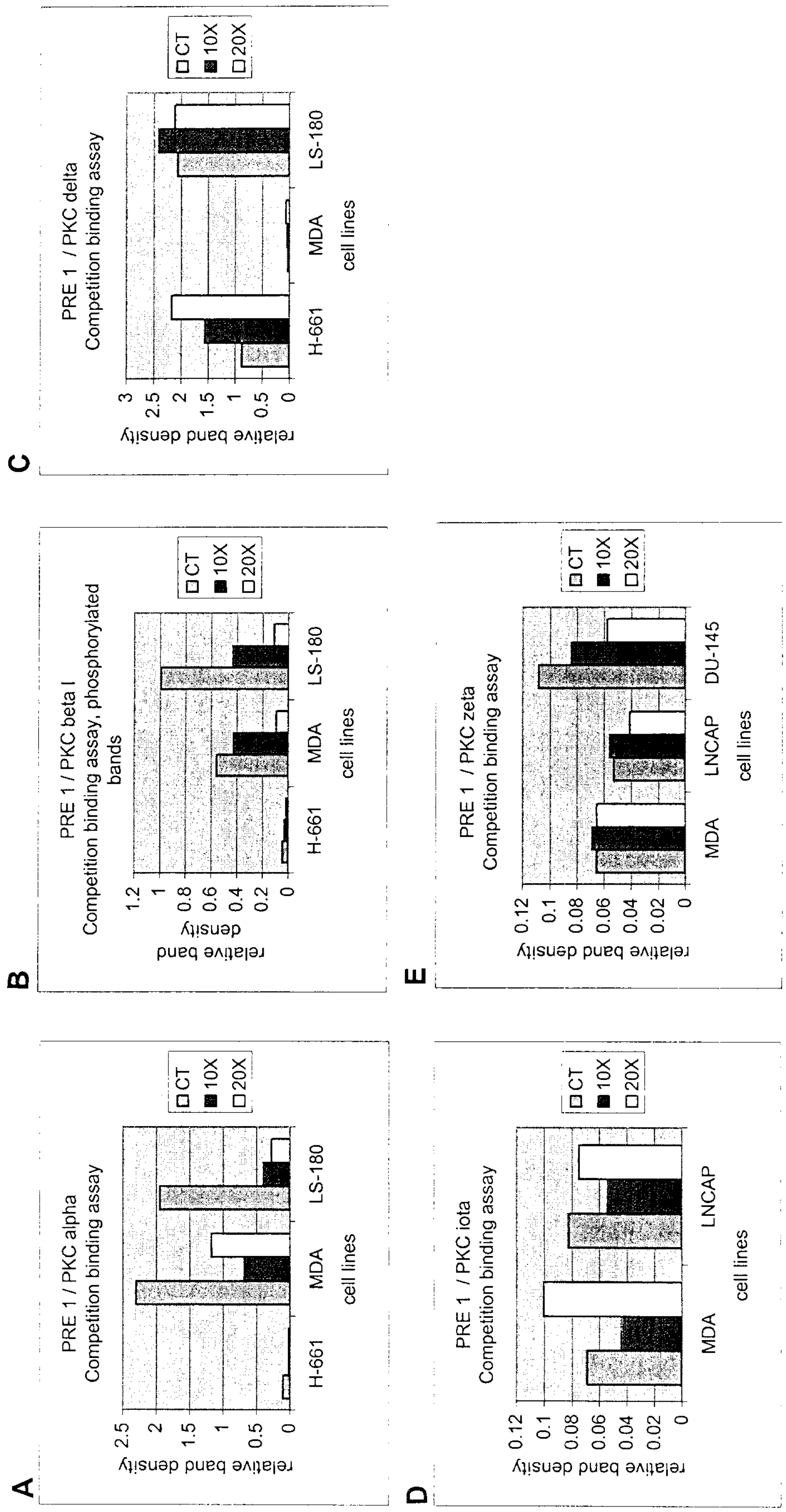
A
PRE 1 / PKC alpha
Competition binding assay
relative band density
cell lines
H-661
MDA
LS-180
CT
10X
20X
B
PRE 1 / PKC beta I
Competition binding assay, phosphorylated bands
relative band density
cell lines
H-661
MDA
LS-180
C
PRE 1 / PKC delta
Competition binding assay
relative band density
cell lines
H-661
MDA
LS-180
D
PRE 1 / PKC iota
Competition binding assay
relative band density
cell lines
MDA
LNCAP
E
PRE 1 / PKC zeta
Competition binding assay
relative band density
cell lines
MDA
LNCAP
DU-145

# FIGURE 7

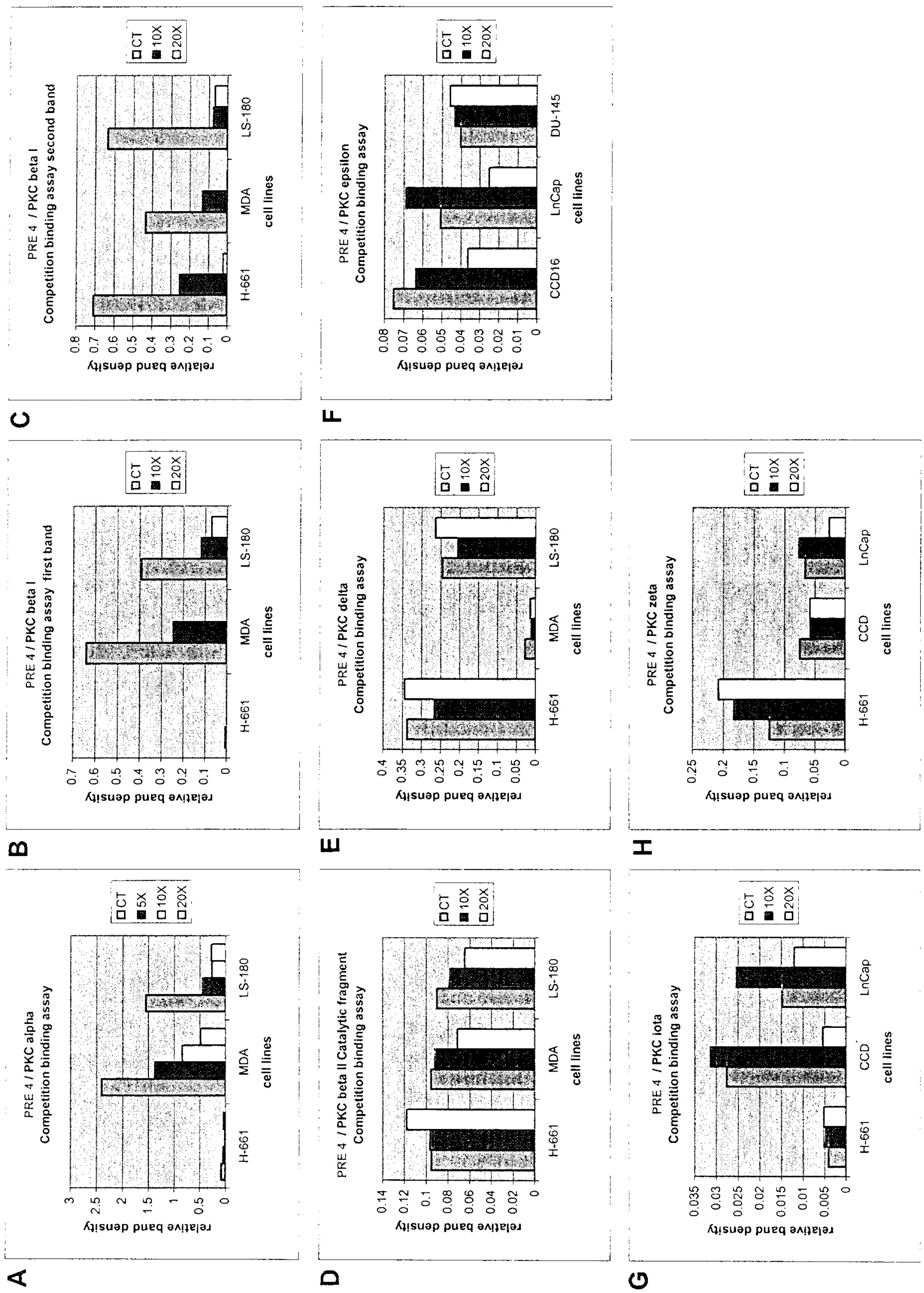
A
PRE 4 / PKC alpha
Competition binding assay
relative band density
cell lines
H-661
MDA
LS-180
CT
5X
10X
20X
B
PRE 4 / PKC beta I
Competition binding assay first band
relative band density
cell lines
H-661
MDA
LS-180
CT
10X
20X
C
PRE 4 / PKC beta I
Competition binding assay second band
relative band density
cell lines
H-661
MDA
LS-180
CT
10X
20X
D
PRE 4 / PKC beta II Catalytic fragment
Competition binding assay
relative band density
cell lines
H-661
MDA
LS-180
CT
10X
20X
E
PRE 4 / PKC delta
Competition binding assay
relative band density
cell lines
H-661
MDA
LS-180
CT
10X
20X
F
PRE 4 / PKC epsilon
Competition binding assay
relative band density
cell lines
CCD16
LnCap
DU-145
CT
10X
20X
G
PRE 4 / PKC iota
Competition binding assay
relative band density
cell lines
H-661
CCD
LnCap
CT
10X
20X
H
PRE 4 / PKC zeta
Competition binding assay
relative band density
cell lines
H-661
CCD
LnCap
CT
10X
20X

# FIGURE 8

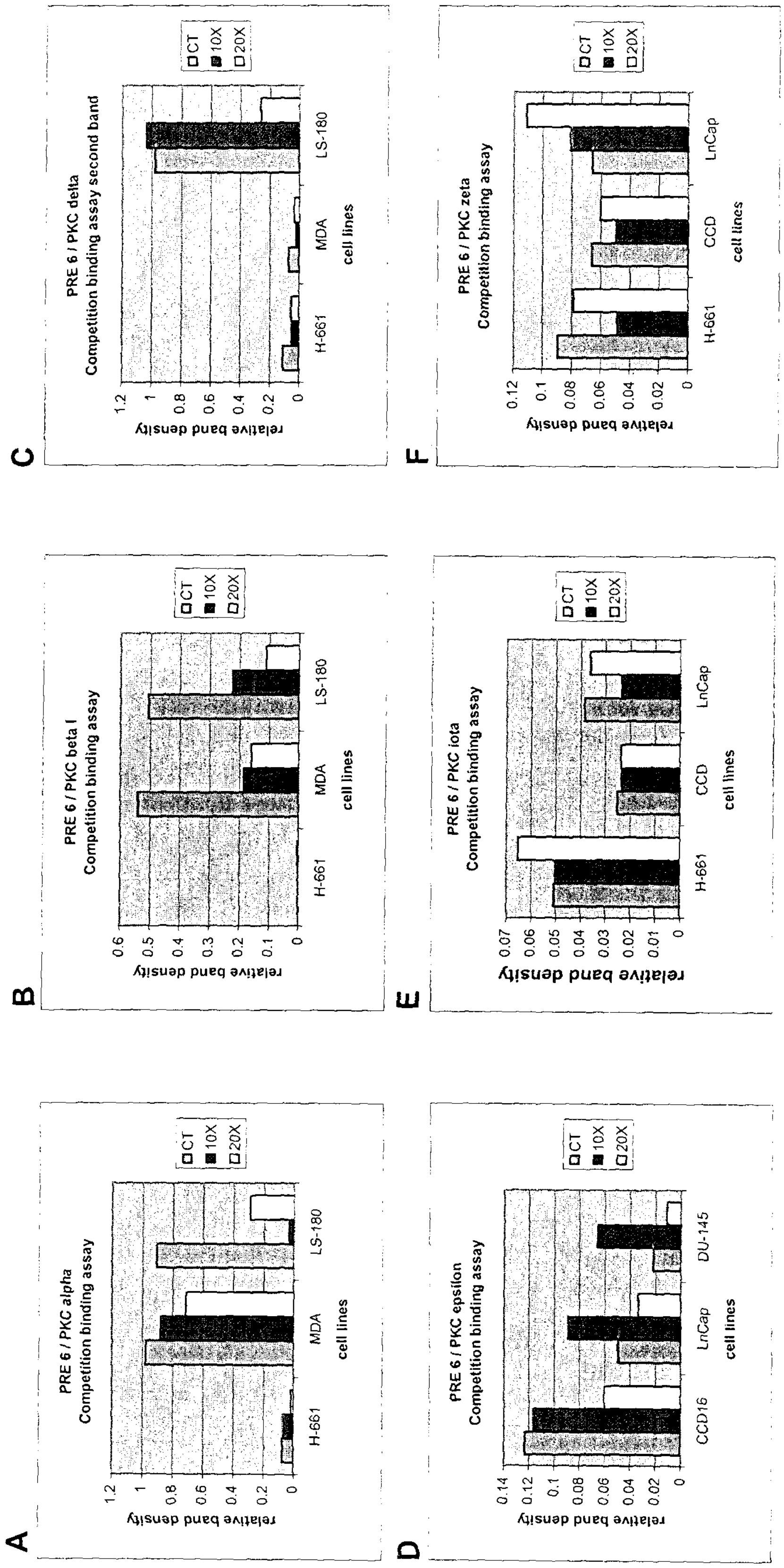

A
PRE 6 / PKC alpha
Competition binding assay
relative band density
cell lines
H-661
MDA
LS-180
CT
10X
20X
B
PRE 6 / PKC beta I
Competition binding assay
relative band density
cell lines
H-661
MDA
LS-180
CT
10X
20X
C
PRE 6 / PKC delta
Competition binding assay second band
relative band density
cell lines
H-661
MDA
LS-180
CT
10X
20X
D
PRE 6 / PKC epsilon
Competition binding assay
relative band density
cell lines
CCD16
LnCap
DU-145
CT
10X
20X
E
PRE 6 / PKC iota
Competition binding assay
relative band density
cell lines
H-661
CCD
LnCap
CT
10X
20X
F
PRE 6 / PKC zeta
Competition binding assay
relative band density
cell lines
H-661
CCD
LnCap
CT
10X
20X

# FIGURE 9

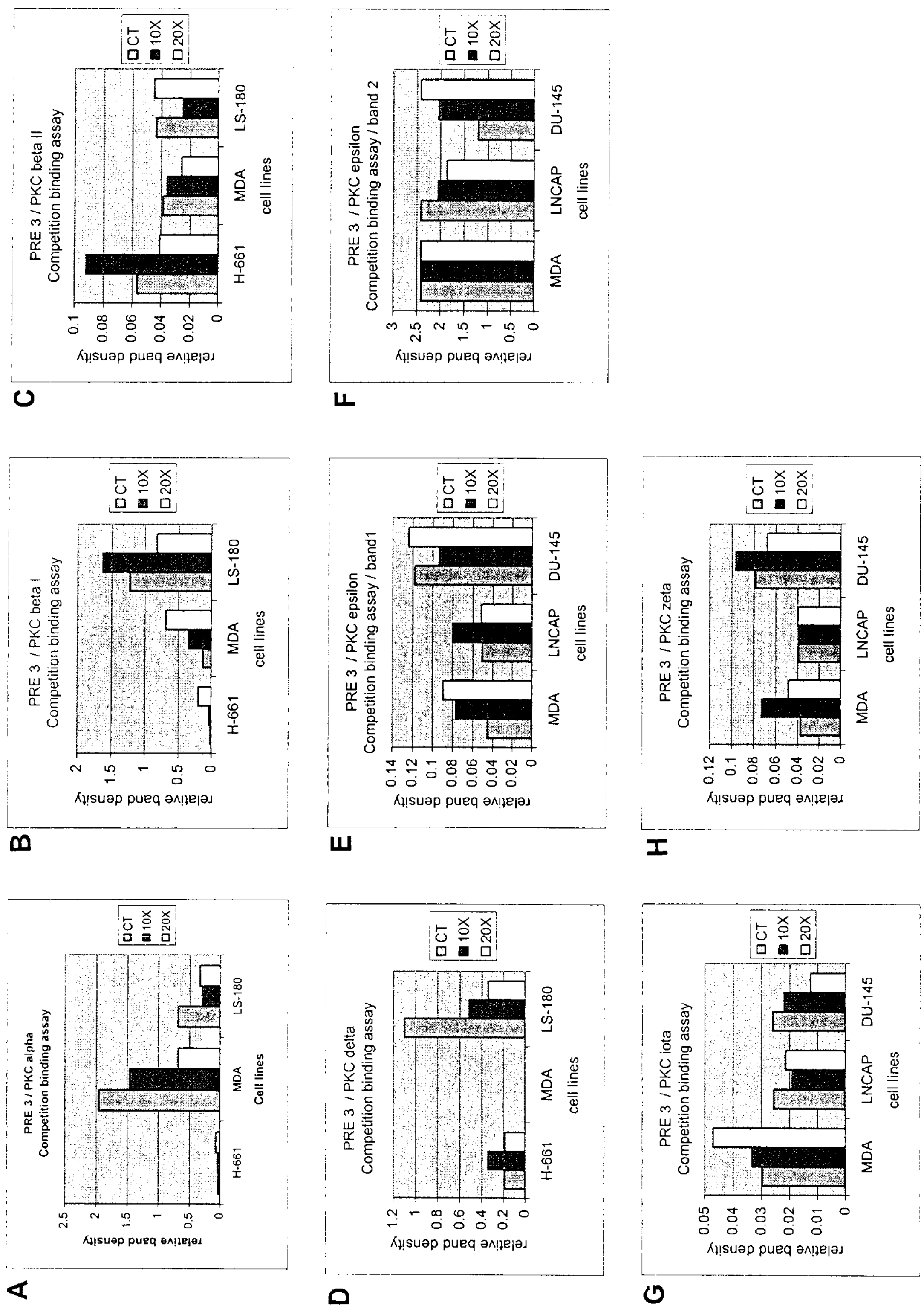

A
PRE 3 / PKC alpha
Competition binding assay
B
PRE 3 / PKC beta I
Competition binding assay
C
PRE 3 / PKC beta II
Competition binding assay
D
PRE 3 / PKC delta
Competition binding assay
E
PRE 3 / PKC epsilon
Competition binding assay / band1
F
PRE 3 / PKC epsilon
Competition binding assay / band 2
G
PRE 3 / PKC iota
Competition binding assay
H
PRE 3 / PKC zeta
Competition binding assay
relative band density
cell lines
H-661
MDA
LS-180
LNCAP
DU-145
CT
10X
20X

# FIGURE 10

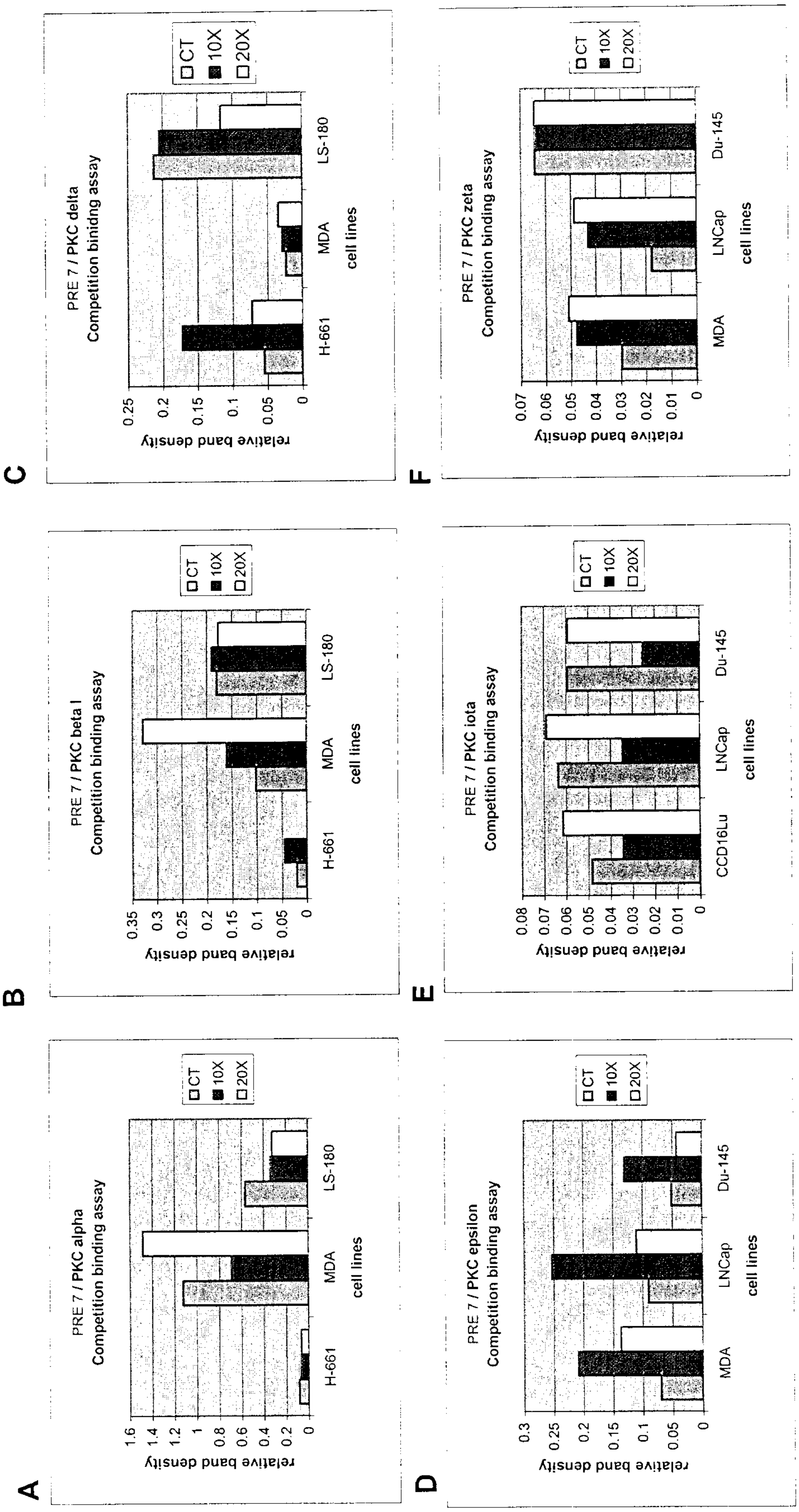
A
PRE 7 / PKC alpha
Competition binding assay
B
PRE 7 / PKC beta I
Competition binding assay
C
PRE 7 / PKC delta
Competition binidng assay
D
PRE 7 / PKC epsilon
Competition binding assay
E
PRE 7 / PKC iota
Competition binding assay
F
PRE 7 / PKC zeta
Competition binding assay
relative band density
cell lines
H-661
MDA
LS-180
LNCap
Du-145
CCD16Lu
CT
10X
20X

# FIGURE 11

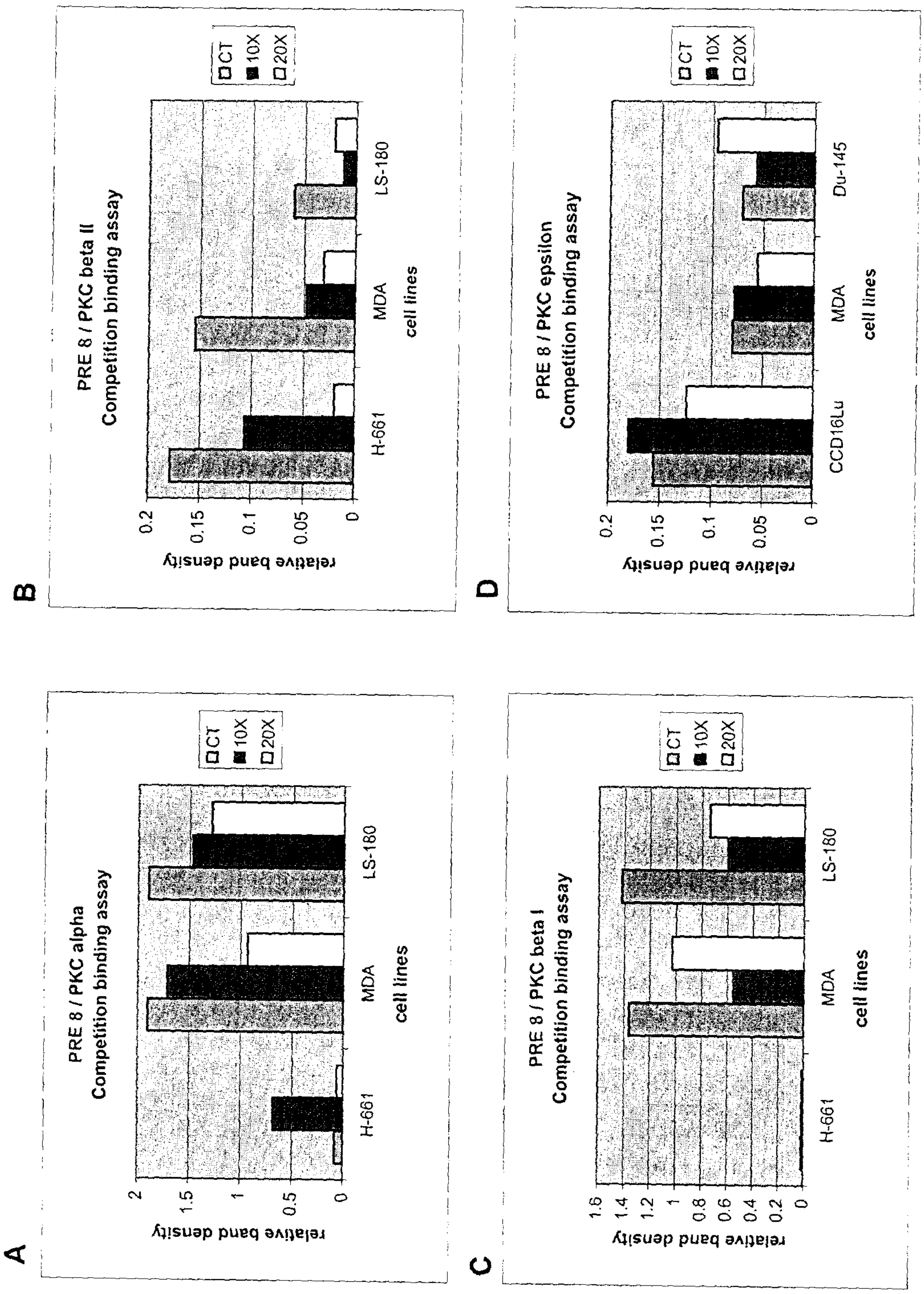
A
PRE 8 / PKC alpha
Competition binding assay
relative band density
2
1.5
1
0.5
0
H-661
MDA
LS-180
cell lines
CT
10X
20X
B
PRE 8 / PKC beta II
Competition binding assay
relative band density
0.2
0.15
0.1
0.05
0
H-661
MDA
LS-180
cell lines
CT
10X
20X
C
PRE 8 / PKC beta I
Competition binding assay
relative band density
1.6
1.4
1.2
1
0.8
0.6
0.4
0.2
0
H-661
MDA
LS-180
cell lines
CT
10X
20X
D
PRE 8 / PKC epsilon
Competition binding assay
relative band density
0.2
0.15
0.1
0.05
0
CCD16Lu
MDA
Du-145
cell lines
CT
10X
20X

# FIGURE 12

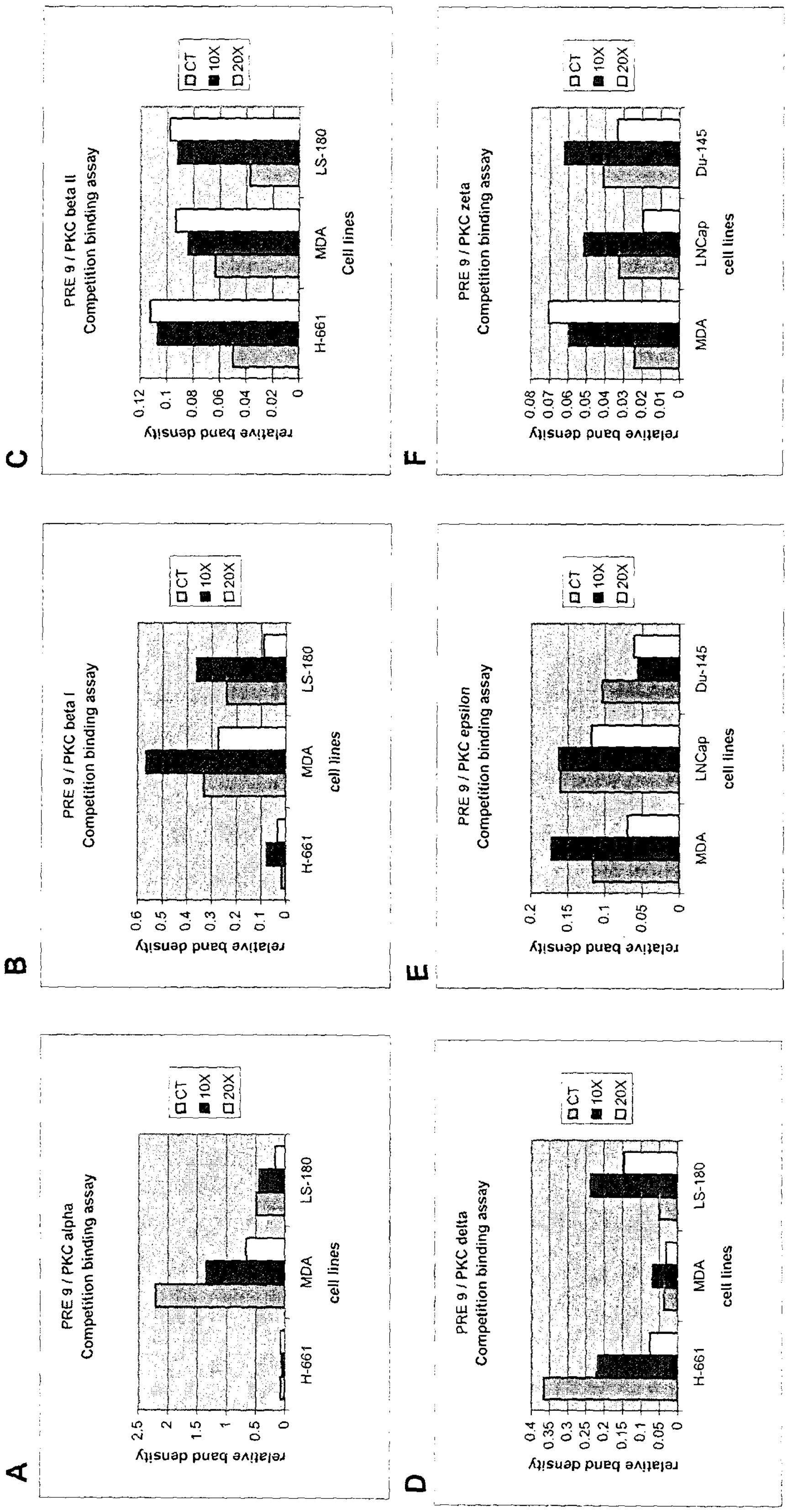
A
PRE 9 / PKC alpha
Competition binding assay
relative band density
2.5
2
1.5
1
0.5
0
H-661
MDA
LS-180
cell lines
CT
10X
20X
B
PRE 9 / PKC beta I
Competition binding assay
relative band density
0.6
0.5
0.4
0.3
0.2
0.1
0
H-661
MDA
LS-180
cell lines
CT
10X
20X
C
PRE 9 / PKC beta II
Competition binding assay
relative band density
0.12
0.1
0.08
0.06
0.04
0.02
0
H-661
MDA
LS-180
Cell lines
CT
10X
20X
D
PRE 9 / PKC delta
Competition binding assay
relative band density
0.4
0.35
0.3
0.25
0.2
0.15
0.1
0.05
0
H-661
MDA
LS-180
cell lines
CT
10X
20X
E
PRE 9 / PKC epsilon
Competition binding assay
relative band density
0.2
0.15
0.1
0.05
0
MDA
LNCap
Du-145
cell lines
CT
10X
20X
F
PRE 9 / PKC zeta
Competition binding assay
relative band density
0.08
0.07
0.06
0.05
0.04
0.03
0.02
0.01
0
MDA
LNCap
Du-145
cell lines
CT
10X
20X

# FIGURE 13

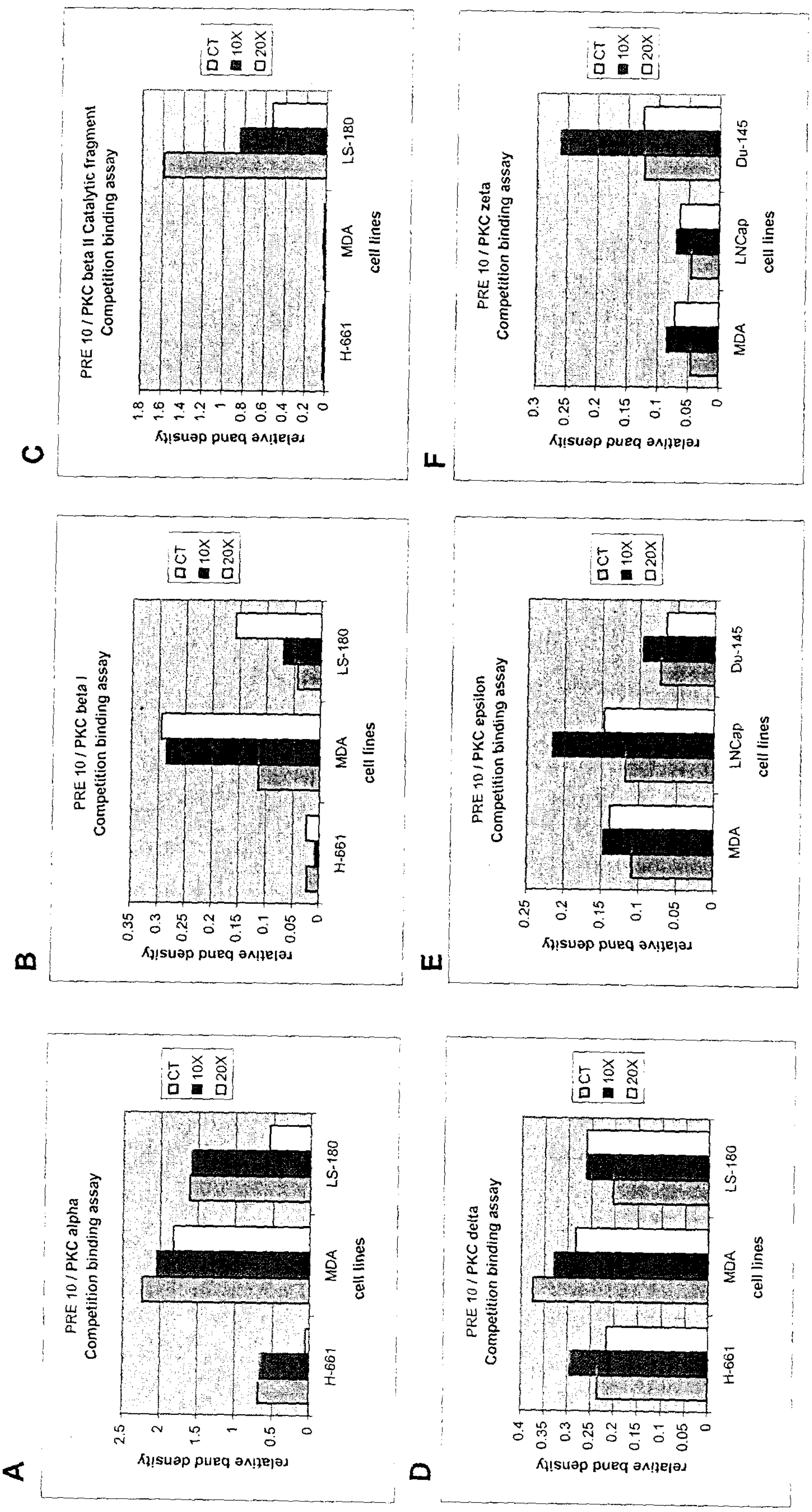
A
PRE 10 / PKC alpha
Competition binding assay
B
PRE 10 / PKC beta I
Competition binding assay
C
PRE 10 / PKC beta II Catalytic fragment
Competition binding assay
D
PRE 10 / PKC delta
Competition binding assay
E
PRE 10 / PKC epsilon
Competition binding assay
F
PRE 10 / PKC zeta
Competition binding assay
relative band density
cell lines
H-661
MDA
LS-180
LNCap
Du-145
CT
10X
20X

# FIGURE 14

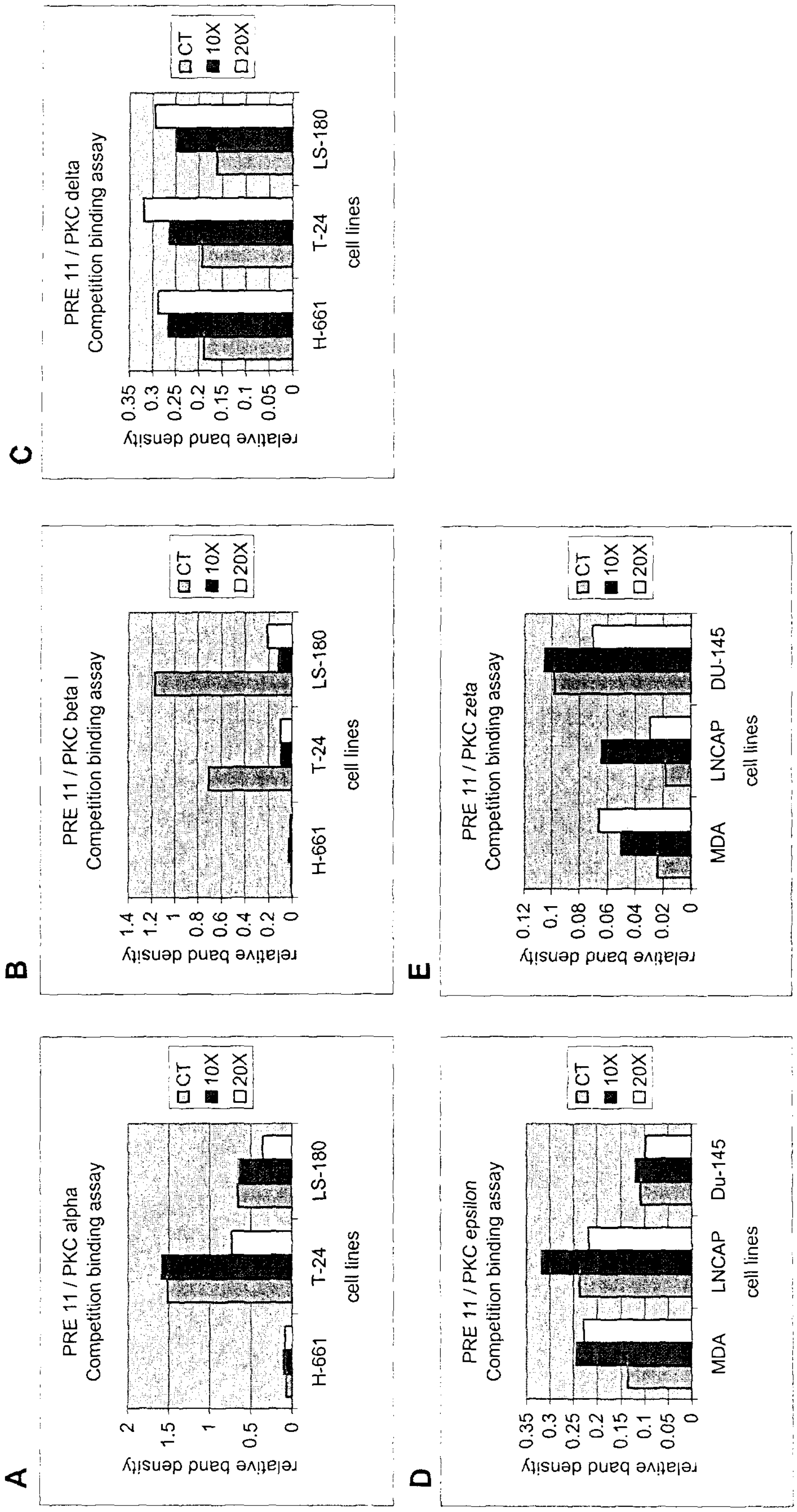
A
PRE 11 / PKC alpha
Competition binding assay
relative band density
2
1.5
1
0.5
0
H-661
T-24
LS-180
cell lines
CT
10X
20X
B
PRE 11 / PKC beta I
Competition binding assay
relative band density
1.4
1.2
1
0.8
0.6
0.4
0.2
0
H-661
T-24
LS-180
cell lines
CT
10X
20X
C
PRE 11 / PKC delta
Competition binding assay
relative band density
0.35
0.3
0.25
0.2
0.15
0.1
0.05
0
H-661
T-24
LS-180
cell lines
CT
10X
20X
D
PRE 11 / PKC epsilon
Competition binding assay
relative band density
0.35
0.3
0.25
0.2
0.15
0.1
0.05
0
MDA
LNCAP
Du-145
cell lines
CT
10X
20X
E
PRE 11 / PKC zeta
Competition binding assay
relative band density
0.12
0.1
0.08
0.06
0.04
0.02
0
MDA
LNCAP
DU-145
cell lines
CT
10X
20X

# FIGURE 15

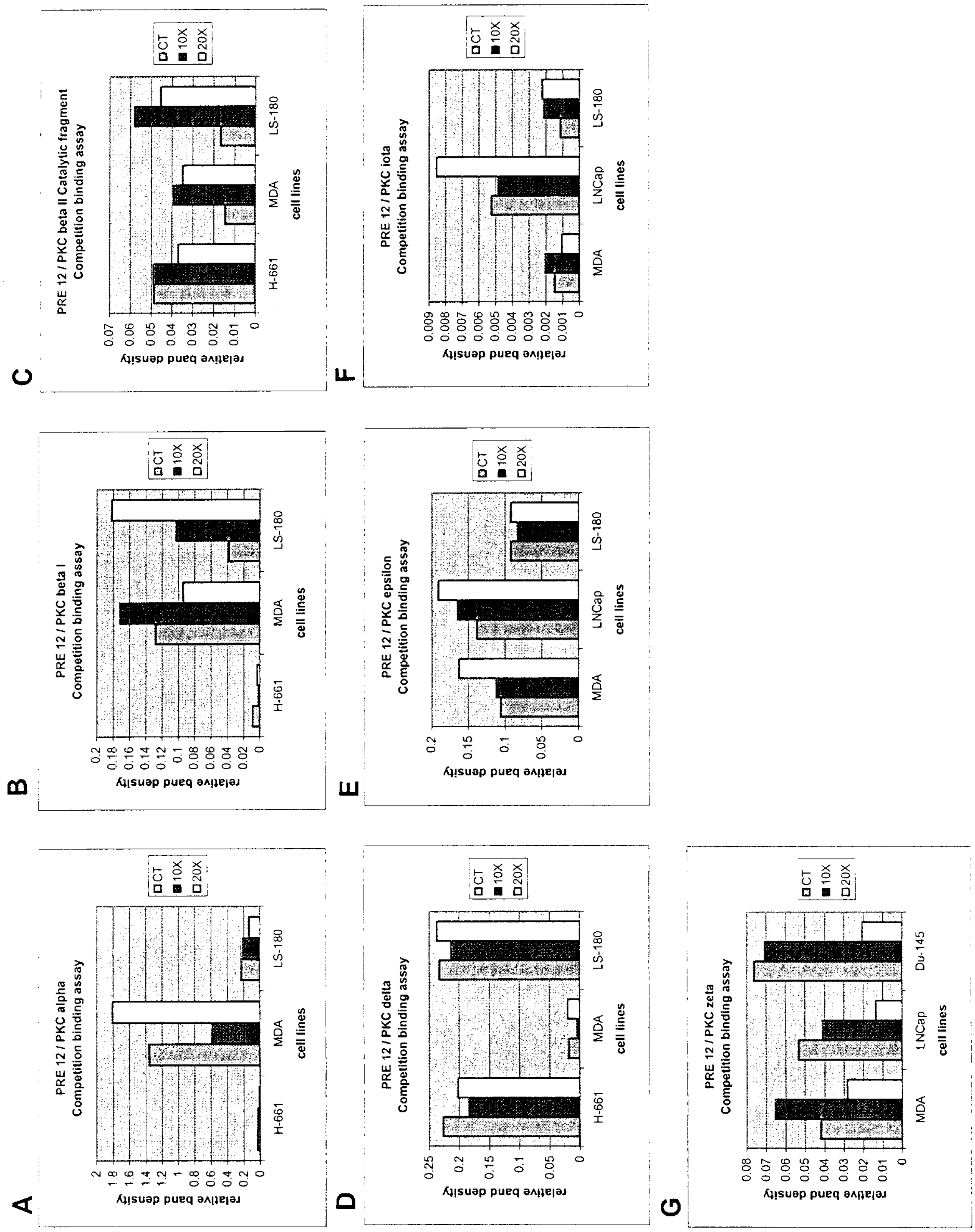
A
PRE 12 / PKC alpha
Competition binding assay
relative band density
cell lines
H-661
MDA
LS-180
CT
10X
20X
B
PRE 12 / PKC beta I
Competition binding assay
relative band density
cell lines
H-661
MDA
LS-180
C
PRE 12 / PKC beta II Catalytic fragment
Competition binding assay
relative band density
cell lines
H-661
MDA
LS-180
D
PRE 12 / PKC delta
Competition binding assay
relative band density
cell lines
H-661
MDA
LS-180
E
PRE 12 / PKC epsilon
Competition binding assay
relative band density
cell lines
MDA
LNCap
LS-180
F
PRE 12 / PKC iota
Competition binding assay
relative band density
cell lines
MDA
LNCap
LS-180
G
PRE 12 / PKC zeta
Competition binding assay
relative band density
cell lines
MDA
LNCap
Du-145

# FIGURE 16

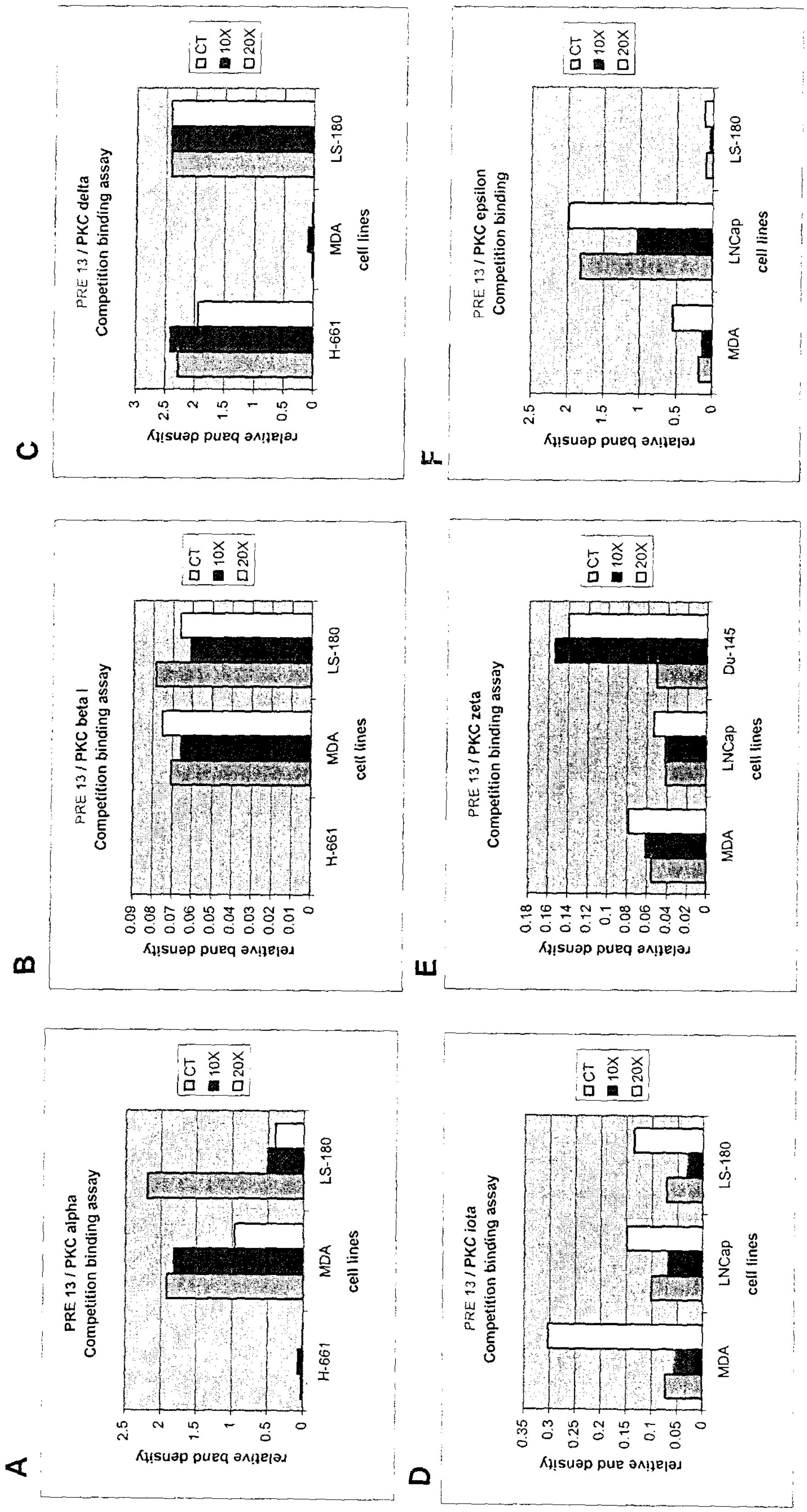
A
PRE 13 / PKC alpha
Competition binding assay
relative band density
cell lines
H-661
MDA
LS-180
CT
10X
20X
B
PRE 13 / PKC beta I
Competition binding assay
relative band density
cell lines
H-661
MDA
LS-180
CT
10X
20X
C
PRE 13 / PKC delta
Competition binding assay
relative band density
cell lines
H-661
MDA
LS-180
CT
10X
20X
D
PRE 13 / PKC iota
Competition binding assay
relative and density
cell lines
MDA
LNCap
LS-180
CT
10X
20X
E
PRE 13 / PKC zeta
Competition binding assay
relative band density
cell lines
MDA
LNCap
Du-145
CT
10X
20X
F
PRE 13 / PKC epsilon
Competition binding
relative band density
cell lines
MDA
LNCap
LS-180
CT
10X
20X

# FIGURE 17

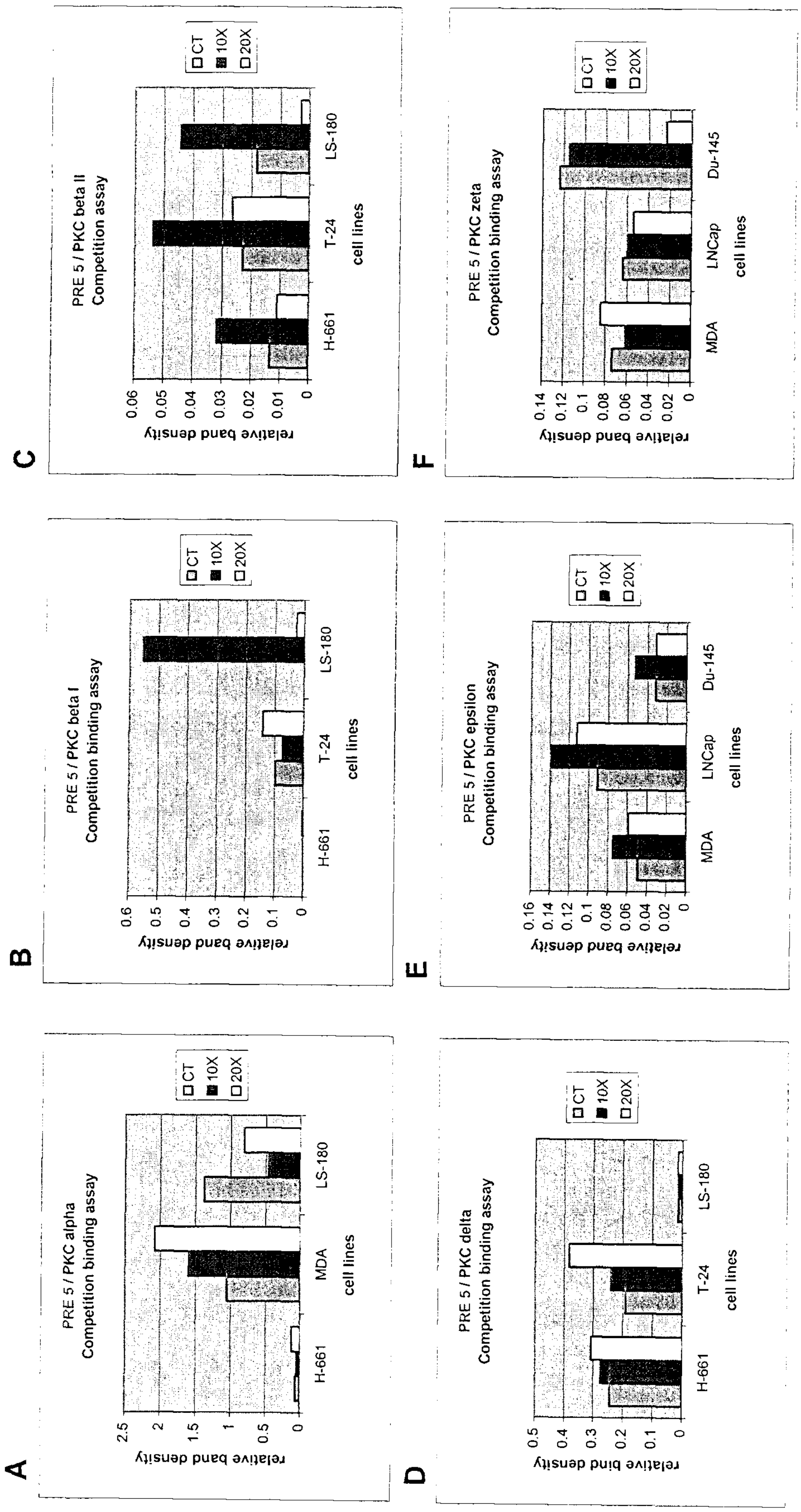
A
PRE 5 / PKC alpha
Competition binding assay
relative band density
cell lines
H-661
MDA
LS-180
CT
10X
20X
B
PRE 5 / PKC beta I
Competition binding assay
relative band density
cell lines
H-661
T-24
LS-180
CT
10X
20X
C
PRE 5 / PKC beta II
Competition assay
relative band density
cell lines
H-661
T-24
LS-180
CT
10X
20X
D
PRE 5 / PKC delta
Competition binding assay
relative bind density
cell lines
H-661
T-24
LS-180
CT
10X
20X
E
PRE 5 / PKC epsilon
Competition binding assay
relative band density
cell lines
MDA
LNCap
Du-145
CT
10X
20X
F
PRE 5 / PKC zeta
Competition binding assay
relative band density
cell lines
MDA
LNCap
Du-145
CT
10X
20X

FIGURE 18

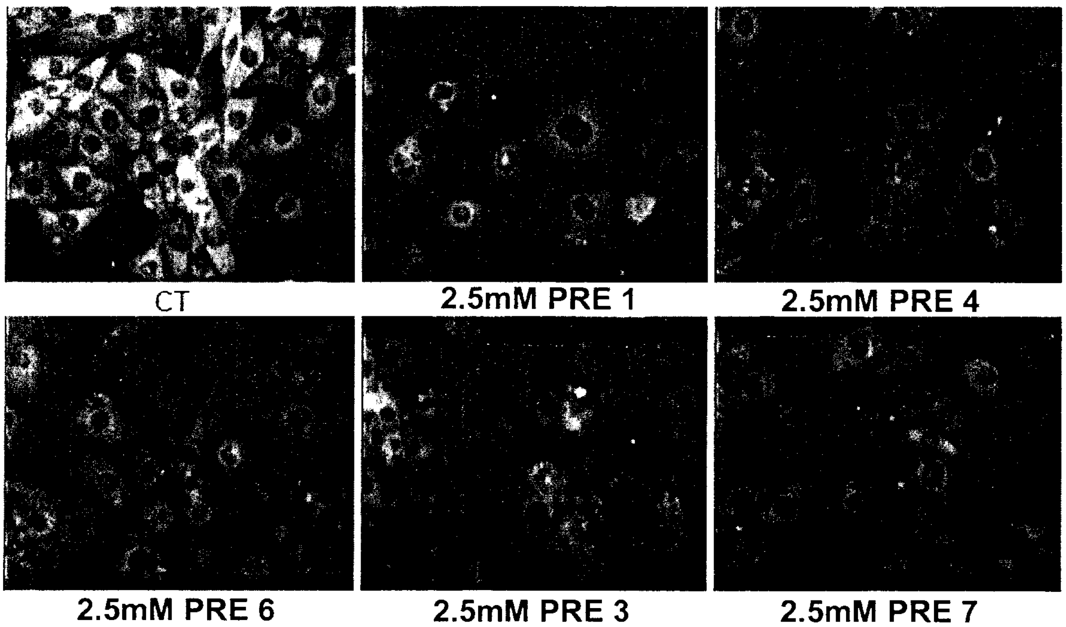

# FIGURE 19

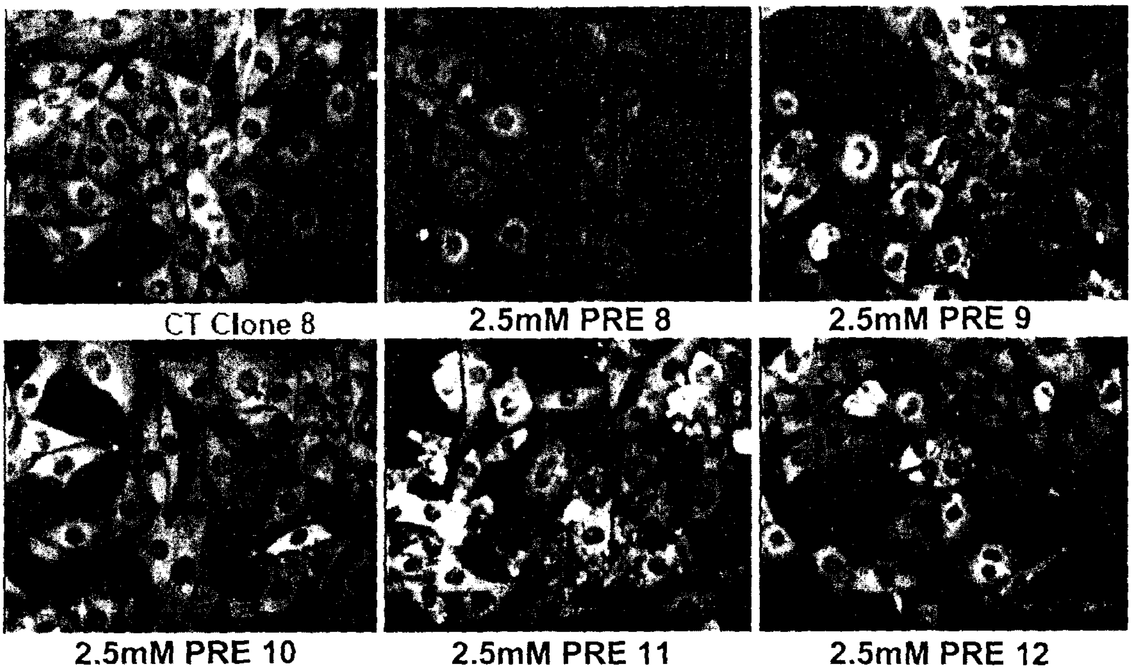
CT Clone 8
2.5mM PRE 8
2.5mM PRE 9
2.5mM PRE 10
2.5mM PRE 11
2.5mM PRE 12

# FIGURE 20

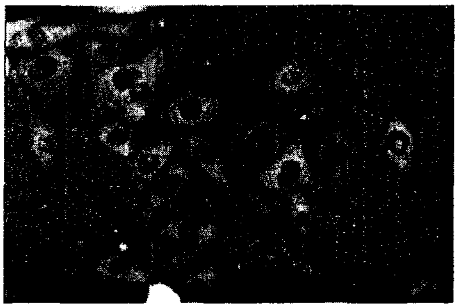
CT – Untransfected IMR-32

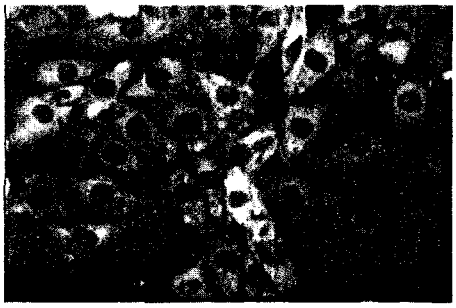
CT - V5E Transfected IMR-32

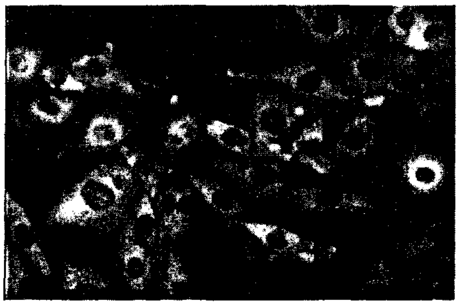
2.5mM PRE 13

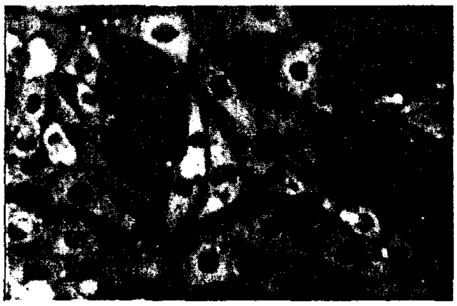
2.5mM PRE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6165977 A
- US 20030223981 A
- EP 9300816 W
- WO 9320101 A
- US 4522752 A
- EP 45665 A, Szelke
- EP 1982 A
- US 5643731 A


### Non-patent literature cited in the description


- **DUTIL, E.M. ; NEWTON, A.C.** *J. Biol. Chem.,* 2000, vol. 275 (14), 10697-10701
- **NEWTON, A.C.** *J. Biol. Chem.,* 1995, vol. 270 (48), 28495-28498
- **GOEKJIAN, P.G. ; JIROUSEK, M.R.** *Expert Opin. Investig. Drugs,* vol. 10, 2117-2140
- **XU et al.** *J. Biol. Chem.,* 2004, vol. 279 (48), 50401-50409
- **JOHNSON, J.A. et al.** *J. Biol. Chem.,* 1996, vol. 271, 24962-24966
- **GOEKJIAN, P.G. ; JIROUSEK, M.R.** *Curr. Medicinal Chem.,* 1999, vol. 6, 877-903
- **CLARK et al.** *Cancer Research,* 2003, vol. 63, 780-786
- **LAHN.** *Acta-Haematol.,* 2004, vol. 115, 1-8
- **POWELL et al.** *Cell Growth and Differentiation,* 1996, vol. 7, 419-428
- **KOIVUNEN et al.** *Cancer Research,* 2004, vol. 64, 5693-5701
- **DETJEN et al.** *J. Cell Sci.,* 2000, vol. 113, 3025-3035
- **HANAUSKE, A-R. et al.** *Curr. Pharm. Design,* 2004, vol. 10, 1923-1936
- **HOFMANN, J.** *Current Cancer Drug Targets,* 2004, vol. 4, 125-146
- **BROOKS G. et al.** *Carcinogenesis,* 1989, vol. 10, 283-288
- **WRIGHT M ; MCMASTER C.** *Biol. Res.,* 2002, vol. 35, 223-229
- **WANG W-L et al.** *J. Biol. Chem.,* 2003, vol. 278, 37705-37712
- **ROTENBERG S ; SUN X-G.** *J. Biol. Chem.,* 1998, vol. 273, 2390-2395
- **GOODMAN et al.** *Perspectives in Peptide Chemistry,* 1981, 283-294
- **SPATOLA.** *Vega Data,* 1983, vol. 1 (3
- **SPATOLA.** Chemistry and Biochemistry of Amino Acids Peptides and Proteins. Marcel Dekker, 1983, 267
- **MORLEY, J. S.** *Trends Pharm. Sci.,* 1980, 463-468
- **HUDSON et al.** *Int. J. Pept. Prot. Res.,* 1979, vol. 14, 177-185
- **SPATOLA et al.** *Life Sci.,* 1986, vol. 38, 1243-1249
- **HANN.** *J. Chem. Soc. Perkin Trans. I,* 1982, 307-314
- **ALMQUIST.** *J. Med. Chem.,* 1980, vol. 23, 1392-1398
- **JENNINGS-WHITE et al.** *Tetrahedron Lett.,* 1982, vol. 23, 2533
- **HOLLADAY et al.** *Tetrahedron Lett.,* 1983, vol. 24, 4401-4404
- **HRUBY.** *Life Sci.,* 1982, vol. 31, 189-199
- **VIVES, E. ; LEBLEU, B.** Cell-Penetrating Peptides. CRC, 2002, vol. 1, 3-23
- **PENNINGTON, M.W. ; DUNN, B.M.** Methods in Molecular Biology. Humana Press, 1994, vol. 35
- **DUGAS, H. ; PENNEY, C.** Bioorganic Chemistry. Springer-Verlag, 1981, 54-92
- **MERRIFIELD, J. M.** *Chem. Soc.,* 1962, vol. 85, 2149
- **STEWART ; YOUNG.** Solid Phase Peptide Synthesis. Freeman, 1969, 24-66
- Current Protocols in Protein Science. John Wiley & Sons, 2005
- Solid Phase Peptide Synthesis. Methods in Enzymology. Academic Press, 1997
- **AUSUBEL et al.** Current Protocols in Molecular Biology. Wiley & Sons, 1997
- **SAMBROOK et al.** Molecular Cloning: A Laboratory Manual. Cold-Spring Harbor Press, 2001
- **MORRISON ; BOYD.** Organic Chemistry. Prentice Hall, 1992
- **J. MARCH.** Advanced Organic Chemistry. Wiley, 1992
- **G. T. HARMANSON.** Bioconjugate Techniques. Academic Press, Inc, 1995
- **S. S. WONG.** Chemistry of Protein Conjugation and Cross-Linking. CRC Press, Inc, 1991
- **DIANOUX, A.C. et al.** *Biochemistry,* 1989, vol. 28, 424-431

- **GREENE, N.M.** *J. Biol. Chem.,* 1995, vol. 270, 6710-6717
- **OHGURO, H. et al.** *J. Biol. Chem.,* 1996, vol. 271, 5215-5224
- **HUANG, K.-P. et al.** *J. Biol. Chem.,* 1986, vol. 261, 12134-12140
- Affinity Chromatography: Principles & Methods. Pharmacia LKB Biotechnology, 1988
- **DOONAN.** Protein Purification Protocols. The Humana Press, 1996
- Current Protocols in Pharmacology. J. Wiley & Sons
- **GENNARO, A.** Remington: The Science and Practice of Pharmacy. Lippincott, Williams & Wilkins, 2000
- **RAO J ; OTTO W.** *Analytical Biochem.,* 1992, vol. 207, 186-192

SEQUENCE LISTING

<110> PharmaGap Inc.
PHIPPS, Jenny
TERREUX, Raphael

<120> PEPTIDES TARGETED TO PROTEIN KINASE C
ISOFORMS AND USES THEREOF

<130> 1360-115PCT

<150> 60/706,064
<151> 2005-08-05

<150> 2,539,218
<151> 2006-02-22

<160> 39

<170> FastSEQ for Windows Version 4.0

<210> 1
<211> 13
<212> PRT
<213> Artificial Sequence

<220>
<223> Peptide

<400> 1
```
Arg Arg Lys Lys Gly Gly Lys Asp Phe Val Val Lys Arg
 1               5                  10
```

<210> 2
<211> 11
<212> PRT
<213> Artificial Sequence

<220>
<223> Peptide

<400> 2
```
Lys Asp Ala Gln Asn Leu Ile Gly Ile Ser Ile
 1               5                  10
```

<210> 3
<211> 11
<212> PRT
<213> Artificial Sequence

<220>
<223> Peptide

<400> 3
```
Lys Asp Ala Asn Gln Leu Ile Gly Ile Ser Ile
 1               5                  10
```

```
<210> 4
<211> 20
<212> PRT
<213> Artificial Sequence

<220>
<223> Peptide

<400> 4
Ala Lys Gly Ile Gln Glu Val Lys Gly Gly Asp Ala Gln Asn Leu Ile
 1               5                  10                  15
Gly Ile Ser Ile
            20


<210> 5
<211> 17
<212> PRT
<213> Artificial Sequence

<220>
<223> Peptide

<400> 5
Ile Leu Glu Asp Lys Gly Gly Asp Ala Gln Asn Leu Ile Gly Ile Ser
 1               5                  10                  15
Ile


<210> 6
<211> 11
<212> PRT
<213> Artificial Sequence

<220>
<223> Peptide

<400> 6
Arg Asp Ala Gln Asn Leu Ile Gly Ile Ser Ile
 1               5                  10


<210> 7
<211> 21
<212> PRT
<213> Artificial Sequence

<220>
<223> Peptide

<400> 7
Ala Lys Gly Ile Gln Glu Val Lys Gly Gly Lys Asp Ala Gln Asn Leu
 1               5                  10                  15
Ile Gly Ile Ser Ile
            20


<210> 8
<211> 11
```

<212> PRT
<213> Artificial Sequence

<220>
<223> Peptide

<400> 8
Lys Asp Ala Gln Asn Leu Ile Gly Ile Ser Leu
 1               5                  10

<210> 9
<211> 7
<212> PRT
<213> Artificial Sequence

<220>
<223> Peptide

<400> 9
Lys Asp Ala Gln Asn Leu Ile
 1               5

<210> 10
<211> 7
<212> PRT
<213> Artificial Sequence

<220>
<223> Peptide

<400> 10
Arg Asp Ala Gln Asn Leu Ile
 1               5

<210> 11
<211> 11
<212> PRT
<213> Artificial Sequence

<220>
<223> Peptide

<220>
<221> AMIDATION
<222> (11)...(11)
<223> C-terminus is amidated

<400> 11
Lys Asp Ala Gln Asn Leu Ile Gly Ile Ser Leu
 1               5                  10

<210> 12
<211> 20
<212> PRT
<213> Artificial Sequence

<220>

<223> Peptide

<220>
<221> ACETYLATION
<222> (1)...(1)
<223> N-terminus is acetylated

<220>
<221> AMIDATION
<222> (20)...(20)
<223> C-terminus is amidated

<400> 12
```
Ala Lys Gly Ile Gln Glu Val Lys Gly Gly Asp Ala Gln Asn Leu Ile
 1               5                  10                  15
Gly Ile Ser Ile
            20
```

<210> 13
<211> 11
<212> PRT
<213> Artificial Sequence

<220>
<223> Peptide

<220>
<221> ACETYLATION
<222> (1)...(1)
<223> N-terminus is acetylated

<220>
<221> AMIDATION
<222> (11)...(11)
<223> C-terminus is amidated

<400> 13
```
Lys Asp Ala Gln Asn Leu Ile Gly Ile Ser Ile
 1               5                  10
```

<210> 14
<211> 21
<212> PRT
<213> Artificial Sequence

<220>
<223> Peptide

<220>
<221> ACETYLATION
<222> (1)...(1)
<223> N-terminus is acetylated

<220>
<221> AMIDATION
<222> (21)...(21)
<223> C-terminus is amidated

<400> 14

```
Ala Lys Gly Ile Gln Glu Val Lys Gly Gly Lys Asp Ala Gln Asn Leu
 1               5                   10                  15
Ile Gly Ile Ser Ile
            20


<210> 15
<211> 12
<212> PRT
<213> Artificial Sequence

<220>
<223> Peptide

<220>
<221>
<222> (1)...(1)
<223> Xaa at position 1 is Dansylglycine

<220>
<221> AMIDATION
<222> (12)...(12)
<223> C-terminus is amidated

<400> 15
Xaa Lys Asp Ala Gln Asn Leu Ile Gly Ile Ser Ile
 1               5                   10


<210> 16
<211> 11
<212> PRT
<213> Artificial Sequence

<220>
<223> Peptide

<220>
<221> ACETYLATION
<222> (1)...(1)
<223> N-terminus is acetylated

<220>
<221> AMIDATION
<222> (11)...(11)
<223> C-terminus is amidated

<400> 16
Lys Asp Ala Asn Gln Leu Ile Gly Ile Ser Ile
 1               5                   10


<210> 17
<211> 11
<212> PRT
<213> Artificial Sequence

<220>
<223> Peptide

<220>
```

<221> ACETYLATION
<222> (1)...(1)
<223> N-terminus is acetylated

<220>
<221> AMIDATION
<222> (11)...(11)
<223> C-terminus is amidated

<400> 17
Ile Ser Ile Gly Ile Leu Gln Asn Ala Asp Lys
1 5 10

<210> 18
<211> 11
<212> PRT
<213> Artificial Sequence

<220>
<223> Peptide with amino acids from position 1 to 11 being D-amino acids

<220>
<221> ACETYLATION
<222> (1)...(1)
<223> N-terminus is acetylated

<220>
<221> AMIDATION
<222> (11)...(11)
<223> C-terminus is amidated

<400> 18
Ile Ser Ile Gly Ile Leu Gln Asn Ala Asp Lys
1 5 10

<210> 19
<211> 11
<212> PRT
<213> Artificial Sequence

<220>
<223> Peptide

<220>
<221> ACETYLATION
<220>
<222> (1)...(1)
<223> N-terminus is acetylated

<220>
<221> AMIDATION
<222> (11)...(11)
<223> C-terminus is amidated

<400> 19
Ile Ser Ile Gly Ile Leu Asn Gln Ala Asp Lys
1 5 10

<210> 20
<211> 11
<212> PRT
<213> Artificial Sequence

<220>
<223> Peptide

<220>
<221> ACETYLATION
<222> (1)...(1)
<223> N-terminus is acetylated

<220>
<221> AMIDATION
<222> (11)...(11)
<223> C-terminus is amidated

<400> 20
Arg Asp Ala Gln Asn Leu Ile Gly Ile Ser Ile
 1               5                  10

<210> 21
<211> 7
<212> PRT
<213> Artificial Sequence

<220>
<223> Peptide

<220>
<221> ACETYLATION
<222> (1)...(1)
<223> N-terminus is acetylated

<220>
<221> AMIDATION
<222> (7)...(7)
<223> C-terminus is amidated

<400> 21
Lys Asp Ala Gln Asn Leu Ile
 1               5

<210> 22
<211> 7
<212> PRT
<213> Artificial Sequence

<220>
<223> Peptide

<220>
<221> ACETYLATION
<222> (1)...(1)
<223> N-terminus is acetylated

<220>

<221> AMIDATION
<222> (7)...(7)
<223> C-terminus is amidated

<400> 22
Arg Asp Ala Gln Asn Leu Ile
 1               5

<210> 23
<211> 11
<212> PRT
<213> Artificial Sequence

<220>
<223> Peptide

<400> 23
Ile Ser Ile Gly Ile Leu Gln Asn Ala Asp Lys
 1               5                  10

<210> 24
<211> 11
<212> PRT
<213> Artificial Sequence

<220>
<223> Peptide with amino acids from position 1 to 11 being D-amino acids

<400> 24
Ile Ser Ile Gly Ile Leu Gln Asn Ala Asp Lys
 1               5                  10

<210> 25
<211> 11
<212> PRT
<213> Artificial Sequence

<220>
<223> Peptide

<400> 25
Ile Ser Ile Gly Ile Leu Asn Gln Ala Asp Lys
 1               5                  10

<210> 26
<211> 11
<212> PRT
<213> Artificial Sequence

<220>
<223> Peptide

<400> 26
Arg Arg Arg Arg Gly Gln Gln Asn Asn Leu Ser
 1               5                  10

<210> 27
<211> 13
<212> PRT
<213> Artificial Sequence

<220>
<223> Peptide

<400> 27
Lys Lys Lys Lys Gly Gly Asn Leu Val Lys Arg Ile Leu
1 5 10

<210> 28
<211> 25
<212> PRT
<213> Artificial Sequence

<220>
<223> Peptide

<400> 28
Ala Arg Ile Gln Gln Glu Ile Leu Lys Lys Arg Gly Gly Gly Lys Asp
1 5 10 15
Ala Gln Asn Leu Ile Gly Ile Ser Leu
20 25

<210> 29
<211> 21
<212> PRT
<213> Artificial Sequence

<220>
<223> Peptide

<400> 29
Ala Arg Gly Ile Gln Glu Phe Arg Gly Gly Lys Glu Ala Gln Asn Leu
1 5 10 15
Val Ile Ser Ile Leu
20

<210> 30
<211> 11
<212> PRT
<213> Artificial Sequence

<220>
<223> Peptide

<400> 30
Arg Glu Ala Gln Asn Leu Ile Gly Ile Ser Ile
1 5 10

<210> 31
<211> 10
<212> PRT
<213> Artificial Sequence

<220>
<223> Peptide

<400> 31
Glu Ala Gln Asn Leu Ile Gly Ile Ser Ile
1               5                   10

<210> 32
<211> 11
<212> PRT
<213> Artificial Sequence

<220>
<223> Peptide

<400> 32
Glu Ala Gln Asn Val Ile Val Ile Ser Ile Leu
1               5                   10

<210> 33
<211> 6
<212> PRT
<213> Artificial Sequence

<220>
<223> Peptide

<400> 33
Glu Ala Gln Val Ser Ile
1               5

<210> 34
<211> 7
<212> PRT
<213> Artificial Sequence

<220>
<223> Peptide

<400> 34
Lys Ala Gln Asn Ile Ser Ile
1               5

<210> 35
<211> 9
<212> PRT
<213> Artificial Sequence

<220>
<223> Peptide

<400> 35
Arg Asp Ala Gln Val Val Arg Ile Val
1               5

```
<210> 36
<211> 21
<212> PRT
<213> Artificial Sequence

<220>
<223> Peptide - corresponds to a portion of the V5
      domain of protein kinase C-alpha

<400> 36
Asp Phe Glu Gly Phe Ser Tyr Val Asn Pro Gln Phe Val His Pro Ile
 1              5                   10                  15
Leu Gln Ser Ala Val
            20


<210> 37
<211> 66
<212> DNA
<213> Artificial Sequence

<220>
<223> Nucleotide sequence - encodes a portion of the V5
      domain of protein kinase C-alpha

<400> 37
gattttgaag ggttctcgta tgtcaacccc cagtttgtgc accccatctt acagagtgca 60
gtatga                                                            66

<210> 38
<211> 49
<212> DNA
<213> Artificial Sequence

<220>
<223> Sense Primer

<400> 38
ggggacaagt ttgtacaaaa aagcaggctt ggattttgaa gggttctcg             49

<210> 39
<211> 47
<212> DNA
<213> Artificial Sequence

<220>
<223> Antisense Primer

<400> 39
ggggaccact ttgtacaaga aagctgggtt catactgcac tctgtaa               47
```